# FASCICULE DE BREVET EUROPEEN

(11) **EP 1 446 004 B1**
(45) Date de publication et mention de la délivrance du brevet: **30.04.2008**
(21) Numéro de dépôt: 02796854.4
(22) Date de dépôt: 21.11.2002
(51) Int. Cl.: A01K 67/027, C12N 15/90, C12N 15/63, C12N 15/06, C12P 21/00

(54) **SYSTEME D'EXPRESSION DE PROTEINES EXOGENES DANS UN SYSTEME AVIAIRE**
AVIÄRES EXPRESSIONSSYSTEM FÜR FREMDPROTEINE
EXOGENOUS PROTEIN EXPRESSION SYSTEM IN AN AVIAN SYSTEM

(30) Priorité: 22.11.2001 FR 0115111
(43) Date de publication de la demande: 18.08.2004
(73) Titulaire: Vivalis, 49450 Roussay (FR); INSTITUT NATIONAL DE LA RECHERCHE AGRONOMIQUE (INRA), 75007 Paris (FR); Centre National de la Recherche Scientifique - CNRS, 75016 Paris Cedex 16 (FR); ENS - Ecole Normale Supérieure de Lyon, 69000 Lyon (FR)
(72) Inventeur: PAIN, Bertrand, F-69003 Lyon (FR); SAMARUT, Jacques, F-69100 Villeurbanne (FR); VALARCHE, Isabelle, F-44100 Nantes (FR); CHAMPION-ARNAUD, Patrick, F-44300 Nantes (FR); SOBCZYK, André, F-44100 Nantes (FR); KUNITA, Ryota, Atsugi-shi, 243-0035 Kanagawa (JP)
(74) Mandataire: Warcoin, Jacques
(86) Numéro de dépôt international: PCT/FR2002/003992
(87) Numéro de publication internationale: WO 2003/043415

(56) Documents cités:
- WO-A-01/84920
- WO-A-97/47739
- WO-A-98/38283
- WO-A-99/19472
- TOBA M ET AL: "Introduction of DT40 cells into chick embryos." ASIAN JOURNAL OF ANDROLOGY. CHINA MAR 2001, vol. 3, no. 1, mars 2001 (2001-03), pages 49-53, XP002208512
- PAIN B ET AL: "CHICKEN EMBRYONIC STEM CELLS AND TRANSGENIC STRATEGIES" CELLS TISSUES ORGANS, KARGER, BASEL, CH, vol. 165, 1999, pages 212-219, XP000882175 ISSN: 1422-6405 cité dans la demande
- ZAJCHOWSKI LAURA D ET AL: "Transgenic chickens: Past, present, and future." AVIAN AND POULTRY BIOLOGY REVIEWS, vol. 11, no. 2, 2000, pages 63-80, XP001100013 ISSN: 1470-2061
- PRELLE K ET AL: "ESTABLISHMENT OF PLURIPOTENT CELL LINES FROM VERTEBRATE SPECIES - PRESENT STATUS AND FUTURE PROSPECTS" CELLS TISSUES ORGANS, KARGER, BASEL, CH, vol. 165, no. 3/4, 1999, pages 220-236, XP000914313 ISSN: 1422-6405
- Abstract available at: http://www.poultryscience.org/pba/1952-200 3/2001/2001%20Christman.pdf Retrieved: Oct. 3rd, 2007 50th Annual National Breeders Roundtable, St Louis, Missouri, May 3-4, 2001. Speaker: Dr Leandro Christmann: "Development of nuclear transfer (cloning) technology in avian species".

## Description

### DOMAINE TECHNIQUE:

La présente invention se rapporte à l'utilisation d'une cellule aviaire pour la production d'une protéine exogène d'intérêt dans un animal appartenant à l'espèce aviaire, ladite cellule étant transformée par un vecteur d'expression comprenant le gène codant pour ladite protéine, ladite cellule étant introduite dans la cavité sub-germinale d'un embryon, ou la circulation sanguine de l'embryon.

### ETAT DE LA TECHNIQUE ANTÉRIEURE:

Traditionnellement, les premiers principes pharmaceutiques étaient obtenus par extraction à partir de tissus de différentes origines tant végétales, animales que même humaines. Trois principaux facteurs ont contribué à la mise en place de substituts à cette technique d'extraction. La raréfaction des tissus en raison de l'augmentation de la demande, les risques inhérents aux dérivés animaux et humains ont augmenté, avec l'apparition ou l'identification de nouvelles pathologies, notamment virales et non conventionnelles. Enfin et surtout, la recherche et l'utilisation des découvertes des différentes branches de la biologie (biochimie, biologie moléculaire ...) ont permis de découvrir et donc de satisfaire la demande sans cesse croissante de nouvelles molécules.

Pour répondre à la demande en produit thérapeutique, les systèmes d'expression de molécules, comme les protéines à usage thérapeutique, ont été associés à une composante biologique active (bactérie, levure, cellule eucaryote supérieure...) et un élément génétique qui apporte le support nécessaire à la production. Les différents supports génétiques utilisés sont le plus souvent soient des vecteurs d'expression simples optimisés pour l'expression mais dont l'intégration demeure aléatoire soient des systèmes viraux ou rétroviraux. Ces derniers ne permettent pas non plus de contrôler ni le lieu et ni le niveau d'expression.

Ces méthodes de production sont actuellement en train de vivre une nouvelle révolution tant numérique que qualitative. Ainsi, en l'espace de quelques années, grâce aux progrès constants, rapides de la génomique et de la biologie moléculaire, le nombre de molécules qui possède une ou plusieurs potentialités thérapeutiques et médicales a significativement augmenté. Par exemple, les anticorps à visée thérapeutique représentent déjà environ 50% de ces molécules. De plus, la complexité de ces molécules recombinantes s'est accentuée. Des simples peptides ou petites cytokines, produits facilement dans les bactéries, on arrive progressivement à des molécules complexes, avec une activité biologique strictement dépendante de leur structure secondaire, de leur repliement et de modifications post traductionnelles complexes. En conséquence, le simple moyen de production dans les bactéries ne suffit plus.

Des techniques plus sophistiquées ont alors émergé, parmi lesquelles on peut citer la culture in vitro de cellules eucaryotes, les levures et les cellules d'insecte. Plus récemment encore, les progrès réalisés en transgénèse végétale et animale ont été mis à profit pour produire ces molécules complexes soit dans des feuilles ou des graines d'espèces végétales (tabac, mais...) soit dans les liquides physiologiques des animaux comme le lait d'animaux (chèvre, vache, truie, lapin...).

En outre, la corrélation entre l'efficacité de ciblage d'un locus donné et son niveau de transcription, notamment au niveau des cellules ciblées par le vecteur dont les cellules souches n'est pas absolue. Ainsi, ces méthodes présentent toujours des problèmes tels que le coût de production, la quantité de produits obtenus et des difficultés techniques lorsqu'ils s'agit de produire des molécules complexes.

### RÉSUMÉ DE L'INVENTION:

Pour résoudre ces problèmes, il est proposé dans le cadre de l'invention de produire des molécules thérapeutiques en ciblant l'expression dans des tissus spécifiques, notamment dans l'oeuf et en particulier dans le blanc de l'oeuf à l'aide d'animaux modifiés avec des cellules souches, elles même modifiées par des vecteurs de recombinaison.

Les différentes étapes nécessaires à la mise en oeuvre de l'invention impliquent d'abord la construction de vecteurs, qui permet le ciblage du locus choisi dans une cellule aviaire et en particulier une cellule souche, l'obtention de cellules recombinées qui intègre ce vecteur dans le locus choisi et la génération des animaux modifiés avec ces cellules.

Un des avantages de l'ensemble de ce système est le caractère contrôlé de l'expression de la protéine d'intérêt. En effet, l'action de remplacement substitution d'un locus endogène aviaire par un gène ou un fragment de gène exogène d'intérêt permet d'obtenir une production de la protéine exogène introduite en lieu et place du locus endogène. Par exemple, en introduisant dans le locus de l'ovalbumine le gène à produire par l'ensemble du mécanisme décrit, le produit de ce gène sera produit selon le profil d'expression de l'ovalbumine, parfaitement connu. Ainsi, l'invention apporte une solution aux problèmes soulevés par l'intégration au hasard d'une séquence hétérologue dont l'expression pourrait dépendre de son environnement chromatinien.

En outre et contrairement aux méthodes évoquées ci-dessus, le système de production aviaire de l'invention permet d'obtenir des quantités supérieures de substances actives à moindre coût étant donné qu'elles peuvent être facilement isolées et purifiées.

Un processus de recombinaison dans une lignée de cellules lymphoïdes (la lignée DT40) a été rendu possible grâce à un processus de recombinaison en raison d'une mutation particulière (la mutation affecte le niveau d'expression de la protéine Rad54) (Kim et al., 1990 ; Baba et al., 1988; Buerstedde et al., 1990 ; Buerstedde and Takeda, 1991 ; Bezzubova et al., 1993; Bezzubova et al., 1997). Dans cette publication, les auteurs reconnaissent ne pas avoir été en mesure d'effectuer de la recombinaison homologue avec des cellules ES.

Le document WO9919472 décrit la préparation de poulets transgéniques exprimant un gène hétérologue d'intérêt dans les cellules de l'oviduct.

Le document par Prelle K et al (1999) dans "Cells, Tissues and Organs" (ISSN: 1422-6405), vol. 165, pages 220-236, décrit, à propos des cellules embryonnaires souches aviaires qu'elles sont les seules cellules embryonnaires souches, à part les cellules embryonnaires souches de souris, dont la transmission dans la lignée germinale a été montrée.

Contrairement à cet enseignement, le demandeur a démontré qu'un processus de recombinaison homologue ciblé permet une expression d'une molécule exogène d'intérêt dans des systèmes aviaires et que ce même processus est maîtrisable au niveau expérimental dans différents types de cellules aviaires, dont des lignées, des cellules primaires et des cellules souches et cellules souches embryonnaires.

L'objet de l'invention est donc :

Un Procédé d'obtention d'une cellule souche embryonnaire (ES) aviaire modifiée par recombinaison homologue, ledit procédé comprenant les étapes suivantes :
A) l'introduction d'un vecteur de recombinaison homologue dans ladite cellule ES aviaire par une méthode de transfection ;
B) la sélection des cellules par l'addition d'un agent de sélection dans le milieu de culture ; et
C) le criblage des clones résistants et amplification,
caractérisé en ce que :
ledit vecteur de recombinaison homologue introduit à l'étape A) comprend dans une base plasmidique un enchaînement d'au moins un élément pris successivement parmi
   a) un fragment d'ADN génomique contenant le bras d'homologie 5' du gène ciblé fusionné à,
   b) une séquence nucléotidique signal de sécrétion fusionnée à,
   c) une courte séquence nucléotidique intronique fusionnée à,
   d) une séquence nucléotidique codant pour une protéine exogène d'intérêt fusionnée à,
   e) une séquence poly A de terminaison de la transcription fusionnée à,
   f) une cassette de sélection positive comprenant un promoteur, un gène de résistance à un agent de sélection et une séquence poly A de terminaison de transcription, ladite cassette pouvant être fusionnée à,
   g) un fragment d'ADN génomique contenant le bras d'homologie 3' du gène ciblé,
   h) une cassette de sélection négative comprenant un promoteur, un gène assurant la transformation d'un substrat présent dans le milieu de culture en une substance toxique pour la cellule qui exprime le gène et une séquence polyA de terminaison de transcription, et
caractérisé en ce que :
le locus ciblé par le vecteur de recombinaison est le locus de l'ovalbumine ou le locus du lysozyme.

Un Procédé de production d'une protéine d'intérêt comprenant l'extraction de la protéine exogène exprimée dans le surnageant des cellules issues du procédé selon l'une des revendications 1 à 15.
· L'Utilisation d'une cellule aviaire obtenue par un procédé selon l'une des revendications 1 à 15 pour la production de la protéine exogène d'intérêt.
· Un Procédé d'obtention d'un animal appartenant à l'espèce aviaire capable d'exprimer une protéine exogène d'intérêt, caractérisé en ce qu'il comprend les étapes suivantes:
   a) obtention de cellules aviaires modifiées par le procédé défini selon l'une des revendications 1 à 15,
   b) introduction de la cellule obtenue à l'étape a) dans la cavité sub-germinale d'un embryon, ou dans la circulation sanguine de l'embryon, et
   c) incubation de l'embryon obtenu à l'étape b).

Un Procédé de production d'une protéine d'intérêt comprenant l'extraction de la protéine exogène exprimée dans les tissus d'un animal obtenu à partir du procédé selon l'une des revendications 18 à 20.

Un Procédé selon la revendication 21, caractérisé en ce que la protéine est extraite du sang, du jaune ou du blanc d'oeuf.

Un OEuf susceptible d'être obtenu à partir d'un animal obtenu par un procédé selon l'une des revendications 18 à 20, caractérisé en ce qu'une partie de l'ovalbumine, ou du lysozyme est partiellement ou totalement remplacée par la protéine d'intérêt exogène.

L'ensemble de cette invention permet donc de proposer un système original de production de molécules d'intérêt selon un mode parfaitement prédictible car contrôlé tant au niveau spatial que temporel, apportant les réponses aux problèmes existant dans l'état de la technique.

### DESCRIPTION

Ainsi, dans un premier aspect, l'invention se rapporte à l'utilisation d'une cellule aviaire pour la production d'une protéine exogène d'intérêt dans un animal appartenant à l'espèce aviaire, caractérisée en ce que ladite cellule est transformée par un vecteur d'expression comprenant le gène codant pour ladite protéine, ladite cellule étant introduite soit dans la cavité sub-germinale d'un embryon, soit dans la circulation sanguine de l'embryon.

Par cellule aviaire, on entend une cellule souche embryonnaire (ES), un clone cellulaire des cellules précitées, une lignée établie à partir des cellules précitées ou un corps embryoïde obtenu à partir des cellules précitées.
Les différentes cellules visées précédemment sont davantage définies ci-après :
- Par cellule souche embryonnaire (ES), on entend une cellule non différenciée dérivant d'un embryon et qui a les potentialités de donner une très large variété de cellules spécialisées.
- Par clone cellulaire, on entend tout ensemble de cellules génétiquement identiques à la cellule de laquelle elles dérivent par division.
- Par corps embryoïdes, on entend tout agrégat de cellules dérivant des cellules souches embryonnaires maintenues en culture. Les corps embryoïde ne participent pas au développement normal d'un embryon et s'observent seulement in vitro.
- Par lignée, on entend toute culture cellulaire capable de proliférer indéfiniment en gardant les mêmes caractéristiques phénotypiques.

### I- Différents vecteurs peuvent être mis en oeuvre pour la transformation desdites cellules :

Le vecteur utilisé à cet effet, un vecteur de recombinaison permet une expression tissu-spécifique, en particulier dans l'oviducte, le foie, le sang, la moelle et les organes lymphoïdes.

### 1.1 Vecteurs de recombinaison.

Le vecteur de recombinaison homologue permet de cibler un locus endogène pour exprimer un gène d'intérêt en lieu et place de ce locus endogène. Au niveau de la chromatine, la recombinaison homologue est un processus qui consiste à remplacer partiellement ou totalement une partie d'un gène par une construction qui reprend en partie ou en totalité certaines séquences à l'identique de ce même gène. Les parties identiques permettent de réaliser l'échange de l'ADN apporté dans le locus endogène et d'introduire les parties modifiées dans l'environnement considéré et donc respecté du locus ciblé. Il en résulte une insertion dans le locus ciblé de modifications apportées par certaines parties de la construction. Les parties différentes de la construction permettent de contrôler l'expression des cassettes introduites. Ces cassettes sont soient dépendantes de leur propre promoteur, soient dépendantes de l'activité transcriptionnelle du locus ciblé, quand la cassette est placée sous la dépendance du promoteur interne.
La réaction de recombinaison peut être divisée en trois grandes étapes :
- La première étape est l'appariement des régions homologues, conduisant à une structure transitoire 'trois brins' du DNA.
- La seconde étape est la réalisation d'un double crossing over indépendants dans les régions d'homologies, conduisant à la formation d'une molécule hybride entre le chromosome ciblé et le vecteur introduit
- La troisième est l'intégration de ce vecteur comme élément génétique stable du chromosome ciblé, la modification étant alors propagée comme l'ensemble du matériel génétique avec les divisions cellulaires

Tout élément, présent dans le système ou apporté de façon stable ou transitoire qui contrôle, modifie ou influence ces différents mécanismes aura une contribution essentielle dans le processus de recombinaison et donc dans l'efficacité d'intégration du vecteur.

Dans le cadre de l'invention, le vecteur de recombinaison qui permet de produire les molécules dans le système aviaire, notamment chez le poulet n'est pas un simple vecteur de destruction, mais un vecteur de remplacement substitutif. Ces vecteurs complexes sont le résultat de l'association ordonnée et successive dans un plasmide de clonage classique (pBSK, pUC,...) d'au moins un des éléments suivants :
- Un fragment d'ADN génomique, de taille variable, pris en amont de l'ATG (site de démarrage de la traduction) du gène ciblé. Cet élément constitue le bras 5'.
- Un cDNA ou une partie du ou des gènes d'intérêt, placé directement sous la dépendance de l'ATG du gène ciblé endogène. Cette association des fragments est dans la plupart des cas directement réalisée par une fusion sur l'ATG du locus ciblé avec le deuxième acide aminé de la protéine. De plus, pour permettre l'exportation directement dans l'oeuf de la protéine d'intérêt, une séquence nucléotidique spécifiant un peptide signal doit être placée en amont du cDNA d'intérêt et en aval des signaux du gène ciblé. Elle est soit apportée soit par le fragment d'intérêt, soit par une séquence nucléotidique exogène supplémentaire.

Par cDNA, on entend toute ou partie d'une séquence codante d'acide nucléique pour une protéine d'intérêt.
Par protéine d'intérêt, on entend une protéine, un fragment d'une protéine ou un peptide qui présente un intérêt thérapeutique pour l'homme ou l'animal, mais aussi toute forme protéique susceptible d'apporter à l'homme ou à l'animal un bienfait quant à son statut physique, sa santé, son comportement ou sa vitalité, ce qui comprend notamment une protéine ou un peptide d'intérêt endogène et une protéine ou un peptide d'intérêt exogène définie ci-dessous :
Par protéine d'intérêt endogène, on entend toute protéine présente dans le système, l'oeuf en particulier, présente à des niveaux variables mais détectables dans une situation physiologique normale.
Par protéine d'intérêt exogène, on entend toute protéine d'origine exogène au système, non identifiée dans le système, l'oeuf en particulier, dans un état physiologique normal. Sa présence est le résultat direct ou indirect de la modification génétique et /ou biochimique induite par le vecteur dans le système.

- Une cassette de résistance (dite cassette de sélection positive à un système exogène de sélection) constituée généralement d'un promoteur, d'un cDNA qui apporte la résistance à la sélection exogène et d'une séquence poly A permettant l'arrêt de la transcription de cet élément transcriptionnel indépendant. Le promoteur peut être de différentes origines.
- Un fragment d'ADN génomique, de taille variable, pris en aval de ces différents éléments et donc de l'ATG du gène ciblé. Ce fragment présente en général une taille différente du fragment d'ADN génomique situé en 5'. Cet élement constitue le bras 3'.
- Une cassette supplémentaire dite de sélection négative peut être incorporée dans ce vecteur. Elle est alors en général intégrée en aval du bras génomique 3'. Située en 3' du bras d'homologie 3', cette cassette ne persiste pas dans le génome de la cellule ciblée si l'intégration a lieu selon le schéma correct. Seuls les événements non recombinés correctement sont sensibles à cette action.
- Au moins un site de linéarisation unique sur le vecteur, recommandé par l'expérience. Cette étape semble nécessaire pour l'introduction efficace de ce vecteur dans le patrimoine génétique de la cellule ciblée.
- Différents éléments permettant notamment des modifications conditionnelles peuvent être insérés à différents endroits de la construction

Les principales caractéristiques d'un vecteur de recombinaison tel qu'ils sont décrits à ce jour dans les systèmes mammifères tiennent dans la succession des différents éléments au sein du plasmide et dans le positionnement des bras génomiques (origine du DNA, taille des éléments génomiques homologues, dissymétrie relative...). Dans 1a majorité des cas, la fréquence de recombinaison observée in vitro est dépendante du locus ciblé donc de l'environnement chromatinien ciblé (Ramirez-Solis et al., 1993 ; Hasty et al., 1995 ; Hasty et al., 1991). Cet environnement est étroitement lié à la physiologie de la cellule. Il apparaît que la pression de sélection appliquée sur les cellules transfectées permet un nombre d'intégrations majoritairement non homologues par rapport au nombre d'intégrations homologues. Le processus de criblage, dont le principe et les exemples sont développés dans le texte permet de distinguer les deux types d'évènements. Connaître, évaluer et maîtriser la proportion d'intégration homologue est essentielle pour une stratégie d'expression satisfaisante. La fidélité de la recombinaison entre les fragments apportés et ceux d'origine doit également être la meilleure possible. Cela semble être le cas au moins pour les cellules ES de souris et les fibroblastes humains (Zheng et al., 1991; Lederman et al., 2000 ; Templeton, 2000).

Par ailleurs, la seule composition en base d'un locus peut fortement influencer les efficacités de recombinaison, notamment par la forte présence de nombreux îlots CpG (Yanez and Porter, 2000). D'autres éléments cryptiques (séquence poison) peuvent également modifier l'efficacité du processus de recombinaison homologue (sites d'initiation de réplication, séquences répétées...). De plus, la fréquence de recombinaison homologue semble très dépendante de la cellule cible, avec des situations particulières comme pour la lignée de cellules lymphoïdes DT40 de poulet, qui présente des taux de recombinaison impressionnants (> 10%). Cette spécificité est liée à une sur-expression de la molécule Rad54, sans doute liée à une mutation, non encore identifiée qui intervient dans le contrôle du niveau de régulation de ce gène (Bezzubova et al., 1993 ; Bezzubova et al., 1994; Bezzubova et al., 1997). Cette observation peut donner lieu à une augmentation de la fréquence de recombinaison.

Dans le cadre de l'invention, on a démontré l'efficacité de la recombinaison homologue dans des cellules embryonnaires aviaires et certaines autres cellules aviaires primaires qui ont été établies en lignée.
Dans un mode complémentaire de réalisation, on peut faire surexprimer Rad54 ou une protéine de la famille rad dans le but d'améliorer l'efficacité du processus de recombinaison.

### Longueur des fragments homologues

Quelques études réalisées dans des cellules souches embryonnaires de souris sur différents loci indiquent qu'une longueur minimum des bras 5' et 3 ' d'homologie est nécessaire pour assurer une recombinaison au locus avec une fréquence satisfaisante (Hasty et al., 1991; Thompson et al., 1989 ; Thomas and Capecchi, 1987). Si une taille minimale de 250-500 bp semble être suffisante pour assurer un événement de recombinaison d'un des deux cotés, le paramètre clef serait plus la longueur totale d'homologie que la taille respective des bras d'homologie (Elliott et al., 2001 ; Philips and Calos, 1999 ; Fujitani et al., 1995). Cette limite serait de 5 à 6 kb au minimum. L'extrapolation des résultats à partir des données très complètes obtenues chez les bactéries, pourrait indiquer que le facteur limitant dans une efficacité de recombinaison ne serait pas au niveau de la recherche des homologies entre les fragments d'ADN donneurs apportés par le vecteur et les parties d'ADN receveurs du locus à cibler (Yancey-Wrona and Caerini-Otero, 1995), mais plutôt dans le système d'échange entre ces fragments qui succède à l'appariement. Néanmoins, une grande prudence reste de mise quant à l'interprétation de ces résultats et leur extrapolation dans le système eucaryote, éventuellement sensibles à la présence de points facilitants ('hot spot') la recombinaison dans des loci particuliers.
Les loci d'immunoglobulines semblent à cet égard très particuliers. Des évènements d'intégration homologue d'un vecteur ne présentant une homologie que dans le bras en 3' ont été observés (Berinstein et al., 1992). Par ailleurs les recombinaisons dans les loci d'immunoglobulines apparaissent particulier en raison de l'activité des sites reconnus par les recombinases propres à ces gènes. Certaines interférences avec l'équipement de ces cellules et le processus de recombinaison pourraient être observées.

Ainsi le vecteur est un vecteur de recombinaison homologue possédant des bras 5' et 3' d'homologie aux séquences d'un locus donné. Le locus ciblé est sélectionné parmi le locus de l'ovalbumine et du lysozyme.

A ce titre le vecteur comporte des segments hétérologues :
Cette présence d'une partie importante homologue est d'autant plus nécessaire que les segments hétérologues sont importants (Kumar and Simons, 1993). Ainsi, l'approche isogénique (Te Riele et al., 1992) est renforcée par l'observation de baisse de fréquence de recombinaison homologue en cas d'interruption de long fragment d'homologies par des 'stretchs' variables, liés à la présence de polymorphisme d'un locus (Lukacsovich and Waldman, 1999). Mais la présence symétrique de segments hétérologues, situés en amont des éléments d'homologies propres peut également renforcer les niveaux d'intégration homologue du vecteur. Cette présence protégerait les homologies des dégradations induites par des exonucléases.

### Induction d'une coupure ciblée

Toute induction ciblée (par une méganucléase par exemple) d'une coupure double brin, coupure spécifique et rare dans le DNA de la cellule ciblée accroît les fréquences de recombinaison. Il semblerait que cette induction déclenche le système de réparation et facilite l'incorporation du DNA exogène (Bibikova et al., 2001; Sargent et al., 2000 ; Donoho et al., 1998 ; Sargent et al., 1997; Brenneman et al., 1996; Hasty et al., 1992b). Cette approche est utilisée notamment en introduisant un site spécifique reconnu par une méganucléase, notamment de type I-Scel ou autre, afin de favoriser les évènements de recombinaison (Sarig et al., 2000 ; Cohen-Tanoudji et al., 1998; Robine et al., 1998 ; Jasin et al., 1996).

### Linéarisation d'un vecteur

Des tests comparatifs entre vecteurs linéaire et circulaire ont été réalisés. Dans le système murin, il semble que la linéarisation soit un préalable nécessaire pour accroître les fréquences. D'autres actions de délivrance ont parfois une influence (Yanez et Porter, 1999).

### Structure conditionnelle des vecteurs d'expression

Les différents éléments présents actuellement dans les vecteurs sont actifs de façon constitutive une fois l'événement de recombinaison homologue réalisé. Or l'expression d'une protéine exogène dans l'oeuf peut entraîner une éventuelle toxicité à la fois au niveau du tissu qui va assurer la production et éventuellement au niveau de l'oeuf, lieu d'accumulation final. Cette accumulation doit donc pouvoir être induite et décidée tant au niveau temporel que spatial.
Une des approches possible est l'utilisation de systèmes inductibles et conditionnels.
Par système inductible on entend par exemple le système appelé Tet off/tet on. Ce dernier est constitué de deux éléments : le donneur et l'opérateur. Le système donneur comprend lui même une construction génétique dérivée d'un système de résistance à la tétracycline, identifiée dans un transposon bactérien Tn10. En l'absence de tétracycline, la protéine répresseur TetR, exprimé constitutivement bloque la transcription du gène assurant la résistance à la tétracycline. En présence de la tétracycline, le réprésseur ne peut plus se lier aux séquences de contrôle. Ce système a été mis à profit en fusionnant cette séquence tetR avec le domaine transactivateur de la protéine virale VP16 (Gossens and Bujard, 1992), brevet US N° 5, 589,362. Il en résulte les protéines tTA (pour Tetracycline controlled Trans Activator) pouvant agir sur le promoteur lui même modifié TetO (pour Tet Opérateur). Dans le cas des vecteurs décrits dans l'invention, un premier vecteur 'donneur' (vecteur simple ou ciblant par recombinaison un locus spécifique du tissu oviducte par exemple) est introduit dans la cellule souche et les éléments sensibles à l'action de la tétracycline sont placés au sein de la construction de recombinaison qui cible le locus de l'ovalbumine par exemple. Ces éléments sont insérés dans une région du promoteur ovalbumine pour contrôler au niveau transcriptionnel le niveau de protéine exprimé.
Parmi les autres systèmes inductibles on peut citer les fusions de ces différents acteurs (VP16, Ga14...) avec des séquences protéiques de contrôle comme certains récepteurs aux hormones nucléaires, plus ou moins modifiées pour répondre de façon spécifique à la présence d'analogue d'hormones (ER Muté liant le tamoxifène (Metzger et al.,1995 ; Indra et al., 1999), l'ecdysone Récepteur, le PR liant le RU486...).

Par système conditionnel on entend par exemple les systèmes mettant en oeuvre certaines enzymes comme certaines recombinases. Ces enzymes reconnaissent des petites séquences particulières et spécifiques (séquences loxP pour l'enzyme CRE, séquence FLP pour la FRT recombinase...) qui sont alors insérées dans la construction à contrôler. Le mode d'action général est de procéder à une délétion des éléments situés entre ces séquences lors de l'action de la recombinase. L'introduction de la recombinase est effectuée par différentes voies (transfection transitoire, expression stable intégrée...) et son expression conditionne la réaction d'excision au niveau des séquences introduites.
En combinant la présence de ces recombinases avec les systèmes inductibles par les récepteurs des hormones nucléaires, on rend l'ensemble du système conditionnel inductible. Ainsi, on peut par exemple fusionner 1a CRE recombinase avec la forme mutée du ER, sensible au tamoxifène. Ce système a été mis à profit dans le système murin (Metzger and Chambon, 2001; Vallier et al., 2001). D'autres systèmes ont été décrits pour le système murin comme le FLT-EcDR (Sawicki et al., 1998).

### Eléments de base des constructions

L'origine des éléments de base des vecteurs est variable. Les squelettes des principaux plasmides sont d'origine commerciale. Ils permettent une propagation facile des constructions qu'ils portent dans un environnement bactérien bien maîtrisé, celui de E. coli, même si différentes souches sont utilisées. Ainsi, les plasmides pBSK, pMCS5, pCI Néo sont utilisés pour être une base générale à beaucoup de vecteurs intermédiaires ou terminaux
Les cassettes de sélection positives sont constituées :
- d'un promoteur provenant de différentes origines, virales comme par exemple le promoteur CMV (cyomégalovirus), le promoteur du virus SV40, la LTR du RSV ou non virales comme des promoteurs de gènes ubiquitaires (comme le promoteur b-actine de poulet) ou des promoteurs de gènes spécifiquement exprimés à certaines phases de développement ou des promoteurs de gènes spécifiquement exprimés dans certains tissus. Un cas particulier est d'utiliser un promoteur spécifique de l'état de cellule souches.
- d'un gène codant pour une résistance 'positive' à un antibiotique comme par exemple le gène de résistance à la néomycine, à l'hygromycine, à la puromycine, à la phléomycine, à la zéomycine, à la blasticidine, à la viomycine mais aussi le gène DHFR (dihydrofolate reductase) assurant la résistance au methotrexate, le gène HPRT (Hypoxanthine phosphoribosyl transférase) assurant la transformation de bases spécifiques présentes dans le milieu de sélection HAT (aminoptérine, hypoxanthine, thymidine) ainsi que d'autres gènes assurant les détoxifications vis à vis de certaines drogues.
Les cassettes de sélection négatives sont constituées:
- d'un promoteur comme précédemment décrit
- d'un gène assurant la transformation d'un substrat présent dans le milieu de culture en une substance toxique pour la cellule qui exprime le gène. Parmi ces molécules, on peut citer les gènes de détoxification de la toxine diphtérique (DTA) (Yagi et al., 1998; Yanagawa et al., 1999), le gène de la thymidine kinase du virus Herpes (HSV TK), sensible à la présence de gancyclovir ou de FIAU. Le gène HPRT peut également être utilisé comme sélection négative par addition dans le milieu de la 6-thioguanine (6TG).
- et pour l'ensemble des sélections positives et négatives, d'une séquence poly A de terminaison de transcription de différentes origines dont les plus classiques sont celles dérivées du poly A de SV40 ou d'un poly A de gène eucaryote (hormone de croissance bovine, b-globine de lapin...).
- Un cas particulier est la mise de la cassette de sélection positive sous la dépendance directe du locus endogène ciblé. Cette approche requiert la fonctionnalité, donc l'activation transcriptionnelle du locus ciblé pour pouvoir être utilisée et donc de lier la sélection des clones après transfection à un état de différenciation ou de non différenciation particulier. Ces exemples ne sont pas limitatifs.

Ainsi, le vecteur de recombinaison homologue selon l'invention comprend dans une base plasmidique un enchaînement de au moins un élément pris successivement parmi
h) un fragment d'ADN génomique contenant le bras d'homologie 5' du gène ciblé fusionné à,
h) une séquence nucléotidique signal de sécrétion fusionnée à,
h) une courte séquence nucléotidique intronique fusionnée à,
h) la séquence nucléotidique codante pour la protéine d'intérêt fusionnée à,
h) une séquence poly A de terminaison de la transcription fusionnée à,
h) une cassette de sélection positive comprenant un promoteur, un gène de résistance à un agent de sélection et une séquence poly A de terminaison de transcription, ladite cassette pouvant être fusionnée à,
h) un fragment d'ADN génomique contenant le bras d'homologie 3' du gène ciblé,
h) une cassette de sélection négative comprenant un promoteur, un gène assurant la transformation d'un substrat présent dans le milieu de culture en une substance toxique pour la cellule qui exprime le gène et une séquence polyA de terminaison de transcription.

- Par promoteur, on entend toute séquence nucléotidique capable d'assurer et de diriger l'initiation de la transcription d'un gène placé en aval de cette séquence.
   Ce vecteur peut également comprendre d) la séquence codante pour la protéine exogène fusionnée en son extrémité 5' avec c) une courte séquence intronique en particulier comprenant la séquence SEQ ID N° 1 elle-même fusionnée avec b) une séquence peptide signal de sécrétion, en particulier la séquence codante pour le peptide signal du lysozyme comprenant la séquence SEQ ID N° 2.
   Le vecteur selon l'invention peut également comprendre d) la séquence codante pour la protéine exogène fusionnée en son extrémité 3' avec une séquence poly A.
   Le vecteur peut également comprendre au moins une séquence IRES en fusion avec au moins deux séquences codantes pour la protéine d'intérêt exogène ou au moins une séquence IRES en fusion avec au moins deux séquences codantes pour différentes chaînes constituant une protéine d'intérêt, en particulier les chaînes lourde et légère d'un anticorps de quelque nature que ce soit, en particulier un anticorps monoclonal, un fragment fab.
   Parmi les IRES, on choisit les IRES de groupe I ou de groupe II, en particulier les séquences V130 (Brevets US 5,925,565 ; WO 9601324), Idemfix et Zam (WO99/29844), (Leblanc et al. 1997 ; 1999).

### 1.2 Vecteur d'expression simple (Exemple Comparatif)

Ces vecteurs sont caractérisés par l'association successive d'un promoteur, d'un cDNA ou d'une partie d'un gène, d'une séquence poly A, permettant l'arrêt de la transcription. Après transfection de ces vecteurs dans des cellules eucaryotes, ces vecteurs présentent l'avantage et l'inconvénient de s'intégrer au hasard dans le génome. L'efficacité est souvent bonne et un grand nombre de sites d'intégration peuvent être obtenus. Cependant, le côté aléatoire de cette insertion rend l'expression à partir du promoteur porté par le vecteur dépendante de l'environnement du site d'insertion (méthylation, imprinting, enhancer, silencer...).

Ainsi, parmi les promoteurs utilisables répondant à ces difficultés et utilisés dans un but d'expression tissu spécifique, on peut mentionner de façon non exhaustive le promoteur du gène du lysozyme (de 2500 à 100 bp), mais aussi le promoteur du gène de l'ovalbumine, dans des formes longues (de 5 à 1 kb) contenant des régions de régulations positives et négatives ou courtes (de 1000 à 100 bp), sachant qu'un promoteur minimal de 100 bp environ peut être spécifiquement activé par différentes hormones (Monroe and Sanders, 2000).
L'exemple 1 (Exemple Comparatif) ci-après illustre les possibilités d'obtention de clones de cellules souches avec de tels vecteurs simples d'expression.

Le vecteur peut donc être un vecteur d'expression comprenant la séquence codante pour la protéine d'intérêt fusionnée avec au moins un élément pris parmi
a) un promoteur, en particulier sélectionné parmi les promoteurs des gènes de l'ovalbumine, des ovomucoïdes, de la conalbumine et du lysozyme,
b) une séquence peptide signal
c) une séquence nucléotidique polyA de terminaison de transcription.

Ce vecteur d'expression et le vecteur de recombinaison peuvent comprendre une séquence IRES fusionnée avec au moins deux séquences codantes pour la même protéine d'intérêt ou pour des séquences codantes différentes.

Les vecteurs précités permettent de transformer une cellule aviaire telle que définie précédemment.
Avantageusement, ladite cellule est une cellule aviaire embryonnaire primaire, une cellule aviaire embryonnaire souche, en particulier les cellules embryonnaires souches issues de la mise en culture de blastodennes.
- par blastoderme, on entend un embryon aviaire à un stade précédant la gastrulation et comportant une organisation cellulaire simple avec au moins deux couches de cellules séparées par une cavité.

La cellule embryonnaire aviaire de l'invention présente un phénotype de cellule souche embryonnaire alcaline phosphatase positive.
Les cellules embryonnaires aviaires, les cellules souches embryonnaires et les cellules germinales embryonnaires se caractérisent en ce qu'elles réagissent spécifiquement avec au moins un anticorps sélectionné parmi ECMA-7, SSEA-1, SSEA-3, TEC-01, EMA-1 et EMA-6.

Dans un mode particulier de réalisation, la cellule est une cellule aviaire dérivée des cellules souches embryonnaires induites à différencier sous l'action de différents agents inducteurs en particulier l'acide rétinoïque, le diméthylsulfoxide, le TPA ou de conditions de cultures spécifiques, en particulier par la formation de corps embryoïdes (voir définition ci-dessus).

Alternativement, dans ces exemples comparatifs la cellule est une cellule aviaire établie en lignée, en particulier les cellules hépatiques LMH, les cellules monocytes HD11 et les cellules fibroblastiques QT6.

Comme évoqué précédemment, ladite cellule peut être transformée avec un vecteur d'expression exprimant une protéine de la famille Rad, en particulier la protéine Rad54.

Ainsi, dans un deuxième aspect, l'invention se rapporte à un procédé d'obtention d'une cellule aviaire modifiée par un des vecteurs définis ci-dessus.
Ce procédé peut comprendre les étapes suivantes
a) introduction du vecteur défini ci-dessus dans une cellule embryonnaire aviaire par une méthode de transfection, en particulier à l'aide d'un liposome, d'un polycation ou par électroporation
b) sélection des cellules par l'addition d'un agent de sélection dans le milieu de culture, en particulier la généticine dans une gamme de concentration de 100 à 500 µg/ml
c) criblage des clones résistants et amplification.
De préférence, cette sélection est réalisée pendant au moins environ 2 à 10 jours, notamment 2, 3, 4, 5 ou 6 jours.

Avantageusement, l'invention se rapporte à un procédé d'obtention d'une cellule souche embryonnaire aviaire modifiée par un des vecteurs définis ci-dessus comprenant les étapes suivantes :
a) introduction du vecteur de recombinaison défini selon l'une des revendications ci-dessus dans une cellule embryonnaire souche aviaire par une méthode de transfection, en particulier à l'aide d'un liposome, d'un polycation ou par électroporation
b) sélection des cellules par l'addition d'un agent de sélection dans le milieu de culture, en particulier la généticine dans une gamme de concentration de 100 à 500 µg/ml
c) obtention de clones recombinés génétiquement stables présentant une morphologie et des caractéristiques similaires aux cellules parentales (taux d'alcaline phosphatase endogène, réactivité à des anticorps de surface, et activité télomérase)
d) criblage des clones résistants et amplification des cellules résistantes résultantes correctement recombinées.

Dans un aspect particulier, le vecteur de recombinaison cible le locus du lysozyme.

L'invention a également trait à un procédé mentionné précédemment caractérisé en ce que le surnageant de culture des clones recombinés contient la protéine exogène d'intérêt, en particulier après induction du clone à l'aide de différents inducteurs,en particulier l'acide rétinoïque, le diméthylsulfoxide, le TPA ou de conditions de cultures spécifiques, en particulier par la formation de corps embryoïdes.

Dans ce procédé, les deux allèles du locus ciblé sont de préférence modifiés.
De plus, les cellules sont des cellules aviaires embryonnaires souches, en particulier les cellules embryonnaires souches issues de la mise en culture de blastodermes. Ces cellules présentent un phénotype de cellule souche embryonnaire alcaline phosphatase positive.
Les cellules embryonnaires aviaires du procédé se caractérisent en ce qu'elles réagissent spécifiquement avec au moins un anticorps sélectionné parmi ECMA-7, SSEA-1, SSEA-3, TEC-01, EMA-1 et EMA-6.

L'invention se rapporte également à un procédé défini ci-dessus dans lequel les cellules sont des cellules souches embryonnaires induites à différencier sous l'action de différents agents inducteurs en particulier l'acide rétinoïque, le diméthylsulfoxide, le TPA ou de conditions de cultures spécifiques, en particulier par la formation de corps embryoïdes.

Lesdites cellules définies ci-dessus peuvent être transformées en outre avec un vecteur d'expression exprimant une protéine de la famille Rad, en particulier la protéine Rad54.

Pour ce procédé, le milieu utilisé peut comprendre des anticorps anti-acide rétinoïque (ARMA) et une cytokine choisie dans le groupe constitué de LIF, IL-11, IL-6 et leurs différents mélanges.
Le milieu utilisé peut également comprendre différents facteurs en particulier le SCF, l'IGF-1, le bFGF, le CNTF et l'Oncostatine.

Dans un troisième aspect, l'invention se rapporte à un procédé d'obtention d'un animal appartenant à l'espèce aviaire capable d'exprimer une protéine exogène d'intérêt, caractérisé en ce qu'il comprend les étapes suivantes :
a) obtention de cellules aviaires modifiées par le procédé défini ci-dessus,
b) introduction de la cellule obtenue à l'étape a) dans la cavité sub-germinale d'un embryon, ou dans la circulation sanguine de l'embryon, et
c) incubation de l'embryon obtenu à l'étape b).

De préférence, le vecteur utilisé à l'étape a) permet une expression tissu-spécifique, en particulier dans l'oviducte, le foie, le sang, la moelle et les organes lymphoïdes.
- Par moelle, on entend le tissu qui rempli la cavité de la plupart des os et qui contient les cellules souches hématopoiétiques, à partir desquelles dérivent toutes les cellules rouges et blanches du sang.

L'invention porte également sur le procédé précité pour obtenir un animal appartenant à l'espèce aviaire présentant une expression tissu-spécifique d'une protéine exogène d'intérêt, caractérisé en ce que le vecteur est un vecteur de recombinaison homologue possédant parmi différents éléments constitutifs nécessaires à son fonctionnement des bras 5' et 3' d'homologie aux séquences d'un locus sélectionné parmi le locus de l'ovalbumine, et du lysozyme.
Un tel vecteur peut comprendre la séquence codante pour la protéine exogène fusionnée avec au moins un élément sélectionné parmi une séquence intronique, une séquence peptide signal de sécrétion, en particulier le peptide signal du lysozyme comprenant la séquence SEQ ID No 2, une séquence poly A, un IRES et un promoteur, en particulier choisi parmi les promoteurs des gènes de l'ovalbumine, des ovomucoïdes, de la conalbumine et du lysozyme.

L'étape b) du procédé pour obtenir un animal appartenant à l'espèce aviaire présentant une expression tissu-spécifique d'une protéine exogène d'intérêt peut comprendre en outre la transformation des cellules aviaires avec un vecteur exprimant une protéine de la famille Rad, en particulier Rad54.

Dans un aspect supplémentaire, l'invention se rapporte à un procédé de production d'une protéine d'intérêt comprenant l'extraction de la protéine exogène exprimée dans les tissus d'un animal obtenu à partir du procédé évoqué ci-dessus. Dans ce procédé, la protéine est de préférence extraite du sang, du jaune ou du blanc d'oeuf.

Alternativement, le procédé de production d'une protéine d'intérêt peut consister en l'extraction de la protéine exogène exprimée dans le surnageant des cellules issues du procédé selon l'invention.

L'invention a également trait à un animal appartenant à l'espèce aviaire susceptible d'être obtenu à partir du procédé décrit ci-dessus, caractérisé en ce qu'il exprime une protéine exogène dans un tissu spécifique, par exemple dans le foie, le sang, la moelle, les organes lymphoïdes ou l'oviducte.

Dans un autre aspect, l'invention vise un oeuf susceptible d'être obtenu à partir d'un animal décrit ci-dessus, caractérisé en ce qu'une partie de ces composants, en particulier l'ovalbumine et le lysozyme soit partiellement ou totalement remplacée par une protéine d'intérêt exogène, sélectionnée notamment parmi les peptides à intérêt thérapeutique, les interleukines, les cytokines, les hormones, et les anticorps.
- Par anticorps, on entend une protéine présentant une affinité spécifique pour un antigène comprenant les anticorps polyclonaux, monoclonaux et leur fragment Fab.

L'oeuf de l'invention peut comporter une proportion de protéine exogène comprise entre quelques mg (1 à 10 mg) et 500 mg de matières sèches en lieu et place d'une partie ou de la totalité d'au moins une protéine endogène, choisie notamment parmi l'ovalbumine et le lysozyme.

Des exemples non limitatifs des modes de réalisations de l'invention sont donnés ci-après

### Abbréviations

cDNA : complementary DNA
CMV : Cytomégalovirus
DNA : Desoxyribonucleic Acid
ER : Estrogene Receptor
FGF : Fibroblast Growth Factor
GFP : Green Fluorescent Protein
GR : Glucocorticoïd Receptor
HRE : Hormone Responsive Element
IGF-1 : Insulin Growth factor 1
LPS : Lipopolysaccharides
LTR : Long Terminal Repeat
MAR : Matrix Attachment Region
MCSF : Macrophage Colony Stimulating Factor
NRE : Negative Response Element
PR : Progesterone Receptor
RAR : Retinoic Acid Receptor
RNA : Ribonucleic Acid
RSV : Rous Sarcoma Virus
RXR : Retinoïd X Receptor
SCF : Stem cell Growth factor
SDRE : Steroid Dependent Regulatory Element
TPB: Tryptose Broth Phosphate
TPA: TriPhorbol Ester
TR : Thyroid hormone Receptor
VDR : Vitamin D Receptor

### Exemple 1 : L'oeuf comme système de production

L'expression d'une protéine d'intérêt dans un liquide physiologique d'un animal, le poulet par exemple, notamment dans l'oeuf et en particulier dans le blanc d'oeuf apparaît réalisable avec l'aide de différents outils moléculaires dont les vecteurs d'expression. Le principe général de l'invention est de faire exprimer directement dans l'oeuf une molécule exogène d'intérêt en lieu et place d'une molécule endogène ou d'une fraction de cette molécule endogène.

### 1.1 Les protéines du blanc

L'oeuf est un milieu particulièrement adapté pour l'expression de molécules exogènes. Le blanc d'oeuf est un milieu complexe dont la composition biochimique est assez bien caractérisée (voir Tableau 1 ci-après).

**Tableau 1 : Teneur moyenne (en g) pour un oeuf de 60g**

| | OEuf entier | blanc | Jaune |
|---|---|---|---|
| Eau | 40 | 31 | 9 |
| Matière sèche | 20 | 4 | 10 |
| Protéines | 6,9 | 3,6 | 3,2 |
| Lipides | 6,4 | <0,2 | 6,4 |
| glucides | 0,2 | 0,2 | < 0,1 |

D'après Sauveur, 1988 op cité

Pauvre en lipides (0,02%), en ions inorganiques et en glucides (0,5% dont du glucose libre), le blanc est composé principalement d'eau à 88 %, et de protéines en solution (11,5%). Les protéines du blanc d'oeuf sont bien caractérisées dans leur ensemble, même si leur nombre exact est encore soumis à variation (Stevens, 1991; Li-Chan and Nakai, 1989; Sauveur, 1988). On s'accorde en général pour avoir identifié environ 40 protéines différentes. Parmi celles-ci, on distingue cinq protéines majoritaires (ovalbumine, conalbumine, ovomucoïde, ovomucine a and b et le lyzozyme) qui représentent à elles seules près de 83-84% des protéines du blanc. Au regard de cette composition et d'un poids moyen de matière sèche de 6-7 g environ, ces protéines majoritaires représentent donc l'équivalent de 4,8-5,6 g environ. Les protéines mineures, mais significativement détectables représentent de 5 à 6% des protéines totales. Parmi ces composants minoritaires, on peut citer l'avidine, très connue dans le monde du diagnostic pour sa très haute affinité et sa très grande spécificité vis à vis de la biotine. Les autres composantes sont identifiées dans des proportions faibles, variables d'une préparation à une autre et sont souvent mal caractérisées tant au niveau biochimique que moléculaire. Le nombre d'études les concernant est d'ailleurs peu important en accord avec leur faible représentativité. Les propriétés physicochimiques des protéines majoritaires du blanc d'oeuf sont présentées (voir Tableau 2 ci-dessous).

**Tableau 2 : Composition protéique du blanc d'oeuf de poule**

| Protein | % of protein | MW | pI | % carb | Function | AA | S-S |
|---|---|---|---|---|---|---|---|
| Ovalbumin | 54 | 45.0 | 4.5 | 3.5 | Structural | 385 | 1 |
| Conalbumin | 12-13 | 77.7 | 6.0 | 2.6 | Iron transport | 686 | 15 |
| Ovomucoïd | 11 | 28.0 | 4.1 | 16 | Proteinase inibitor | 185 | 9 |
| Ovomucin a Ovomucin b | 1.5-3.5 | 210 720 | 4.5 | 13 58 | Structural | 1276 246 | |
| Lysozyme | 3.5 | 14.3 | 10.7 | 0 | Proteolytic enzyme | 129 | 4 |
| Ovoinhibitor | 0.5-1.5 | 49.0 | 5.1 | 5-9.6 | Proteinase inibitor | | 21 |
| OvoGlycoprotein | 0.5-1.0 | 24.4 | 3.9 | | ? | | |
| Ovoflavoprotein | 0.8 | 32.0 | 4.0 | 11 | Riboflavin transport | 234 | 9 |
| Ovostatin | 0.5 | 780 | 4.9 | 5.8 | Proteinase inibitor | 2762 | |
| OvoGlobulin G2 | >1 | 47 | 4.9 | ? | ? | 102 | |
| OvoGlobulin G3 | >1 | 50 | 4.8 | ? | ? | 103 | |
| Cystatin | 0.05 | 12.7 | 5.1 | 0 | Proteinase inibitor | 124 | 2 |
| Avidin | 0.05 | 68.3 | 10 | 7 | Biotin transport | 4x129 | 4x1 |
| Thiamin binding protein | <0.05 | 38 | | 0 | Thiamin transport | | |
| Autres protéines | 9 | | | | | | |

D'après : Stevens (1991), Comp. Bioch. Physiol 100b 1-9 and Li-Chan and Nakai (1989), Critical review in Poultry Biology 2, 21-58.

Les protéines du blanc sont toutes élaborées par les cellules de l'oviducte, au niveau du magnum. Différents types cellulaires sont trouvés et une certaine spécialisation est observée entre les cellules, responsables de la secrétion. Les cellules épithéliales calciformes (appelées également cellules à mucus) sont spécialisées dans la production de l'avidine et de l'ovomucine, tandis que les cellules des glandes tubulaires secrètent préférentiellement le lysozyme et l'ovalbumine. La répartition de ces différents types cellulaires est variable dans l'épithélium sécrétoire du magnum, mais aucune régionalisation n'est observée (Sauveur, 1988).
La synthèse des protéines du blanc est continue au niveau des cellules. Les cellules épithéliales glandulaires et calciformes 'stockent' les protéines, qui sont déchargées autour du jaune lors du transit dans le magnum en quelques heures (environ 3h30). La déformation mécanique induit cette sécrétion et ce dépôt très rapide (Sauveur, 1988).

Ainsi, il apparaît essentiel pour le bon déroulement de la sécrétion des protéines exogènes dans le blanc de l'oeuf de maintenir tous ces signaux de régulation, au niveau moléculaire et physique.

Au niveau d'un animal chimérique, le préalable à toute production d'une molécule exogène d'intérêt est la présence de la modification génétique dans l'oviducte et dans les cellules du magnum en particulier. L'approche privilégiée de l'utilisation des cellules souches embryonnaires semble permettre ce mosaïcisme. Au niveau de l'établissement d'une lignée d'animaux, cette contribution est transmise de façon automatique à l'état hétérozygote puis homozygote par les croisements adéquats, car intégrée au niveau du génome.

### 1.2 Les composants du jaune

Le jaune d'oeuf est essentiellement composé d'une accumulation de lipides (Tableau 1 ci-dessus) sous forme de lipoprotéines. Il est le résultat d'une étroite association des deux protéines majoritaires, la vitelline et la vitellenine, avec des phospholipides et des triglycérides. Les autres composants (cholestérol, vitamines et pigments liposolubles) sont minoritaires. A contrario des protéines du blanc, toutes les protéines et les composants du jaune sont synthétisés par le foie et transportés via la circulation sanguine pour s'accumuler au niveau du jaune lors du développement des follicules (Sauveur, 1988 ; Nau, 1987). On trouve également dans le jaune une proportion non négligeable d'immunoglobulines qui sont accumulées directement au cours de l'élaboration de ce vitellus. La présence de ces immunoglobulines d'origine maternelles assure d'ailleurs une certaine protection immunitaire au cours des premiers jours, voir plus, de la vie du poussin. Le mécanisme de transport de ces immunoglobulines et leur accumulation dans l'oeuf commence à être étudié. Le processus de purification de ces molécules IgY spécifiques de l'oiseau (équivalentes aux IgG des mammifères) est bien maîtrisé. Cette propriété peut être mise à profit pour obtenir des immunoglobulines aviaires en quantité relativement importante (parfois de l'ordre de 10 mg par oeuf), voir d'en favoriser l'accumulation par une immunisation ciblée de l'animal en ponte. Il a été par ailleurs montré que les immunoglobulines humaines peuvent également s'accumuler dans le jaune, par un mécanisme similaire aux immunoglobulines endogènes (Mohammed et al., 1998). Dans cette optique de faire produire par la poule des immunoglobulines particulières, une stratégie d'expression spécifique peut être développée.

### Exemple 2 : Description des loci moléculaires et leur régulation transcriptionnelle

La plupart des gènes qui spécifient les protéines majoritaires du blanc d'oeuf ont été étudiés au niveau moléculaire. Les phases codantes sont en général identifiées et la plupart des séquences des cDNAs correspondants se trouvent dans les banques de données. Au niveau génomique, les progrès ont été plus lents. La structure des loci de la plupart des molécules majoritaires a été publiée complètement ou partiellement. Néanmoins, les séquences publiées peuvent se révéler incomplètes (voir Tableau 3 ci-dessous).

**Tableau 3 : Séquences identifiées des protéines du blanc d'oeuf**

| | cDNA | gene | gene (partiel) |
|---|---|---|---|
| Ovalbumin | V00383 | J00895 | |
| Ovalbumin X | | | J00917 5' ex1, J00918 ex5, |
| | | | J00919 ex6, J00920 ex7 |
| Ovalbumin Y | | J00922 | |
| Conalbumin | X02009 | Y00407 | |
| Ovomucoid | J00902 | | J00897 (5'), J00898 |
| | | | (3' coding) |
| | | | J00899 (3' non cod) |
| Ovomucin □ | AB046524 | | |
| Lysozyme | X61198 | X98408 | J00882 5' ex1 |
| | | X00589 | J00883 ex2 |
| | | X00591 | J00884 ex3 |
| | | X05461 | J00885 ex4 3' |
| | | X05463 | |
| | | X05462 | |
| Ovoinhibitor | | | M 15962 ex1, M 16127 ex2, |
| (16 exons) | | | M16128 ex3 |
| | | | M16129 ex4, M16130 ex5, |
| | | | M16131 ex6 |
| | | | M16132 ex7, M16133 ex8, |
| | | | M16134 ex9 |
| | | | M16135 ex10, M16136 ex11, |
| | | | M16137 ex12 |
| | | | M16138 ex13, M16139 ex14, |
| | | | M16140 ex15 |
| | | | M16141 ex16 |
| Ovostatin | X78801 | | |
| Cystatin | J05077 | M95725 | X62413 (synthetic mut V55D) |
| | | | X62411 (synthetic mut Q53N S56A) |
| | | | X62412 (synthetic mut R52L Q53E) |
| | | | X62409 (synthetic mut Q53N) |
| | | | X62408 (synthetic mut Q53E) |
| | | | X62407 (synthetic mut L54M G57A) |
| | | | X62410 (synthetic mut Q53N G57C) |
| | | | X62406 (synthetic mut G57A) X14685 (1-23 S1M 129 L89) |
| Avidin (Avd) | X05343 | AJ311647 | |
| Avr1 | | AJ311647 | Z21611 |
| Avr2 | | AJ311648 | Z21554 |
| Avr3 | | | Z21612 |
| Avr4 | | | Z22883 |
| Avr5 | | | Z22882 |
| Avr6 | | AJ237658 | |
| Avr7 | | AJ237659 | |

### 2.1 Locus de l'ovalbumine

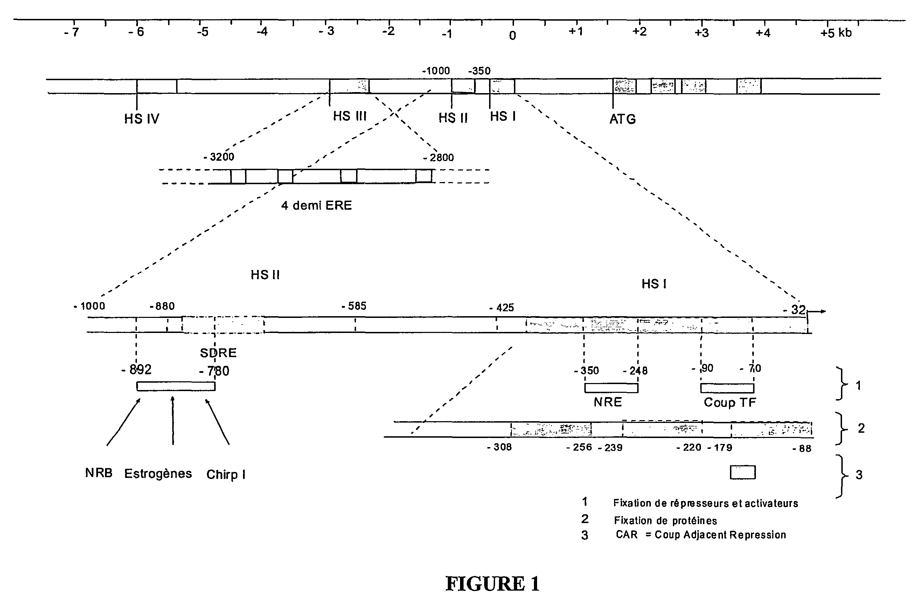

Le locus de l'ovalbumine d'une taille de 100 kb appartient à la famille multigénique des serpines et contient 3 gènes apparentés, ova, ovaX et ovaY. Ils sont tous exprimés dans l'oviducte d'une poule en ponte (LeMeur et al., 1981; Baldacci et al., 1981). Connu structurellement depuis 1979 par des études de microscopie électronique (Gannon et al, 1979), le locus de l'ovalbumine est situé sur le chromosome 2 (Dominguez-Steglich et al., 1992). La première séquence complète qui a été publiée en 1978 est celle de l'ovalbumine. Son pré-mRNA a une taille de 7564 bp et comprend 7 exons (McReynolds et al., 1978). Le messager épissé fait 1872 bp (Woo et al., 1981).
La transcription du gène codant pour l'ovalbumine (comme celui de la conalbumine d'ailleurs- cf2.3) est contrôlée par les hormones stéroïdiennes. Ainsi, l'addition d'estrogènes stimule de 20 fois la production d'ovalbumine et de 2,5 fois celle de conalbumine (N' guyen et al. 1979). Avant stimulation hormonale, les transcrits de l'ovalbumine ne sont pas détectables dans les cellules de l'oviducte, alors qu'ils représentent, proportionnellement à la dose d'estrogène, plus de 50% des transcrits totaux d'une cellule après stimulation maximale (Dean et al., 1983). Si le taux de transcription de la conalbumine est directement proportionnel à la quantité des récepteurs aux estrogènes dans le noyau, celui de l'ovalbumine indique qu'il existe plusieurs sites de fixation des récepteurs aux estrogènes au niveau du promoteur (Palmiter et al, 1981). La structure nucléosomique des gènes de l'ovalbumine et de la conalbumiune se modifie au cours de l'activation transcriptionnelle. Les fragments génomiques s'associent à la matrice nucléaire et présentent quatre régions majeures d'hypersensibilités à la DNAse, suite au traitement des hormones (Ciejek et al., 1983). Ces régions sont localisées dans la région 5' du promoteur et en 3' du dernier exon au niveau du site de polyadénylation (Bellard et al., 1982 ; Bellard et al., 1986) (Figure 1 : Eléments de régulations du gène de l'Ovalbumine). En association avec les estrogènes, différentes hormones stéroïdes (progestérone, glucocorticoïdes ou androgène) et différents facteurs (Insuline, IGF-1, cAMP, ...) sont également impliqués dans le contrôle transcriptionnel. L'ensemble de ces facteurs augmente surtout la demi vie du mRNA de l'ovalbumine, demi- vie qui passe de 6h à 24 h après addition de certains de ces facteurs (Arao et al., 1996; Kida et al., 1995; Evans and McKnight, 1984). Ces facteurs augmentent également le taux de traduction du messager (Skafar et al., 1985; Skoufos and Sanders, 1992).
Les résultats des nombreux travaux réalisés avec le promoteur de l'ovalbumine ont permis de dégager les différentes régions nucléotidiques cibles de l'action de ces hormones et facteurs (Sensenbaugh and Sanders, 1999). Des travaux de délétions successives au niveau du promoteur et des expériences de transfection de différentes constructions montrent que l'activation du promoteur est optimale dans les cellules primaires d'oviducte de poule, en présence ou non d'estrogènes. La présence d'activateurs et de répresseurs tissus spécifique est donc essentielle pour l'expression de ce gène (Dierich et al., 1987). La Figure 1 illustre les principaux sites de régulations identifiés.
On trouve ainsi successivement une région HSIV vers -6000 bp par rapport au site d'initiation de la transcription qui ne présente pas d'éléments de réponse aux estrogènes. Une région HS III, localisée entre -3200/-2800 bp par rapport au site d'initiation de la transcription comprend les sites majeurs de régulations par les estrogènes. La délétion de cette région abolie la plus grande partie de la réponse HSIII, médiée par quatre séquences répétées de demi ERE, séparées les unes des autres par une centaine de nucléotides. Ces éléments coopèrent avec le site TATA box, situé à proximité. Chaque site fixe faiblement un récepteur, mais la coopération des différents sites assure un caractère inductible fort, dans un environnement de promoteur minimum ou hétérologue. Cette même région montre des éléments inhibiteurs de l'expression du gène, éléments actifs dans les cellules non productrices, mais dans une situation hors de l'oviducte (Kato et al., 1992; Muramatsu et al., 1995).
La région HS II comprend le SDRE (Steroid Dependent Regulatory Element), localisée entre - 800 à -585 bp, par rapport au site (+1), de démarrage de la transcription. Cette région présente des homologies avec le promoteur du gène NF-kappa B, suggérant des contrôles par des facteurs communs (Schweers et Sanders,, 1991). Plusieurs facteurs de la famille des protéines W-H, les protéines Chirp-I, Chirp II, Chirp III (Chicken ovalbumin Induced Regulatory Protein I) se fixent également sur le SDRE uniquement en présence d'estrogène et de glucocorticoïdes (Dean et al., 1996 ; Dean et al., 1998; Dean et al., 2001). A nouveau, comme pour montrer la complexité des régulations mises en jeu, la seule présence de cette région SDRE au sein d'un promoteur hétérologue ne conduit pas à une réponse dépendante des hormones stéroïdes. Toutefois, cette région devient dépendante de ces hormones, si elle est associée avec la région HSI.
Cette région HS I, localisée de -350 à -32, est la région NRE (Negative Response Element) qui contrôle la principale régulation négative du promoteur. NRE réprime l'expression du gène en absence d'hormones stéroïdiennes. La présence de NRE seul ou de SDRE seul ne suffit pas pour réguler un gène exogène. Ces séquences doivent coopérer pour 'induire la dérepression' (Hasty et al., 1994 ; Schweers et al., 1990; Sanders et McKnigth, 1988). Mais la vision est simpliste, car cette région HSI est en fait constituée d'une véritable mosaïque de sites assurant une régulation positive et négative de la transcription du gène. Trois domaines sont discernables quant à l'influence de leurs effets. Domaine I : La région -239 à -220 est une séquence de type silenceur. Les trois zones présentent de l'affinité pour des complexes protéiques spécifiques des cellules de l'oviducte mais les plus importantes sont les régions -280 à -252 et -134 à-88. Cette région porte le motif CAR (COUP Adjacent repression). La région appelée NRE est en fait plus complexe qu'initialement décrite (Haecker et al., 1995). Un site de régulation positif qui fixe le facteur delta EF1 (Estrogen responsive transcription factor) a aussi été identifié. Ainsi, les sites d'interaction (-197 à -95) de la progestérone et des estrogènes se chevauchent partiellement (Dean et al., 1983; Dean et al., 1984). En aval de la position de - 95 bp, des délétions supplémentaires font chuter dramatiquement les taux des transcrits observés (Knoll et al., 1983). Outre les récepteurs aux estrogènes, un des facteurs régulateurs important est le récepteur orphelin COUP-TF, (Chicken Ovalbumin Upstream Promoter Transcription Factor) (Sawaya et al., 1996 ; Robinson et al., 1999). Ce facteur est exprimé de façon ubiquitaire dans tous les tissus. Identifié par co-purification avec le récepteur aux Estrogènes, il présente de l'affinité avec une région située entre -90 et -70 bp (Wang et al., 1987; Hwung et al., 1988a, Hwung et al., 1988b; Monroe and Sanders, 2000). La formation de complexes récepteur-récepteur entre COUP-TF et les différents récepteurs aux hormones nucléaires (VDR, ER, TR, GR, PR, RXR, HNF-4...) au niveau des différents HRE, dont le ERE, favorise les fixations à ces éléments de régulations. COUP-TF régule l'action de ER par des contacts directs avec le DNA mais aussi via des interactions directes protéines - protéines entre récepteurs (Klinge et al., 1997). Les modes d'action de COUP-TF et des différents récepteurs apparentés (COUP-TFII ou ARPI) sont complexes (Kimura et al., 1993) dans un sens positif ou négatif pour la régulation de la transcription selon les partenaires protéiques en jeu. Parmi les protéines impliquées, on peut citer de façon non exhaustive ear2, (Pereira et al., 1995) ; N-CoR (Nuclear Repressor Coreceptor) et SMRT (Silencing Mediator for retinoic acid et Thyroid hormone Receptor), (Shibata et al., 1997).

### 2.2 Le locus du lysozyme

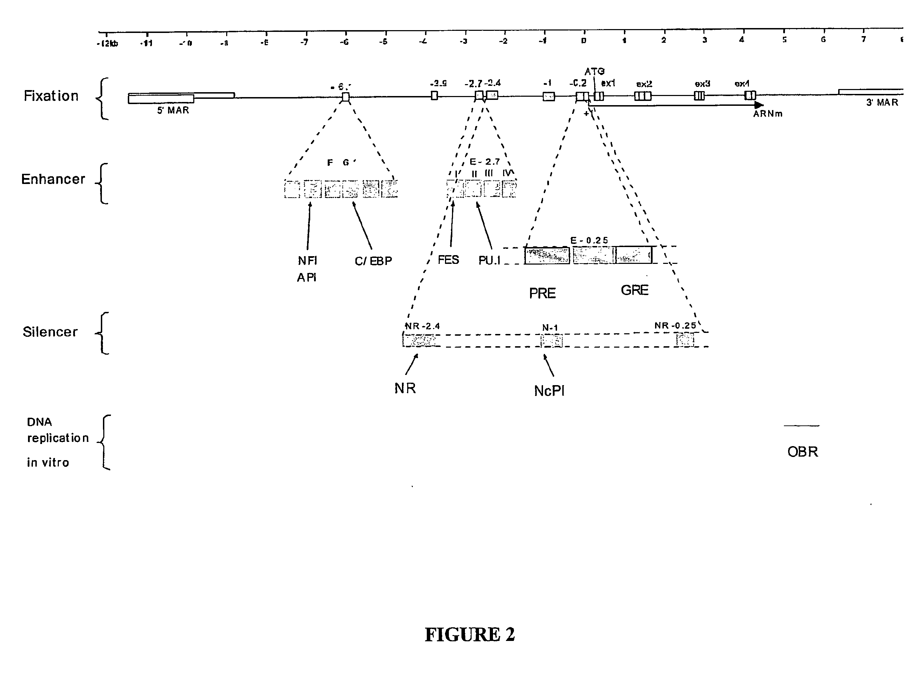

Le locus du lysozyme est un locus plus restreint en taille que celui de l'ovalbumine. Il est estimé à environ 40 kb (Sippel et al., 1978 ; Short et al., 1996). Deux unités MAR (Matrix Attachment Region) situées à -11.1 kb / -8.85 kb et +1.3 kb à +5.0 kb, situées par rapport au site (+1) de l'initiation de la transcription délimitent plus précisément ce locus à 22 kb. Ces séquences MAR sont composées de plusieurs sites d'accrochage à la matrice protéique de la membrane nucléaire (Loc and Strätling, 1988; Phi-van and Strätling, 1996 ; Phi-van, 1996). L'ajout de séquences MAR, à des gènes hétérologues, augmente leur transcription (Stief et al., 1989; Phi-Van et al., 1990). En sus de la présence des MAR, il y au moins une origine de réplication du DNA dans le locus du lysozyme, origine qui contient plusieurs fourches d'intiation de la réplication (Bonifer et al., 1997; Phi-van et al., 1998 ; Phi-van and Strätling, 1999). Ce point semble d'ailleurs rendre le locus du lysozyme tout à fait particulier en terme de clonage, certaines parties étant assez difficile à sous cloner dans des plasmides de clonage. Le gène du lysozyme chez le poulet comprend 4 exons et présente une homologie importante avec les gènes du lysozyme humain et murin. Plusieurs sites secondaires initiateurs de la transcription ont été mis en évidence, en général situés dans des séquences répétées réparties sur l'ensemble du locus (Von Kries et StrÄtling, 1988). Des séquences régulatrices de l'expression ont été identifiées de -14 kb à + 6 kb, soit sur toute la totalité du locus. Le gène du lysozyme est exprimé de façon constitutive dans les macrophages matures, cellules dans lesquelles le lysozyme est utilisé comme marqueur de différenciation, alors que son expression est placée sous le contrôle des hormones stéroïdiennes dans les cellules de l'oviducte (Short et al., 1996 ; Fritton et al., 1987 ; Jantzen et al., 1986 ; Fritton et al., 1983). Ces spécificités d'expression sont dépendantes de différentes séquences dont les principaux sites sont portés sur la figure 2 : Eléments de régulations du gène du lyzosyme.

On distingue deux catégories de séquences régulatrices : les séquences de régulation dites positives ou 'enhancers' et les séquences de régulations dites négatives ou 'silencers'. Ces séquences ont été identifiées comme les régions 'hypersensibles' suite à l'analyse des profils électrophorétiques du DNA, profils obtenus après traitement à la DNAse I.

Les principaux sites enhancers et silencers sont respectivement les régions E-6.1kb, E-2.7kb, E-0.2kb et N-2.4kb, N-1.0kb et N-0.25kb. Certains sont spécifiques des cellules myéloïdes (E-6.1 kb, E-2.7kb, E-0.2kb pour les macrophages matures et N-2.4 kb dans les fibroblastes et les macrophages immatures), (Steiner et al., 1987; Huber et al., 1995). Dans les cellules de l'oviducte actif l'élément E -6.1 kb assure l'essentiel de la spécificité. L'activation séquentielle de l'ensemble de ces différents sites est également un élément de régulation cellule et tissu spécifique (Regenhard et al., 2001 ; Kontaraki et al., 2000). Plus en détail, l'enhancer E-2.7kb est composé de 4 régions (I à IV). La région I fixe le facteur FEF (c-fes Expression Factor), la région II contient un site de reconnaissance du facteur PU.1, de la famille EST et est spécifique des macrophages (Ahne et Strätling, 1994). Tous ces différents éléments de régulations se chevauchent partiellement, au moins dans le gène du poulet, mais pas au niveau du gène de la souris, espèce dans laquelle une duplication ancestrale a séparé le gène et ses éléments de régulation. D'autres éléments ont été localisés, notamment les sites de fixation de facteurs de transcription ubiquitaires comme NF-1, mais aussi AP1 situé en - 0.2 kb et-6.1 kb, site pour lequel une région de 157 bp assure la spécificité d'expression dans les macrophages (Goethe et al., 1994 ; Theisen et al., 1986; Grewal et al., 1992; Nowock et Sippel, 1982). De façon intéressante, l'oncogène v-myc (présent dans les cellules HD11 par exemple) et inhibiteur de la différentiation des monocytes en macrophages bloque la transcription en inhibant la fixation du facteur C/EBP (Mink et al., 1996).
Les éléments (HRE) assurant la sensibilité de la transcription aux hormones stéroïdiennes (PRE pour la progestérone, et GRE glucocorticoïdes) sont présents dans la région située à - 0.2 kb du site (+1), d'initiation de la transcription du promoteur (Renkawitz et al., 1982 ; Renkawitz et al., 1984a ; Renkawitz et al., 1984b; Hecht et al., 1988). Plus particulièrement, deux sites entre -220 et - 140 bp et entre - 80 à - 50 bp assurent l'essentiel de la régulation (Altschmied et al., 1989; Dolle and Strätling, 1990; Von der Ahe et al., 1986).

Une part importante de la régulation est contrôlée par les 'silencers'. Deux d'entre eux (N-1.0kb et N-0.25 kb) inhibent la transcription, même de promoteurs hétérologues (Baniahmad et al., 1987 ; Faust et al., 1999). Le troisième (N-2.4 kb) est reconnu par les récepteurs aux hormones thyroïdiennes (TR) et par son homologue oncogénique v-erbA, ainsi que par le régulateur NeP1 (negative Protein 1) encore appelé CTCF (CCCTC-binding factor, (Burcin et al., 1997; Darling et al., 1993; Kohne et al., 1993 ; Bhat et al., 1994). NeP1 se fixe au niveau de la partie F1 de 50 bp de N-2.4 alors que le TR principalement sur la partie F2 de cet élément. Les associations homodimèriques ou hétérodimèriques avec les autres récepteurs nucléaires (RAR, RXR) modulent le niveau de transcription de ce gène (Baniahmad et al., 1990; Arnold et al., 1996). L'état de phosphorylation du récepteur TR est également un élément important. Enfin, le facteur HNF-I alpha (Hepatic nuclear Factor-I) qui est exprimé dans les cellules de l'oviducte a un rôle dans la régulation du gène lysozyme. Le promoteur du gène contient deux régions reconnues par cette protéine HNF-I. Cette régulation de l'expression du gène du lysozyme par le NHF-I semble avoir été perdue au niveau phylogénétique entre les oiseaux et les mammifères (Grajer et al., 1993).

### 2.3 Le locus de la conalbumine

Le gène de la conalbumine, encore appelé ovotransferrine a été identifié d'abord chez l'homme. Il s'étend sur au moins 33.5 kbp et comprend 17 exons. Le gène poulet est également organisé en 17 exons et 16 introns mais sur 10,5 kb seulement. Le messager a une taille de 2376 bp (Cochet et al., 1979 ; Jeltsch and Chambon, 1982; Jeltsch et al., 1987; Schaeffer et al., 1987). Très peu d'études de la régulation de l'expression de ce gène ont été réalisées. La régulation de la stabilité du messager est particulière. La molécule qui s'apparente à la famille des transferrines, fixe le fer sous forme ionique et régule la stabilité et le niveau de transcription de son propre messager. Si beaucoup d'études ont été réalisées sur la transferrine humaine et son récepteur (CD71), peu de documentation existe quant aux formes aviaires.

### Exemple 3 : Eléments extérieurs facilitateurs

Chez les eucaryotes, la recombinaison homologue est un événement naturel qui se produit surtout au moment de la méiose, afin d'assurer, dans un énoncé simpliste, un brassage des gènes et des allèles. Le rôle de la recombinaison homologue est beaucoup plus délicat à interpréter lors d'une division mitotique. Les phénomènes de recombinaisons homologue et non homologue semblent néanmoins cruciaux dès que le matériel génétique est endommagé. De nombreuses situations dans la vie d'une cellule et d'un organisme expose ainsi le matériel génétique. Par exemple, un défaut soudain de réplication, une ionisation radicalaire liée à des radiations, un stress oxydatif, l'action d'endonucléases ou de topoisomérases, le stress mécanique lié à la ségrégation mitotique, peuvent induire des coupures double brins accidentelles. Si la levure privilégie les mécanismes de réparation homologues, la cellule eucaryote supérieure semble privilégier le raboutage non homologue de ces coupures, même si la réparation homologue est aussi observée. Mais dans tous les cas, comme l'intégrité génétique est un préalable à toute division, pour faire face à cette situation critique de matériel endommagé, la cellule met en route tout un ensemble de gènes, chargés de réparer ces coupures. Décrits initialement dans le système levure, les gènes Rad (rad51, rad52, rad53 et rad54) (Takata et al., 2000 ; Morrison and Takeda, 2000; Bell et al., 1999 ; Shinohara et al., 1997; Bezzubova et al., 1997; Ivanov and Haber, 1997; Porter et al., 1996) impliqués dans la réparation par recombinaison homologue ont été identifiés aussi dans les organismes supérieurs. Les homologues Rad51 et Rad54 de poulet ont été clonés. L'ADN et la protéine présentent de fortes homologies avec les séquences de levure, souris et humain (Essers et al., 1997 ; Dronkert et al., 2000). Des similitudes d'action sont alors observées, mais aussi des différences, notamment en ce qui concerne l'action de Rad 51 (Bezzubova et al., 1993). De même, les homologues humains des gènes ku70 et ku86 de levure interviennent à la fois dans les réparations non homologues, et dans les phénomènes de recombinaison observés dans les réarrangements des loci d'immunoglobulines. Les relations entre ces différents acteurs commencent à être également approchées et mettent en évidence la formation de complexes protéiques dont les régulations sont elles mêmes complexes (Morrison et al., 2000 ; Morrison et al., 1999 ; Yamaguchi-Iwai et al., 1999 ; Takata et al., 1998; Yamaguchi-iwai et al., 1998). Ainsi, une liste non exhaustive de protéines facilitant la recombinaison (recombinase) peut être donnée, de la simple protéine bactérienne RecA (Shcherbakova et al., 2000), aux membres de la famille Rad, mais aussi à de protéines telles que BraC1 (Moynahan et al., 1999).
Dans une approche qui faciliterait des événements de recombinaison homologue, l'action de Rad 54 est évaluée au moment de l'introduction du vecteur de recombinaison homologue. Ainsi, deux approches sont possibles. La première consiste à effectuer une co-transfection d'un vecteur d'expression d'une des protéines de la famille Rad, en particulier Rad 54 avec le vecteur de recombinaison homologue. La seconde consiste à transfecter les cellules avec un vecteur d'expression conditionnelle d'une des protéines de la famille Rad, en particulier Rad 54. Dans ce cas, les cellules sont d'abord sélectionnées, stabilisées et établies pour être à nouveau transfectées avec un vecteur de recombinaison homologue, puis induites pour exprimer au maximum la protéine de la famille Rad. L'action de Rad54 testée au niveau de l'efficacité de transfection est présentée dans le tableau 4 ci-dessous :

**Tableau 4 : Influence de l'expression de Rad 54 sur le nombre de clones en transfection**

| Conditions | 1 | 2 | 3 | 4 | 5 | Témoin |
|---|---|---|---|---|---|---|
| ppréOva RH (µg) | 5 | 5 | 5 | 5 | 5 | 0 |
| pMCS5 (µg) | 5 | 4 | 3 | 2 | 0 | 10 |
| pRad54 (µg) | 0 | 1 | 2 | 3 | 5 | 0 |
| Fugène | 30 | 30 | 30 | 30 | 30 | 30 |
| DMEM | 970 | 970 | 970 | 970 | 970 | 970 |

| Nb de clones | | | | | | |
|---|---|---|---|---|---|---|
| Boite 1 | 344 | 304 | 172 | 248 | 292 | 0 |
| Boite 2 | 128 | 324 | 212 | 264 | 300 | 0 |
| Moyenne | 236 | 314 | 193 | 256 | 296 | 0 |

Afin de tester l'importance d'une des protéines de la famille Rad, le plasmide d'expression pRad54 (plasmide d'expression de la protéine RAD54 sous contrôle du promoteur CMV) est co-transfecté en présence du plasmide pOvaRH, plasmide de recombinaison ne contenant pas de cDNA d'intérêt. La quantité totale de plasmide est de 5ug de pOvaRH, en présence de quantités variables de pRad54. Le complément molaire étant apporté par un plasmide 'carrier', le pMCS5 d'origine commerciale, a peu près de même taille que le pCMV Rad54. Les cellules souches S86N sont ensemencées à 1x10⁶ cellules par boite dans du milieu de prolifération en présence de feeder inactivé par irradiation. Le mélange de transfection est ajouté et la sélection à la néomycine appliquée pendant 7 jours environ. Les clones sont alors dénombrés après fixation au méthanol et coloration au Wright/Giemsa. En conclusion, la présence de Rad54 ne semble pas modifier sensiblement l'efficacité d'obtention de clones.

### Exemple 4 : Choix du signal peptide

Une des approches choisies pour produire les protéines exogènes est leur accumulation dans l'oeuf. La stratégie de substitution remplacement utilisée dans le cadre de l'invention rend utile l'utilisation d'un système actif (mais éventuellement passif pour des protéines spécifiques qui en auraient la capacité) d'accumulation de la protéine exogène dans le blanc de l'oeuf. Ainsi, les phases codantes sont placées sous la dépendance du promoteur endogène du gène ciblé endogène et sont fusionnées avec un signal peptide de référence. La fonction du signal peptide est d'assurer la translocation de la protéine vers l'extérieur de la cellule à travers les différents éléments constitutifs de l'appareil sécrétoire de la cellules (réticulum, Golgi....). Les peptides signaux sont destinés à être clivés au cours du processus de maturation de la protéine. Celle ci s'accumule ensuite dans le liquide physiologique et dans le blanc d'oeuf en particulier. On utilise soit les peptides signaux des protéines endogènes, soit des peptides signaux d'autres molécules, séquences connues pour être correctement traitées. Notamment, le peptide signal du lysozyme est choisi de façon préférentielle car sa structure est bien connue et les 18 premiers acides aminés sont clivés lors de l'exportation de la protéine mature dans le blanc de l'oeuf. De plus, ce peptide signal appartient à une protéine naturellement accumulée dans le blanc de l'oeuf.
Parmi les autres peptides signaux utilisés, on trouve ceux des interleukines, ceux de certains récepteurs membranaires, ceux de facteurs de croissance secrétés par différents types cellulaires, ceux d'autres protéines connues pour être secrétées dans différents systèmes biologiques. La liste n'est pas limitative.

### Exemple 5 : Construction des vecteurs de recombinaison

### 5.1 Obtention des éléments intermédiaires

### - cassette pCMV Néo polyA

Le plasmide commercial pCI Néo (Promega, Madison, US) est ouvert par digestion HindIII et ligaturé sur lui-même en intra moléculaire pour donner le plasmide pCI Néo (Δ HindIII) qui place le gène de la néomycine phosphotransférase sous la dépendance directe du promoteur CMV. Le polyA d'origine est conservé.

### - Préparation des cDNAs d'intérêt

Les cDNAs d'intérêt sont préparés par amplification PCR avec
- un oligonucléotide Sma-3'incDNA S contenant le site SmaI, la partie 3' de l'intron artificiel du vecteur commercial pCINéo et le début de la séquence spécifique du cDNA en 5', en ayant omis l'ATG du cDNA, afin de brancher directement le cadre de lecture du cDNA d'intérêt en phase avec celui de l'ovalbumine ou du lysozyme.
- Un oligonucléotide cDNA-SmaI AS contenant le site SmaI et la région 3' du cDNA d'intérêt, en omettant éventuellement un polyA endogène cDNAet l'oligonucléotide 3' contenant la séquence spécifiques du cDNA et le site SmaI.
Le fragment PCR amplifié est hydrolysé par SmaI (sous réserve de l'absence de ce site dans le cDNA) et cloné en SmaI dans un vecteur pMCS5 (Mobitech, Allemagne) dans lequel une séquence polyA de 277 bp a été préalablement sous clonée en XbaI/XhoI. Cette séquence polyA est elle même issue d'une hydrolyse par XbaI/XhoI du vecteur pIRESHygro (Clontech). L'orientation du fragment est alors contrôlée par simple digestion avec la carte fournie du cDNA d'intérêt.

En cas de présence d'un site de restriction SmaI dans le cDNA d'intérêt, le fragment PCR amplifié est soumis à l'action de la T4 DNA polymérase pour rendre les extrémités franches et directement cloné dans le vecteur pMCS5 ouvert en SmaI (bout franc).

### 5.2 Obtention des fragments génomiques

L'ADN génomique d'embryons de 6 à 9 jours de différentes souches dont la souche CNR (Cou Nu Rouge) et la souche S86N, est extrait soit selon une méthode classique avec un tampon de lyse SDS Protéinase K, suivi d'extractions au phénol et phénol chlorofrome, soit à l'aide d'un kit de préparation (kit Proméga, ou Quiagen par exemple). Les différents protocoles donnent des rendements et des qualités de DNA compatibles avec leur utilisation respectivement la préparation d'une banque d'ADN génomique et une obtention de fragment PCR par amplification directe.
Dans le premier cas, l'ADN génomique est digéré partiellement par l'enzyme Sau3AI et les extrémités générées remplies par la polymérase Klenow en présence de deux des quatres nucléotides nécessaires à la synthèse. Il en résulte un remplissage partiel, afin de prévenir la formation d'inserts concatémaires. Les inserts ainsi partiellement remplis sont insérés par ligature dans le vecteur λ-GEM-12. Les vecteurs résultants de cette opération sont empaquettés dans des capsides de phages. A titre d'exemple, 3 isolats indépendants ont été générés et leur titre déterminé, chaque isolat titre à 3x 10 6 pfu/ml environ. La qualité de la banque a été vérifiée par l'étude de quelques inserts pris au hasard. Avec une sonde ovalbumine, obtenue par PCR génomique avec des oligonucléotides choisis à partir de la séquence publiée (Tableau 3), le criblage des phages permet d'obtenir des inserts, sous clonés en vecteurs plasmidiques pBS SK+ pour donner le plasmide #72. Ces inserts soumis à une cartographie de restriction (dont EcoRI et BamHI) ont été ensuite séquencés. Une comparaison entre le gène publié (réf J00895 et Tableau N°3) et les deux bras 5' et 3' du vecteur preRH ainsi que les fragments du clone #72 montrent que les homologies sont extrêmement élevées au niveau nucléotides. Seuls, deux segments localisés au niveau des introns 6 et introns 7 présentent de faibles variations réparties sur quelques dizaines de nucléotides (résultat non montré). Cette comparaison démontre la grande conservation de ce gène entre les souches analysées.

Dans le deuxième cas, différents oligonucléotides obtenus à partir de la séquence publiée (Tableau 3 ci-dessus) sont utilisés pour obtenir par PCR génomique les fragments donnant les bras 5' et 3' des vecteurs.

Pour le bras 5' du vecteur pOvaRH, les oligonucléotides OvaL 2995 S et OvaL 80 AS, situé respectivement à - 2995 bp et à + 80 bp par rapport à l'ATG du gène de l'ovalbumine amplifient une région de 3075 bp, qui, soumise à l'hydrolyse par NcoI, libère un fragment de 2860 bp. Parallèlement, à partir de la séquence qui spécifie le peptide signal du lysozyme, un fragment appelé lyso 1-18 - intron est préparé par amplification PCR sur le locus du lysozyme avec les oligonucléotides Lyso 1-18 et GE-IN-AS. Ce dernier oligonucléotide contient les 30 bp de l'intron artificiel du plasmide pCINéo. Les deux fragments (Ova5' et Lyso1-18 intron) sont hydrolysés indépendamment par NcoI, purifiés puis ligaturés ensembles et l'amplification sur le produit de ligature avec les oligonucléotides OvaL 2995 S et GE-IN-AS donne le fragment Ova5' Lyso 1-18 de 3050 bp. Ce produit PCR est ligaturé avec des adaptateurs EcoRI, qui permettent de cloner cet ensemble soit dans le vecteur pBSK au site EcoRI soit dans le pMCS5 digéré en bout franc par EcoRV.

Une dernière hydrolyse de ce fragment Ova5'Lysol-18 par Bst981 e Ascl libère le plasmide coupé et un petit fragment de 220 bp en amont du fragment ova5'. Cette petite délétion permet de dessiner deux oligonucléotides Ova5'.1S et Ova5'.2S utilisés ultérieurement pour le criblage des événements de recombinaison.

Le plasmide coupé est soumis à l'action de la polymérase Klenov afin de rendre les bouts francs avant d'être ligaturé avec des adaptateurs AscI et ligaturé de façon intramoléculaire. Il en résulte un plasmide pOvaLyso, qui contient en amont du bras Ova5' un site unique, qui permettra une linéarisation du vecteur final.

Pour le bras 3' du vecteur pOvaRH, l'amplification de la région avale située entre les positions + 1657 et +7707 par rapport à l'ATG du gène de l'ovalbumine avec les oligonucléotides OvaL 1657 S et OvaL7707 AS donne un fragment d'une taille de 6050 pb. Ce fragment a été sous cloné au site EcoRV de pMCS5 pour générer le plasmide pMCS5-Ova3' sens et pMCS5-Ova3' antisens. A partir du plasmide pMCS5-Ova3' antisens une digestion par HpaI (bouts francs) et NarI (5' sortant) permet d'obtenir le fragment de 6kb, qui est sous cloné dans pBS-SK aux sites ApaI (rendu bout franc) et AccI (compatible avec NarI) pour donner le plasmide pBS-SK Ova3'.

Pour le bras 5' du vecteur pLyso RH, les amplifications proximales avec les oligonucléotides lyso 1789 S et GE-IN-Ceu-AS ont permis d'isoler un fragment et de le sous cloner directement après traitement à la T4 DNA polymérase et phosphorylation par la polynucléotide kinase dans le vecteur commercial pMCS5 ouvert en bout franc en EcoRV. Ce fragment pLyso 5' contient les 2900 bp séquence en partie non publiée de la partie proximale du gène du lysozyme ainsi que l'équivalent des 18 premiers acides aminés de l'exon 1 correspondant au signal peptide du lysozyme. Ce fragment contient donc un site donneur d'épissage ainsi que la séquence du petit intron artificiel, séquence ajoutée par l'oligonucléotide AS comme décrit précédemment avec le bras 5' du vecteur pova5'.

Pour le bras 3' du vecteur pLysoRH, les amplifications distales avec les oligonucléotides Lyso 2859 S et Lyso 3185 AS ont permis d'isoler un fragment de 2150 bp en 3' du gène. Ces oligonucléotides portent à leur extrémité des sites SceI et MluI. Ce fragment soumis au traitement de la T4 DNA polymérase, d'une phosphorylation par la polynucléotide kinase et directement ligaturé dans le plasmide pMCS5 ouvert en bout franc par EcoRV donne le plasmide plyso3'. L'hydrolyse de ce plasmide par AflII/AvrII suivi de l'action de la T4 DNA polymérase et d'une ligature intramoléculaire permet de générer le plasmide pLyso3'.

### 5.3 Construction du squelette pOvaRH

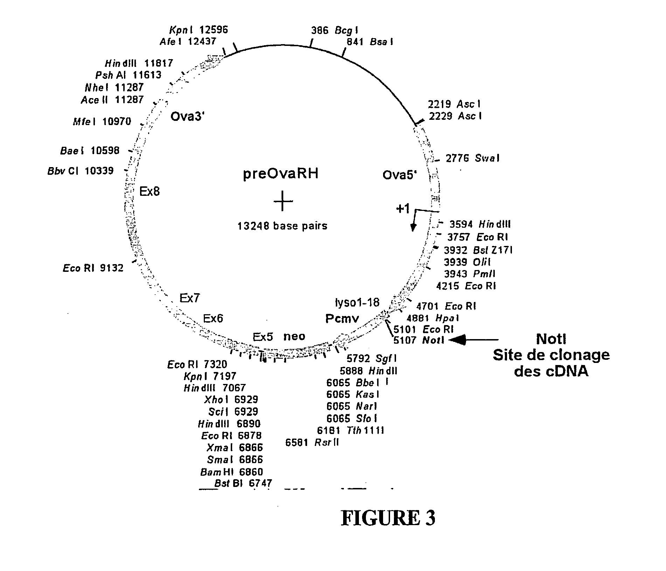

L'assemblage final du vecteur de RH par apports successifs de différentes cassettes (cassette de sélection, insertion du cDNA ou du gène d'intérêt...) est réalisé selon différents schémas. Un schéma est décrit sans qu'il soit exclusif, d'autres possibilités existant en fonction des sites de restriction présents dans certains cDNAs d'intérêt.
Le plasmide pCMV NéoOva3' est construit par ligature du fragment Ova3' qui résulte de la purification après coupure par BamHI du plasmide pOva3', avec le fragment purifié et déphosphorylé pCMV Néo polyA, obtenu par hydrolyse BgIII/BamHI du plasmide pCINeo(Δ-hindIII). Quelques modifications du polylinker présent en aval de l'insert Ova5'lyso sont également effectuées avant de rassembler les deux fragments Ova5'lyso et pCMV-Néo-Ova3' dans un vecteur pOvaRH (figure 3). Le fragment NarI/NotI présent dans pOvaLyso est remplacé par un adaptateur NotI pour donner pOvaLyso-NotI ce qui permet de conserver le site NotI comme site unique de clonage des cDNA d'intéret en éliminant de nombreux sites. Le plasmide pOvaLyso-NotI est initialement coupé initialement par PacI, puis traité par la T4 DNA polymérase pour rendre l'ADN bout franc. La libération de l'insert OvaLyso-NotI est alors obtenue par une coupure NotI. Le fragment de 3kb OvaLyso PacI^{F}/ NotI est cloné aux sites Kspl soumis à l'action de la T4 DNA polymérase afin de rendre les bouts francs / NotI de pCMV-Neo Ova3'. Ce clonage orienté permet de générer le vecteur de pOvaRH final qui présente de 5' en 3' :
- le site unique AscI, qui permet la linéarisation du vecteur avant transfection
- le bras génomique Ova5'lyso (1-18)-debut intron,
- le site unique NotI site unique de clonage des cDNA,
- la cassette de sélection à la néomycine
- le bras génomique Ova3'.
Le vecteur pOva RH est alors utilisable pour insérer les différents cDNAs d'intérêt.

### 5.4 Construction du squelette pLysoRH

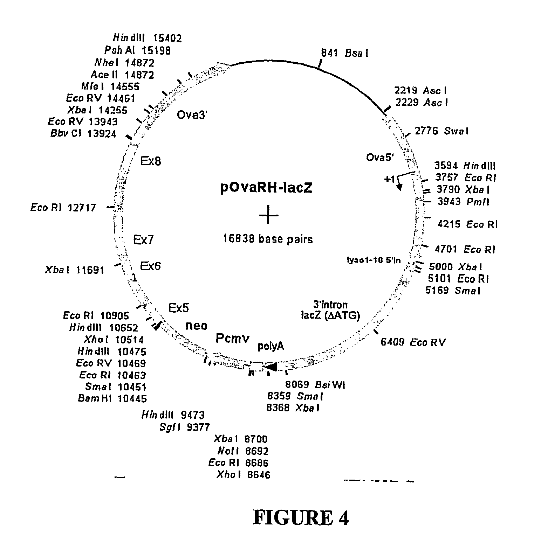

Le fragment Lyso 3', obtenu par l'hydrolyse par MluI du plasmide pLyso3' est ligaturé dans le plasmide plyso5', préalablement ouvert et déphosphorylé après hydrolyse par MluI. Il en résulte le plasmide pLyso5'Lyso3' dans lequel le fragment purifié et déphosphorylé pCMV Néo polyA, obtenu par hydrolyse NotI/MluI du plasmide intermédiaire pMCS5 CMVNéoPolyA. Ce dernier sous clonage est réalisé pour flanquer la cassette de sélection du site NotI en 5', afin de préserver ce site pour les clonages des cDNAs d'intérêt. Il est obtenu par insertion du fragment BglII/BamHI du plasmide pCINeo(Δ-hindIII) dans le pMCS5 ouvert et déphosphorylé en EcoRV.
Le vecteur pLysoRH obtenu contient successivement de 5' en 3' :
- le site unique AscI, qui permet la linéarisation du vecteur avant transfection
- le bras génomique Lyso5' avec le peptide signal 1-18 - debut intron,
- le site unique NotI site unique de clonage des cDNA,
- la cassette de sélection à la néomycine
- le bras génomique Lyso3'.
Le vecteur plysoRH est alors utilisable pour insérer les différents cDNAs d'intérêt (Figure 4).

### 5.5 Application à l'expression de différentes protéines

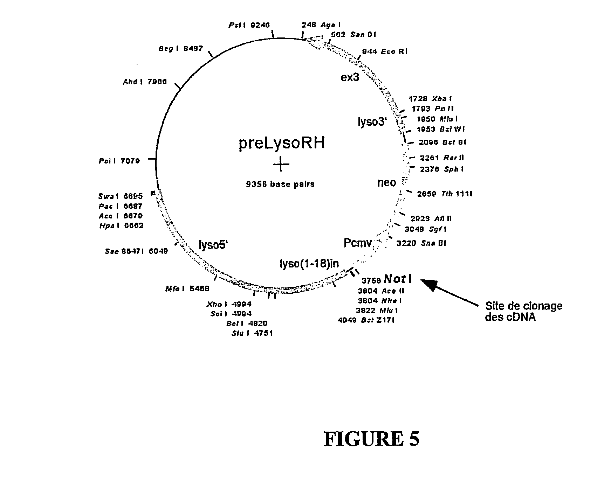

Les cDNAs de différentes protéines d'intérêt ou protéines modèles sont insérés dans ces vecteurs pOvaRH et pLyso RH. De façon non exhaustive, la stratégie consiste à préparer le cDNA selon le schéma présenté précédemment et à l'insérer dans le vecteur ouvert en NotI. Des variantes dans l'ordre des clonages des différentes parties peuvent être nécessaires. Des séquençages partiels ou en totalité sont réalisés afin de vérifier le bon enchaînement des différents éléments insérés. Ainsi, à titre d'exemples non exhaustifs, des vecteurs ont été construits avec les cDNAs de l'érythropoiétine pOvaRH Epo, pLysoRH Epo, de la beta-galactosidase pOvaRH LacZ, pLysoRH LacZ, de l'héliomycine (peptide antibactérien pOvaRH Hélio, plysoRH Hélio) La figure 5 illustre le pOvaRH LacZ.
Un cas particulier est l'application des vecteurs pOvaRH et pLyso RH à la production de molécules présentant leur propre signal peptide. La stratégie de construction utilisée diffère un peu de celles précédemment décrites. Par exemple, le cDNA de l'interleukine 11 est un fragment nucléotidique de 1100 bp, sous cloné dans le vecteur pBS SK dans les sites EcoRI. Comme il n'est donc pas nécessaire de fusionner lecDNA d'intérêt avec le peptide signal du lysozyme, le cDNA est inséré directement en phase au niveau de l'ATG de l'ovalbumine, conduisant à une fusion au niveau du deuxième acide aminé de l'interleukine 11. Dans cette exemple, le cDNA de l'IL-11 est soumis à l'action de la polymérase Taq dans une réaction de PCR en présence de deux oligonucléotides sens et antisens qui présentent les sites Hind III et XbaI. Ce fragment est ainsi soumis à la double hydrolyse HindIII/XbaI et ligaturé dans le vecteur pMCS5, lui-même préparé ouvert en HindIII/XbaI, après double hydrolyse par ces enzymes et traitement de déphosphorylation à la CIAP. Il résulte de ce clonage directionnel le plasmide pMCS5-Il-11. Ce dernier est soumis à la digestion HpaI/NsiI, le fragment est purifié et ligaturé dans le plasmide pOva5' AS, lui-même ouvert en NcoI/PstI, réparé en bout franc par l'action de la polymérase Klenow. Il en résulte le plasmide pOva5' IL-11.
Ce plasmide pOva5'Il-11 est soumis à la digestion par NotI, l'insert est purifié et ligaturé avec le plasmide pCMVNéoOva3', lui-même hydrolysé par NotI et déphosphorylé. Il en résulte le plasmide pOvaRH Il-11 composé de :
- le site NotI (linéarisation du vecteur avant transfection)
- le bras génomique Ova5'avec l'ATG de l'ovalbumine,
- le cDNA de l'IL-11 fusionné sur l'ATG et comportant un polyA
- la cassette de sélection contenant le gène de résistance à la néomycine sous la dépendance du promoteur CMV
- le bras génomique Ova3'
Une stratégie analogue permet d'obtenir le vecteur pLysoRH IL-11 en présence du peptide signal endogène lysozyme et par délétion du peptide signal de l'IL-11 et inversement par délétion du peptide signal lysozyme endogène et apport du peptide signal de l'IL-11.

### Exemple 6 : Transfection des vecteurs dans différentes cellules aviaires

### 6.1 Méthodes de transfections

Deux méthodes générales de transfections sont utilisées : l'électroporation et la transfection par des liposomes. Il semble que dans le système souris, l'électroporation soit en général choisi afin d'accroître l'efficacité de recombinaison des vecteurs de recombinaison homologue. Néanmoins, peu d'études comparatives ont été faites.

### - l'électroporation

L'électroporation est une méthode qui permet de faire rentrer un DNA de taille très variable dans une cellule en créant par un choc électrique un pore transitoire au niveau de la membrane cellulaire. Parmi les différents paramètres pouvant influencer l'efficacité de cette expérience, on peut citer la concentration cellulaire (de 1 à 10 x 10⁶ cellules), la solution conductrice du signal contenant le mélange cellules DNA (PBS, milieu sans sérum...), la concentration de DNA en général linéarisé (de 5 à 25 µg), la distance des électrodes (cuvettes ou électrodes simples directement dans la boite ou le puits de culture), l'amplitude du signal dépendant de la capacitance (de 10 à 1000 µF) et du voltage appliqués (de 5 à 100mV pour les signaux carrés et de 200 à 350 V pour les signaux non carrés), le nombre de chocs électriques délivrés et la forme de ce(s) choc(s) électrique(s) appliqué(s). Par forme, on entend la façon dont une même charge électrique est délivrée, notamment selon un signal carré, non carré, exponentiel inverse...
Le tableau 5 illustre quelques-uns de ces paramètres :

**Tableau 5 : Efficacité d'électroporation du plasmide pCINéo dans les cellules souches**

| **Capacitance (µF)** | **25** | **150** | **300** | **500** | **700** | **1000** |
|---|---|---|---|---|---|---|
| Clones A | 5 | 122 | 226 | 335 | 408 | 286 |
| Clones B | 9 | 149 | 297 | 454 | 443 | 361 |
| Clones C | 10 | 180 | 218 | 361 | 402 | 365 |
| **Moyenne** | **8** | **150** | **247** | **383** | **418** | **337** |
| Ecart Type | 3 | 29 | 43 | 63 | 22 | 45 |

L'électroporation du plasmide pCINéo linéaire est réalisée à l'aide d'un électroporateur BioRad (Gene Pulser II). Les cellules souches dissociées, lavées dans un milieu sans sérum et ajustées à une concentration de 5 x 10 cellules dans 0,8 ml sont placées dans une cuvette d'électroporation (chambre de 4mm). 5 à 20 µg de plasmide linéarisé, préparé dans du TE sont ajoutés aux cellules et le mélange est laissé 5-10 min à température ambiante, puis le choc électrique est appliqué avec une capacitance fixée à 560 uF. Les cellules sont alors placées à 4°c environ 10 min, puis ensemencées dans des boites de culture avec feeder dans les conditions de culture de ces cellules.

Classiquement, l'équivalent d'une cuvette soit 5x 10⁶ cellules sont réparties dans deux boites de 100 mm.
Le tableau présente 3 séries réalisées avec 15 µg de plasmide à 270 V. Les clones sont dénombrés après sélection à la néomycine pendant 7 jours (250 µg/ml), fixation au méthanol et coloration au Wright/Giemsa

### - les liposomes

### - Par liposomes, on entend toute molécule chimique présentant entre autre des caractéristiques de cations lipidiques, seule ou associée à d'autres éléments

L'efficacité de différents liposomes a été comparée sur différents types cellulaires. Les cellules souches embryonnaires étant fragiles, la présence de sérum lors de la transfection est un élément important. Différents matériels commerciaux ont été testés. Il ressort que l'efficacité et la toxicité de ces composants sont variables (Pain et al., 1999). De plus, la présence de sérum permet de diminuer les effets relativement toxiques de l'exposition des cellules aux lipofectants et ainsi de préserver les chances d'obtenir des cellules recombinantes dans un état physiologique et morphologique satisfaisant. Un avantage parmi d'autres de l'utilisation des liposomes est le faible nombre de cellules utilisées par essai. Le tableau 6 ci-dessous illustre une efficacité relative de transfection avec un vecteur simple d'expression :

### Tableau 6 : Transfection des vecteurs de recombinaison dans différents types cellulaires

Les cellules sont ensemencées dans leur milieu respectif à la densité de 5x 10 5 à 10⁶ cellules par boite de 100mm pour chaque type cellulaire. Le mélangede transfection, pouvant varier de 10.5 µg liposomes/ 3.5 µg DNA à 15 µg liposomes/ 5 µg DNA par boitede 100 mm, est mis sur les cellules de 3 à 15 heures selon les cas. La sélection à la néomycine est appliquée à 250 µg/ml et les clones apparaissent de 5 à 10 jours après transfection. Les clones sont prélevés et analysés par PCR afin de détecter les évènements de recombinaison.

Un exemple typique d'amplification est constitué d'un clonage en puits d'une plaque de 24, d'un passage en puits d'une plaque de 12 puits, puis d'une amplification en boite de 60 mm ou de 100 mm. Les étapes de criblage mises en place de façon optimale entre le passage du puits d'une plaque de 24 puits au puits d'une plaque de 12 puits permettent de limiter l'amplification aux seuls clones détectés positifs par PCR. Par puits de plaque de 6 puits, on obtient en général 5x 10⁵ à 10⁶ cellules. Ainsi, ne sont congelés en viable que les clones criblés positifs par PCR. L'amplification pour analyse en Southern blot ne concerne également qu'un faible nombre de clones.
Le tableau rassemble différents exemples obtenus avec des cellules de différentes natures et à différents passages. Il est nécessaire de constater que les efficacités de transfection et d'efficacité de recombinaison semblent variables selon les vecteurs et pour un même vecteur pourraient être dépendantes de la physiologie (age, état...) des cellules utilisées.

| **Cellules** **(nb de passage)** | **Vecteurs** | **Clones analysés** | **Clones positifs par** **PCR** | |
|---|---|---|---|---|
| | | nb | nb | % |
| LMH (p25) | pOvaRH Hélio | 35 | 1 | 3,8 |
| HD11 | pLysoRH GFP | 40 | 1 | 2,5 |
| HD11 | pLysoRH Epo | 36 | 3 | 8,3 |
| Valo4 (p39) | pOvaRH Hélio | 64 | 1 | 1,6 |
| Valo4 (p53) | pOvaRH LacZ | 28 | 1 | 3,6 |
| S86N 48 (p13) | pOvaRH Hélio | 48 | 1 | 2,1 |
| S86N 16 (p123) | pOvaRH Hélio | 264 | 23 | 8,7 |
| S86N 16 (p129) | pOvaRH LacZ | 168 | 1 | 0,6 |
| S86N 16 (p129) | pOvaRH hélio | 157 | 14 | 8,9 |
| S86N 16 (p137) | pOvaRH Hélio | 96 | 3 | 3,1 |
| S86N 16 (pl40) | pOvaRH LacZ | 143 | 7 | 4,9 |
| S86N 16 (p203) | pLysoRHx | 94 | 25 | 26 |
| S86N 45 (p71) | pLysoRHx | 94 | 10 | 10,4 |
| S86N 45 (p79) | pLysoRH Hélio | 96 | 15 | 15,6 |
| S86N 88 (p14° | pOvaRH LacZ | 79 | 1 | 1.2 |
| Valo8 (p10) | pOvaRH Hélio | 35 | 1 | 2.8 |
| Valo8 (p 13) | pOvaRH Hélio | 120 | 3 | 2.5 |

### 6.2 Efficacité de transfection stable avec les vecteurs de recombinaison

L'efficacité de transfection stable est estimée par le nombre de clones obtenus après sélection par la drogue qui est détoxifiée par le gène présent dans la cassette de sélection. Dans les cas reportés, la néomycine a été généralement utilisée. D'autres drogues peuvent être utilisées en fonction de la présence d'un gène de résistance différent dans le vecteur transfecté. Parmi les drogues classiques, on trouve la néomycine, l'hygromycine, la puromycine, la phléomycine, la zéomycine, la blasticidine, la viomycine.... D'autres milieux de sélection peuvent être utilisés comme l'addition d'analogue des bases, les milieux de sélection classique comme le milieu HAT...
Les clones sont comptés en fin de période de sélection correspondant à la disparition des cellules dans les boites témoins de l'expérience. Ces témoins sont soit des cellules non transfectées soit des cellules transfectées avec un vecteur ne contenant pas de gène de résistance à l'antibiotique utilisé.

### 6.3 Cellules utilisées

### 6.3.1 Fibroblastes embryonnaires de poulet (CEFs) (Exemple Comparatif)

Les fibroblastes Embryonnaires de poulet (Chicken Embryonic Fibroblasts) sont obtenus à partir d'une mise en culture d'embryons de poulet incubés de 9 à 13 jours selon un protocole bien établi et connu de la personne de l'art. Ces cellules primaires sont amplifiées et maintenues en culture pendant un nombre de passage peu important (inférieur à 30 en général) car les cellules présentent rapidement (en général à partir des passages 15 à 20) une phase progressive d'arrêt de prolifération et une entrée en sénescence caractérisée par un taux de divisions plus faible, une morphologie étalée de moins en moins fibroblastique. Cette phase de sénescence conduit à la disparition des cellules sauf dans de très rares cas où l'établissement spontané de cellules fibroblastiques aviaires et de poulet en particulier a été observé (lignée DF-1) (Himly et al., 1998 ; Kim et al., 2001).
Les transfections des vecteurs de recombinaison homologue sont donc réalisées lors des passages précoces (de 1 à 10), afin d'optimiser à la fois la possibilité de sélectionner et d'amplifier les clones résistants à la sélection mais aussi pour limiter une éventuelle dérive caryotypique et l'entrée rapide en sénescence. En général, les clones obtenus sont isolables, mais pas ou peu amplifiables. Par contre, ils peuvent être considérés comme des sources potentielles de noyaux modifiés pour des expériences de clonage nucléaire.

### 6.3.2 Lignée LMH (Exemple Comparatif)

La lignée de cellules LMH est une lignée d'hépatocyte de poulet, obtenue par carcinogénèse chimique (Kawaguchi et al., 1987) Cette lignée a été caractérisée au niveau caryotypique et présente un polyploïdie importante. Les cellules sont en général maintenues en DMEM ou HamF12, en présence de 8 % de sérum de veau foetal, de 2% de sérum de poulet, de 10% de TPB et d'antibiotiques. Ces cellules représentent un type cellulaire dendritique intéressant, très différent des cellules monocytes, fibroblastes et des cellules souches et présentent une sensibilité relative à différentes hormones stéroïdiennes. Les transfections de vecteurs de recombinaison pOvaRH notamment dans ces cellules ont permis d'obtenir des clones et des évènements de recombinaison. Le tableau 6 illustre les résultats de ces transfections.

### 6.3.3 Lignée HD11 (Exemple Comparatif)

La lignée de cellule HD11 est une lignée de monocytes de poulet immortalisés par la présence de l'oncogène v-myc apportée par un rétrovirus aviaire (Beug, et al., 1979). Cette lignée est productrice de virus, capables de propager l'oncogène. Certains clones pourraient être éventuellement non producteurs. Les cellules sont en général maintenues en milieu HamF12, complémenté avec 5% de sérum de veau foetal, de 2% de sérum de poulet, de 10% TPB. Comme précisé dans l'introduction, les macrophages activés produisent des quantités de lysozyme endogène. En les modifiant par les vecteurs de recombinaison pLysoRH, ces cellules peuvent donc servir de modèle de production à la molécule insérée dans ce vecteur. On réalise ainsi une stratégie knock in de production. Le tableau 6 illustre ci-dessus les résultats de ces transfections.

### 6.3.4 Cellules souches embryonnaires

Les cellules souches embryonnaires de poulet utilisées dans les exemples sont des cellules souches obtenues in vitro par la mise en culture de cellules de blastodermes de poulet (Pain et al., 1996 ; US 6,114,168 ; WO 96/12793). Les cellules sont maintenues, amplifiées in vitro et sont surtout capables de proliférer sur des périodes de temps long. Les profils de prolifération mettent en évidence des phases de prolifération rapide et plus lente au cours de l'établissement relatif des cellules. Cette particularité d'établissement à partir d'un isolat primaire est tout à fait unique pour une cellule d'origine aviaire, sans l'intervention extérieure d'un agent immortalisant ou un processus de transformation chimique. On peut distinguer ainsi arbitrairement les phases de prolifération précoces de ces cellules, qui sont observées durant les premiers passages (du passage 1 au passage 20) et les phases de prolifération plus tardives (passages > 20). Ces cellules souches sont transfectées avec les différents vecteurs de recombinaison contenant les cDNAs d'intérêt.

Le tableau 6 ci-dessus et le tableau 7 ci-dessous illustrent les résultats obtenus avec différents vecteurs. Il apparaît que ces cellules souches sont modifiables dans différents loci par une approche de recombinaison homologue.

### Tableau 7 : Transfection de cellules CECs avec différents vecteurs d'expression simple

5 µg de vecteur pCINéo avec 15 µg de Fugène 6 (Roche) est transfecté sous sa forme circulaire sur 0,8 x10⁶ cellules S86N16 à différents passages. Après application de la sélection à la néomycine par addition de G418 (250ug/ml) pendant 5 à 8 jours, des clones sont obtenus. Les boites sont fixées au méthanol (100%) et une coloration au Giemsa permet de dénombrer rapidement les clones. Cet exemple démontre dans des essais indépendants que les cellules sont transfectables en stable avec des vecteurs simples, sélectionnables et que les clones peuvent être amplifiés. Néanmoins, les niveaux d'expression des transgènes sont très variables d'un clone à l'autre, comme on peut le visualiser facilement en évaluant l'intensité de fluorescence du marqueur E-GFP par exemple.

| Essais | 1 | 2 | 3 | 4 | 5 | 6 | Moyenne | Ecart type |
|---|---|---|---|---|---|---|---|---|
| Nombre de clones | 75 | 90 | 135 | 124 | 146 | 106 | 113 | 27 |

### 6.4 Etat physiologique des cellules

Par état physiologique, on entend l'état de différenciation, de transcription et d'activation des cellules utilisées. Modifiées au niveau des loci ciblés par les vecteurs simples et les vecteurs de recombinaison homologue, les cellules réagissent au niveau physiologique. Il apparaît donc utile de les suivre en fonction de leur utilisation future dont les deux principales sont :
- l'injection des cellules modifiées dans des embryons receveurs pour suivre leur contribution dans les embryons et plus tard au niveau des poussins et animaux adultes
- la production in vitro par les cellules modifiées des molécules d'intérêt apportées dans les vecteurs notamment mais de façon non exclusive dans le cas des cellules LMH et HD11.

### 6.5 Clonage et détection des événements de recombinaison

### 6.5.1 Clonage

Les clones, observés macroscopiquement et microscopiquement sont prélevés et mis directement en plaque de 24 puits. I1 est important de prélever les clones dès que possible pour limiter les effets de mélange entre clones. L'origine des clones selon les différentes boites est mentionnée pour contrôler le niveau d'indépendance de deux clones positifs par exemple. Les conditions de prolifération des clones sont à peu près les mêmes que celles utilisées pour les cellules parentales. Les clones, une fois prélevés sont observés quotidiennement et dés que la densité des cellules le permet, les clones sont dissociés et amplifiés pour analyse.

### 6.5.2 Criblage des clones par PCR

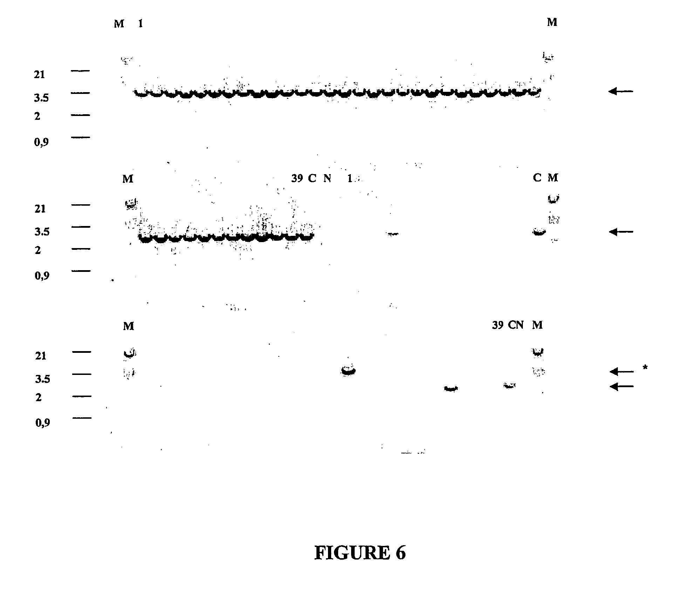

Les cellules des clones en prolifération dans les puits sont lavées, dissociées et préparées pour être d'une part réensemencées pour une amplification et d'autre part pour être analysées. Le DNA est principalement extrait à l'aide de techniques classiques dont des kits commerciaux. Différentes détections sont lancées pour cribler les événements de recombinaison. Les mises au point des conditions de PCR sont réalisées pour chaque couple d'oligonucléotides avec du DNA extrait de cellules transfectées, sélectionnées et non clonées individuellement. Cette approche en 'pool' permet d'obtenir rapidement du matériel à tester. De façon générale, une première PCR est réalisée pour détecter le locus endogène ciblé afin de s'assurer de la qualité des extractions et des DNA. Une deuxième PCR est réalisée pour détecter un événement de recombinaison entre un oligonucléotide externe à la construction et une séquence commune à tous les vecteurs d'une même famille par exemple la séquence peptide signal lyso 1-18. Cette PCR est considérée comme discriminante pour les événements de recombinaison. Sur les clones positifs dans cette dernière PCR, de nouvelles séries de PCR sont réalisées toujours avec le même oligonucléotide externe mais avec un autre oligonucléotide spécifique de chaque cDNA de façon à s'assurer de sa présence et de la non modification générale du cDNA inséré. Cette PCR est considérée comme discriminante et permet de renforcer l'observation des clones positifs.
**La** **figure 6** illustre cette détection par PCR de clones. La majorité des clones est détectée négative, certains détectés positifs recombinés. Ce premier tri permet de ne garder que quelques clones qui sont amplifiés, congelés et analysés par Southern blot.

### Légendes figure 6 : Détection par PCR des clones positifs recombinants

A titre d'exemple le DNA est extrait avec le kit Promega (Madison, WS). Le culot cellulaire de chaque clone est repris dans 300 µl de tampon de lyse (Nuclei Lysis Solution, NLS), en présence de 20 µg/ml de RNAse. Le lysat est homogénéisé, incubé 30 min à 37°c et 100 µl de tampon de précipitation de Protéines (Protein Precipitation solution PPS) ajouté. L'ensemble est incubé à 4°c après agitation forte. Le surnageant est prélevé après centrifugation et 400 µl d'isopropanol ajouté. Après précipitation et centrifugation, le culot est lavé en éthanol 70°c, séché et repris dans 50 µl de TE (10.1). Les volumes peuvent être utilisés de façon proportionnelle pour des extractions avec plus de cellules. La quantité de DNA obtenue est estimée par dosage spectrophotométrique. L'extraction d'un culot équivalent à un puits d'une plaque de 24 puits donne approximativement de 0,5 à 2 µg de DNA selon la densité des cellules dans le puits. Les PCR sont réalisées sur 100 à 500 ng environ.
- Une première PCR est réalisée sur 38 échantillons (N° 1 à 39) pour détecter le locus ovalbumine endogène et s'assurer de la qualité des extractions et des DNA. Cette réaction utilise deux oligonucléotides qui donne une bande amplifiée de taille de 2950 bp (pistes 1 à 39 partie supérieure du gel).
- Une deuxième PCR est réalisée pour détecter un événement de recombinaison entre un oligonucléotide externe et la séquence commune à tous les vecteurs ici, la séquence peptide signal lyso 1-18. La taille attendue dans ce cas est de 2900 bp (pistes 1 à 39, partie inférieure du gel).
   - M : marqueur de taille,
   - C, contrôle DNA (obtenu d'un mélange de clones),
   - Cn, contrôle négatif de la PCR sans DNA

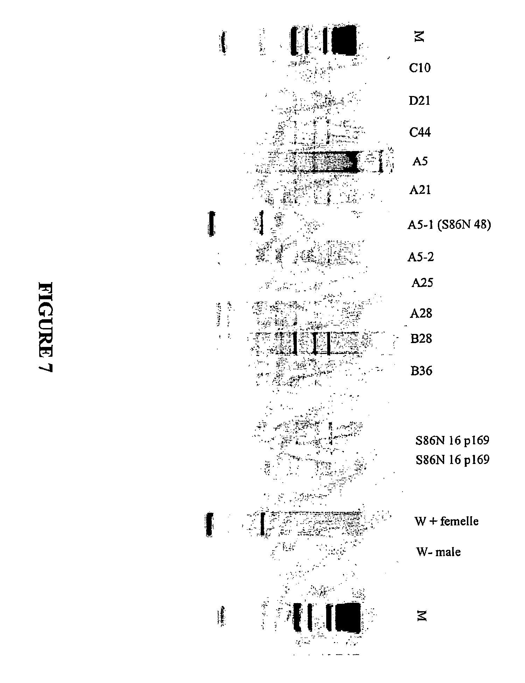

Une autre PCR est réalisée pour déterminer par exclusion le sexe des clones positifs avec des oligonucléotides spécifiques d'une région répétée du chromosome W. Seuls les clones Femelles (ZW) pourront être détectés, les clones males (ZZ) ne le seront pas.
**La** **figure 7** illustre cette détection différentielle sur quelques clones.

### Légende de la Figure 7 : Détection du sexe de différents clones identifiés positifs par PCR

Les DNA de différents clones détectés positifs par PCR pour un événement de recombinaison homologue sont soumis à une PCR génomique pour déterminer le sexe des clones. Les oligonucléotides utilisés sont spécifiques du chromosome W du poulet.

Ils ne peuvent détecter que les clones femelles. Dans la figure, les clones sont majoritairement males, comme les cellules utilisées (S86N16), à l'exception du clone A5-1, issu de cellules précoces d'une autre origine (cellules S86N 48).
Clones obtenus avec pOvaRH Hélio : C10, D21, C44, A5, A21, A5-1
Clones obtenus avec pOvaRH lacZ : A 5-2, A25, A28, B28, B36
Cellules parentales : S86N 16
Control positif : DNA de sang de femelle
Control négatif : DNA de sang de male
M : Marker

### 6.5.3 Southern blot

Afin de vérifier les résultats obtenus en PCR génomiques sur les clones positifs avec les deux PCR discriminantes pour la détection d'un événement de recombinaison, une analyse en Southern blot est réalisée avec du DNA extrait des clones. Cette analyse permet de vérifier que :
les tailles du locus ciblé sont conservées et qu'il n'y a pas de délétion majeure
un seul événement de recombinaison a eu lieu et que le génome ne contient pas d'autres intégrations aléatoires.

Le DNA des clones est extrait selon une méthode classique de Protéinase K-SDS, avec extraction phénolique ou à l'aide d'un kit commercial d'extraction (Qiagen, Promega). Dans une approche classique de biologie moléculaire, une quantité variable de DNA est soumise à l'hydrolyse par différentes enzymes, afin de permettre de repérer les fragments dont la taille est différente en fonction de l'événement de recombinaison. Le Tableau 8 illustre la détection des évènements de recombinaison par Southern Blot en fonction des différentes sondes utilisées

### Tableau 8 : Détection des évènements de recombinaison par Southern blot

Les DNA hydrolysés sont soumis à une migration électrophorétique qui sépare les fragments selon leur taille. Le procédé classique de Southem blot est appliqué, avec un transfert sur une membrane hybond N+ (Amersham Pharmacia biotech, UK). Les sondes utilisées pour l'hybridation sont obtenues soit par PCR, soit par purification d'un fragment plasmidique contenant les différents gènes d'intérêt. A titre d'exemple, les sondes lacZ (1159 bp) et Néo (354 bp) sont obtenues par PCR à partir de matrice plasmidique. La sonde ovalbumine 3' est une sonde intronique de 800 bp, obtenue à partir du DNA génomique. La sonde ovalbumine 5' est une sonde intronique de 870 bp. Ces sondes sont obtenues par PCR.
Les sondes purifiées sont marquées avec l'alcaline phosphatase à l'aide du kit AlkPhos (Amersham Pharmacia Biotech, UK) et peuvent être gardées plusieurs mois à -20°c. Pour la détection les membranes sont pré hybridées sous agitation à raison de 1 ml/cm² dans un tampon d'hybridation et ensuite hybridées à 55°C par addition de la sonde marquée (5 ng/ml). Les membranes sont alors lavées à 65°c dans le tampon primaire contenant de l'urée 2M, du NaCl 150 mM, du NaPhosphate 50mM, du MgC12 1 mM, SDS 0,1% et du 'blocking agent' 0,2%, fourni par le kit, puis dans le tampon secondaire contenant TrisHCl 50 mM pH 10.0, NaCl 100 mM à température ambiante.
Le système de révélation par chimiluminescence, prêt à l'emploi est ajouté sur la membrane lavée (1 ml/cm2) et l'intensité des bandes est révélée par autoradiographie.
La taille des bandes détectées est comparée avec la taille attendue.
Le critère pour retenir les clones est une bonne adéquation entre la taille attendue et celle observée. Des hybridations complémentaires sont réalisées (résultat non montré)

| Digestion | BamH I | | ScaI | |
|---|---|---|---|---|
| Sonde | - RH | RH Hélio | - RH | RH Hélio |
| Ovalbumine 3' | 18.5 kb | 7 kb | 12 kb | 12.7 kb |
| Ovalbumine 5' | 18.5 | 9.5 | 12 | 12.7 |
| Néomycine | / | 2 | / | 12 |

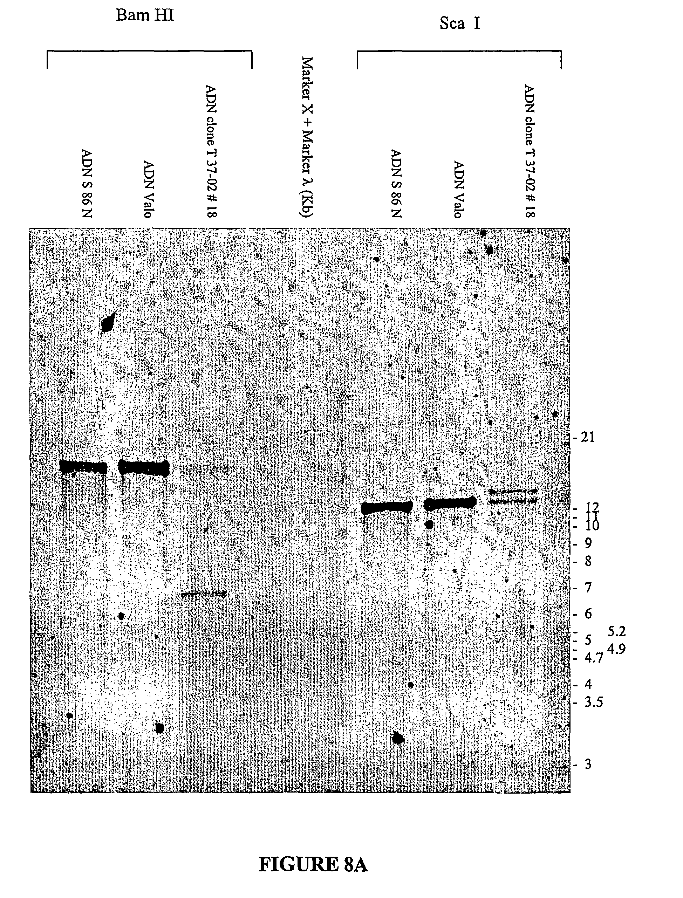

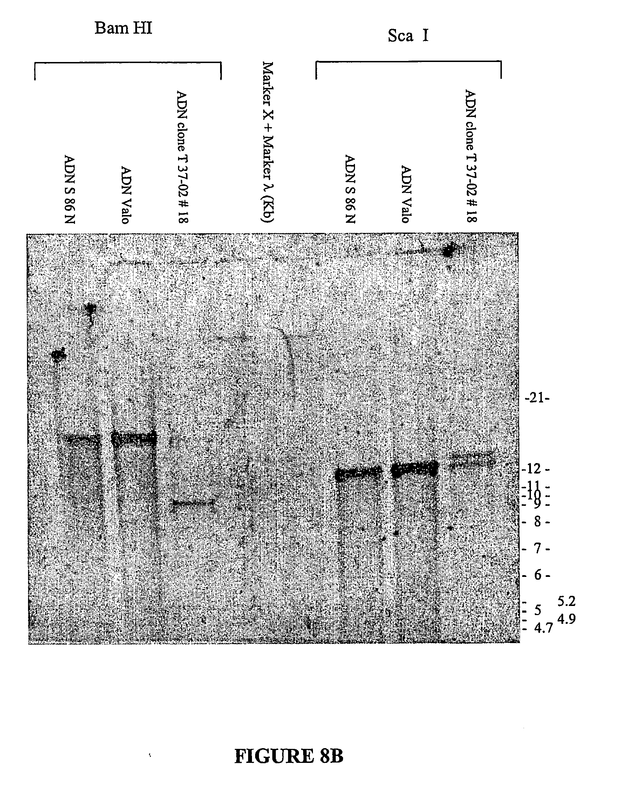

La figure 8 illustre cette analyse en fonction des tailles attendues.

### 6.6 Caractérisation des clones

Les différents critères utilisés pour caractériser les cellules parentales sont utilisés pour caractériser les cellules clonées.

### 6.6.1 Vérification du caryotype

Le caryotype des cellules est analysé sur les métaphases des cellules obtenues selon un protocole classique après un traitement à la colcémide (pulse de quelques heures avec 0,05 à 0,15 µg/ml final, de préférence 0,08 µg/ml) des cellules en prolifération. Les métaphases étalées sont observées sous microscope et le nombre de macrochromosomes compté. Le comptage des minichromosomes est réalisable mais plus délicat et une évaluation plus en détail par hybridation in situ peut également être envisagée. Par ailleurs, la vérification par FISH (hybridation in situ avec une sonde fluorescente) de la présence du transgène renforce les observations réalisées par Southern blot quant à l'unicité de l'événement.

### 6.6.2 Activité Alcaline phosphatase endogène

Le premier test immunocytochimique utilisé est la détection de l'activité alcaline phosphatase endogène. Tous les clones positifs en PCR et validés par l'analyse en Southern Blot sont étudiés comparativement aux cellules parentales.

### 6.6.3 Anticorps de surface

Comme les cellules parentales sont positives, la présence des antigènes de surface spécifiques des cellules souches est également recherchée au niveau des clones recombinés obtenus. Les réactions avec les anticorps SSEA-1, TEC-01 et EMA-1 sont systématiquement réalisées.

### 6.6.4 Activité télomérase

L'activité télomérase apparaît être un des bons critères pour juger de l'état indifférencié de cellules souches. Aussi, comme pour les cellules parentales, l'activité télomérase des différents clones recombinés est analysée. Lors du passage des clones pour leur amplification et congélation, un petit aliquot de 5 x 10³ à 5x 10⁵ cellules est préparé sous forme de culot cellulaire et l'activité télomérase de ces cellules est évaluée à l'aide du kit 'Telo TAGGGTélomerase PCR Elisa' (Roche). Le niveau de l'activité télomérase apparaît être un bon reflet de la qualité des clones notamment après la pression de sélection appliquée par la drogue.

Le tableau 9 ci-dessous illustre quelques-unes de ces caractéristiques sur des clones recombinés :

**Tableau 9 : Caractérisation de clones pOvaRH Hélio issus du criblage par PCR**

| Cellules | Détection AP* | | Anticorps TEC01 (%) | | Anticorps EMA-1 (%) | | Télomérase (blanc= 0,07) | |
|---|---|---|---|---|---|---|---|---|
| | - RA | +RA | RA | + RA - | -RA | + RA - | -RA | +RA |
| S86N16* | ++++ | - | 90 | <10 | 90 | 10 | > 1,5 | <0,2 |
| C10 | +++ | - | 70 | <10 | 90 | <10 | 1,23 | 0,282 |
| C44 | +++ | - | 70 | <10 | 90 | <10 | 1,13 | 0,20 |
| D21 | +++ | - | 90 | <10 | 90 | <10 | 1,04 | 0,15 |
| A5-1 | NT | NT | NT | NT | NT | NT | 1,08 | 0,257 |
| A21 | +++ | + | NT | NT | NT | NT | 1,13 | 0,62 |
| A 37 | +++ | +/- | NT | NT | NT | NT | 1,09 | 0,566 |

| | | | | | | | | |
|---|---|---|---|---|---|---|---|---|
| Les cellules sont ensemencées dans des puits d'une plaque de 12 puits à 10⁴ cellules par puit en moyenne. Les différentes caractérisations sont effectuées après fixation dans le cas de la détection AP et anticorps et sur culot sec qui est lysé selon le protocole établi dans le kit Roche dans le cas de l'activité télomérase (NT = Non Testé). Conclusion : les clones sont dans leur ensemble semblables aux cellules parentales *(S86N16) quant aux différents marqueurs utilisés pour caractériser l'état des cellules. | | | | | | | | |

### 6.7 Conversion allélique

Les clones obtenus et sélectionnés après l'analyse en PCR et en Southern blot sont a priori modifiés sur un seul allèle (détection de l'allèle sauvage en analyse Southem Blot). L'obtention des deux allèles modifiés peut se réaliser in vivo et in vitro. In vivo, les animaux homozygotes pour la modification sont obtenus après croisement des animaux hétérozygotes, eux mêmes descendants des animaux chimères, directement obtenus après injection des cellules modifiées. In vitro, la conversion allélique du deuxième allèle non encore modifié est notamment possible par l'application sur les clones d'une sélection à de fortes doses de la drogue G418 (de 0,5 à 5 mg/ml). Cette approche permet d'obtenir des quantités intéressantes de protéines à la fois in vitro et in vivo.

### Exemple 7 : Test in vitro des vecteurs de recombinaison pour la production des protéines d'intérêt.

Le but de l'invention est de produire les molécules dans le liquide physiologique de l'animal, notamment le sang et le blanc d'oeuf. Pour réaliser cette approche les vecteurs, notamment les vecteurs de recombinaison homologue sont construits selon les schémas décrits précédemment. Ces vecteurs de recombinaison ciblent différents loci dont principalement le locus de l'ovalbumine et celui du lysozyme. Un point important est la validation de ces vecteurs par une approche in vitro. Notamment il est important de suivre le repliement de la protéine à produire, les modifications post traductionnelles obtenues in vitro dans un système aviaire et l'influence de l'état homozygote du locus modifié sur l'expression de 1a molécule d'intérêt. Les stratégies de sécrétion sont également étudiées via un modèle in vitro, avec des signaux peptides et des stratégies d'épissage différents selon les constructions.
Deux systèmes complémentaires sont développés pour étudier chacune des principales classes de vecteurs.

### 7.1 Différenciation in vitro des clones recombinés

Dans une première approche la différenciation in vitro des clones de cellules souches peut être mise à profit pour suivre la production des molécules dans le surnageant des cultures. Les conditions de milieux utilisés font appel à la formation de corps embyroïdes soumis à la présence de différents agents tels que des inducteurs comme par exemple le diméthylsulfoxide, l'acide rétinoïque, le TPA, le PMA, le LPS, des facteurs comme par exemple le FGF, TGFβ, MCSF, cMGF, des hormones, comme les hormones stéroïdes, l'oestradiaol, la dexaméthasone, la progestérone ou d'autres familles d'hormones.

### 7.2 Stimulation des cellules en lignée recombinées

### 7.2.1 Validation des vecteurs pOvaRH

Les cellules LMH sont des cellules aviaires issues d'une tumeur hépatique. D'après quelques exemples pris dans la littérature, le gène de l'ovalbumine semble être activable dans la lignée LMH selon différentes conditions de stimulations hormonales. Notamment, une combinatoire d'insuline, d'oestradiol et de dexaméthasone (stéroïde mimant l'action des glucocorticoïdes) permet une induction plus ou moins importante du gène de l'ovalbumine. Cette stimulation peut être utilisée sur les clones recombinés des cellules LMH. Des co-transfections des récepteurs nucléaires ER et COUTF sont également envisagées pour accroître le niveau de ces récepteurs dans les cellules et ainsi permettre une meilleure induction du gène de l'ovalbumine sous l'effet des différents inducteurs utilisés. Les vecteurs de la famille pOvaRH sont préférentiellement utilisés dans cette approche.

### 7.2.2 Validation des vecteurs pLyso RH

Il apparaît possible d'induire la différenciation des cellules HD11, lignée de monocytes de poulet en cellules macrophages par différents traitements, dont une action par les lipopolysaccharides (Goethe et al., 1994 ; Goethe and Phi-Van, 1998). Dans une approche de stimulation par ces substances, le surnageant des clones recombinés, notamment avec les vecteurs de la famille pLysoRH est étudié et permet d'obtenir des quantités importantes de protéines d'intérêt.
Le Tableau 10 ci-dessous illustre cette production de molécules d'intérêt à partir des cellules HD11 recombinées avec un des vecteurs de 1a famille pLysoRH spécifiquement sous l'action d'inducteur dont les LPS.

### Tableau 10 : Stimulation des cellules HD11 transfectées avec le vecteur pLyso RH Epo

Les cellules HD11 sont transfectées et des clones recombinés obtenus avec le vecteur pLysoRH Epo. Sur les clones stimulés par le LPS (5 µg/ml), deux analyses sont effectuées :
A : une réaction de RT-PCR pour détecter les messagers du lysozyme endogène dans les cellules non trasnfectées induites et les messagers de l'érythropoiétine dans les clones de cellules recombinées.
B : Une détection par réaction Elisa (kit Roche) du surnageant des clones stimulés ou non stimulés permet de titrer la présence d'érythropoïétine

| | | D.O. à 450 | Concentration |
|---|---|---|---|
| Standard | S1 | 0 | 0 |
| | S2 | 0,12 | 11 |
| | S3 | 0,26 | 20 |
| | S4 | 0,46 | 41 |
| | S5 | 0,86 | 86 |
| | S6 | 2,00 | 179 |
| Contrôle positif | | 0,79 | 71,4 |
| Contrôle négatif | | 0,09 | 1,2 |
| HD11 parentales | - LPS | 0 | < 0,5 |
| Clones non recombinés | + LPS | 0 | < 0,5 |
| Clone 1 pLysoRH Epo | - LPS | 0,67 | 61,3 |
| | + LPS | 2,22 | 199 |

Conclusion : le clone 1 produit de grandes quantités d'érythropoïétine dans le surnageant de culture.

### Exemple 8 : Injection des cellules modifiées dans des embryons receveurs et obtention d'animaux.

Les clones de cellules souches modifiées sont caractérisés comme les cellules parentales à l'aide des différents marqueurs d'immunocytochimie décrits précédemment. A l'issue de ces contrôles, certains clones sont utilisés pour générer des animaux modifiés, destinés à produire la molécule d'intérêt. Parmi les différentes stratégies utilisées pour produire ces animaux, on peut distinguer :

### 8.1 Injection intrablastodermique.

Les cellules modifiées sont injectées dans la cavité blastodermique d'un embryon receveur de stade blastula selon un protocole décrit précédemment (Pain et al., 1996. Brevets US 6,114,168 ; WO 96/12793). Les embryons utilisés dans cette approche sont non incubés ou juste incubés. Les cellules vont participer à la colonisation des embryons. Différentes méthodes complémentaires permettent d'évaluer le niveau de contribution des cellules injectées, par exemple, une méthode est basée sur une différence phénotypique entre une souche receveur non pigmentée blanche et une souche donneur colorée (dont sont issues les cellules utilisées). Une autre méthode est le suivi de la présence du transgène par PCR génomique à l'aide d'oligonucléotides spécifiques.

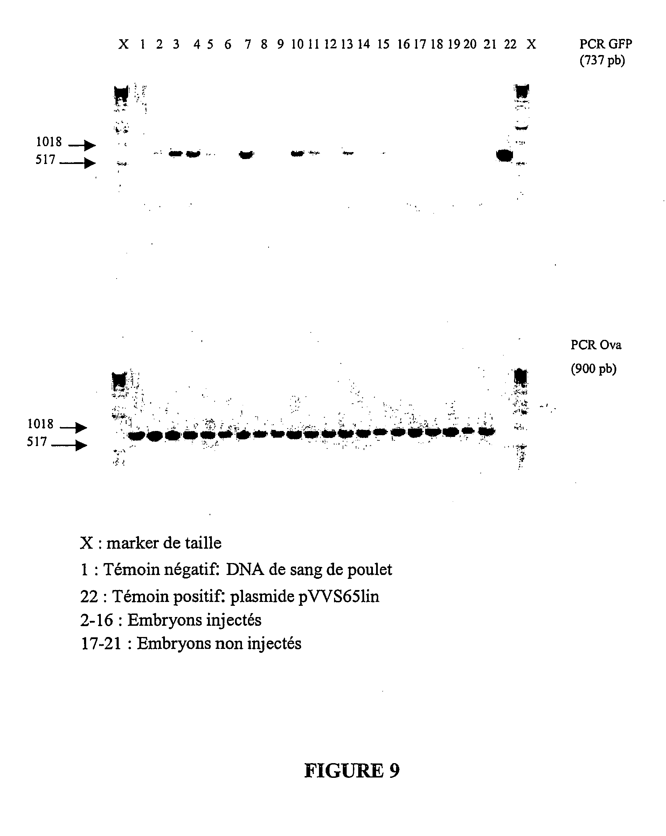

La figure 9 illustre la contribution phénotypique d'un clone modifié avec un vecteur d'expression simple. (Exemple Comparatif).

### Légende de la figure 9 : Obtention de chimères avec les clones modifiés

Chimères obtenues avec des cellules modifiées par le vecteur simple pEGFP- SV40 Néo Les oeufs injectés par voie blastodermique sont ouverts à différents stades et les embryons prélevés pour être analysés. Le DNA extrait est soumis à une réaction de PCR génomique avec différents oligonucléotides pour détecter la présence du transgène (ici la E-GFP). La partie supérieure du gel montre ainsi 21 échantillons analysés dont 11 détectés positifs sur les 15 embryons injectés analysés. La partie inférieure montre l'analyse réalisée avec une autre paire d'oligonucléotides, permettant de valider 1a qualité du DNA et de l'extraction.
Conclusion : les cellules embryonnaires modifiées par un vecteur d'expression simple peuvent contribuer et coloniser les embryons de façon efficace.

Le tableau 11 ci-dessous illustre l'obtention de chimères avec un clone recombiné.

**Tableau 11 : Différentes chimères phénotypiques obtenues avec un clone recombiné par le vecteur pOvaRH Hélio, injecté par voie intra-blastodermique. Le taux apparent de chimérisme est variable.**

| Cellules injectées | Vecteur | Animaux vivants | Chimères (%) |
|---|---|---|---|
| S86N 66 (p5)* | x | 13 | 1 (8 %) |
| S86N 48 clone A5 | pOvaRH Hélio | 37 | 1 (3%) |
| S86N 48 clone A5 | pOvaRH hélio | 43 | 3 (7%)** |

| | | | |
|---|---|---|---|
| * Cellules non modifiées en contrôle ** 6 autres animaux chimériques ont été obtenus à l'état embryonnaire | | | |

Le DNA des animaux chimères vivants a été extrait à partir d'une biopsie de plume et une détection PCR confirme la présence du transgène dans les échantillons (résultat non montré).

### 8.2 Injection intracardiaque

Les cellules modifiées sont injectées dans la circulation sanguine via la voie intracardiaque d'un embryon de 48 à 72 h d'incubation. A ce stade de développement, la morphogénèse de l'embryon permet une accessibilité importante de la cavité cardiaque en formation. A l'aide de fins capillaires les cellules sont injectées directement. Le développement se poursuit normalement jusqu'à l'éclosion.
Le Tableau 12 ci-dessous illustre la contribution phénotypique de clones modifiés dans les tissus d'embryons par injection intracardiaque

### Tableau 12 : Obtention de chimères phénotypiques par injection intracardiaque

Les cellules d'un clone modifié avec un vecteur simple (dans l'exemple le clone 14A est obtenu avec le vecteur pVVS65 contenant le pCMV E-GFP SV40 Néo) ou un vecteur de recombinaison sont injectées en intracardiaque directement dans la circulation d'un embryon incubé quelques heures (de 36 à 72 heures). Dans l'exemple les tissus sont prélevés sur les embryons de 5 à 21 jours selon les cas. Une PCR génomique permet de détecter la présence du transgène, tandis qu'une autre PCR sur un gène endogène valide la qualité du DNA analysé.

| Cellules | Embryons injectés | Embryons analysés | Embryons positifs | % |
|---|---|---|---|---|
| Exp 1 | 40 | 16 | 2 | 12,5 |
| Exp 2 | 40 | 18 | 3 | 16,7 |
| Exp3* | 50 | 14 | 2 | 14,3 |
| Exp4* | 50 | 18 | 2 | 11,1 |
| Exp5* | 50 | 22 | 1 | 4,5 |

| | | | | |
|---|---|---|---|---|
| * différents tissus sont prélevés sur des poussins à l'éclosion et analysés. Les détections PCR sont positives en général sur plusieurs tissus différents. | | | | |

### 8.3 Utilisation des noyaux modifiés comme source de noyaux dans un processus de transfert nucléaire. (Exemple Comparatif).

Les cellules modifiées sont des sources de noyaux modifiés qui peuvent être utilisés dans des techniques de transfert nucléaire dans un ovocyte receveur afin de reconstituer un embryon. Dans ce cas, la modification apportée par le noyau est directement portée par l'animal dans son patrimoine génétique et donc transmis à tous ses descendants. Les techniques actuelles de fusion de la cellule et /ou du seul noyau avec l'ovocyte préparé permettent d'envisager une telle approche. Les phases d'activation permettant alors une reprogrammation efficace du noyau donneur en lieu et place du matériel génétique haploïde de l'ovocyte receveur.

### REFERENCES

Ahne, B. and W. H. Stratling (1994). Characterization of a myeloid-specific enhancer of the chicken lysozyme gene. Major role for an Ets transcription factor-binding site. J Biol Chem 269 : 17794-17801.
Altschmied, J., M. Muller, et al. (1989). Cooperative interaction of chicken lysozyme enhancer sub-domains partially overlapping with a steroid receptor binding site. Nucleic Acids Res 17 : 4975-4991.
Arao, Y., E. Yamamoto, et al. (1996a). Steroid hormone-induced expression of the chicken ovalbumin gene and the levels of nuclear steroid hormone receptors in chick oviduct. Biosci Biotechnol Biochem 60 : 493-495.
Arao, Y., N. Miyatake, et al. (1996b). Steroid hormones differentially induce transcription of the chicken ovalbumin gene, but stabilize the mRNA with the same half-life. J. Biochem 120 : 710-715.
Arnold, R., M. Burcin, et al. (1996). DNA bending by the silencer protein NeP1 is modulated by TR and RXR. Nucleic Acids Res 24 : 2640-2647.
Baba TW et al. (1988). Cell lines derived from avian lymphomas exhibit two distinct phenotypes. Virology 144: 139-151.
Baldacci, P., A. Royal, et al. (1981). DNA organisation in the chicken lysozyme gene region. Nucleic Acids Res 9 : 3575-3588.
Baniahmad, A., C. Steiner, et al. (1990). Modular structure of a chicken lysozyme silencer: involvement of an unusual thyroid hormone receptor binding site. Cell 61: 505-514.
Baniahmad, A., M. Muller, et al. (1987). Activity of two different silencer elements of the chicken lysozyme gene can be compensated by enhancer elements. Embo J. 6 : 2297-2303.
Bell, D. W., D. C. Wahrer, et al. (1999). Common nonsense mutations in RAD52. Cancer Res 59 : 3883-3888.
Bellard, M., G. Dretzen, et al. (1982). Disruption of the typical chromatin structure in a 2500 base-pair region at the 5' end of the actively transcribed ovalbumin gene. Embo J 1 : 223-230.
Bellard, M., G. Dretzen, et al. (1986). Hormonally induced alterations of chromatin structure in the polyadenylation and transcription termination regions of the chicken ovalbumin gene. Embo J 5 : 567-574.
Berinstein, N., N. Pennell, et al. (1992). Gene replacement with one-sided homologous recombination. Mol Cell Biol 12 : 360-367.
Beug H, von Kirchbach A, Doderlein G, Conscience JF, Graf T. (1979) Chicken hematopoietic cells transformed by seven strains of defective avian leukemia viruses display three distinct phenotypes of differentiation. Cell 18 : 375-390.
Bezzubova, O. Y. and J. M. Buerstedde (1994). Gene conversion in the chicken immunoglobulin locus: a paradigm of homologous recombination in higher eukaryotes. Experientia 50 : 270-276.
Bezzubova, O., A. Shinohara, et al. (1993). A chicken RAD51 homologue is expressed at high levels in lymphoid and reproductive organs. Nucleic Acids Res 21 : 1577-1580.
Bezzubova, O., A. Silbergleit, et al. (1997). Reduced X-ray resistance and homologous recombination frequencies in a RAD54-/- mutant of the chicken DT40 cell line. Cell 89 : 185-193.
Bhat, M. K., K. Ashizawa, et al. (1994). Phosphorylation enhances the target gene sequence-dependent dimerization of thyroid hormone receptor with retinoid X receptor. Proc Natl Acad Sci U S A 91 : 7927-7931.
Bibikova, M., D. Carroll, et al. (2001). Stimulation of homologous recombination through targeted cleavage by chimeric nucleases. Mol Cell Biol 21 : 289-297.
Bonifer, C., U. Jagle, et al. (1997). The chicken lysozyme locus as a paradigm for the complex developmental regulation of eukaryotic gene loci. J Biol Chem 272 : 26075-26078.
Brenneman, M., F. S. Gimble, et al. (1996). Stimulation of intrachromosomal homologous recombination in human cells by electroporation with site-specific endonucleases. Proc Natl Acad Sci U S A 93 : 3608-3612.
Buerstedde JM and Takeda S. (1991). Increased ratio of targeted to random integration after transfection of chicken B cell lines. Cell 67: 179-188.
Buerstedde JM et al. (1990). Light chain gene conversion continues at high rate in an ALV-induced cell line. Embo J. 9 : 921-927.
Burcin, M., R. Arnold, et al. (1997). Negative protein 1, which is required for function of the chicken lysozyme gene silencer in conjunction with hormone receptors, is identical to the multivalent zinc finger repressor CTCF. Mol Cell Biol 17 : 1281-1288.
Ciejek, E. M., M. J. Tsai, et al. (1983). Actively transcribed genes are associated with the nuclear matrix. Nature 306 : 607-609.
Cochet, M., F. Perrin, et al. (1979). Cloning of an almost full-length chicken conalbumin double-stranded cDNA. Nucleic Acids Res 6 : 2435-2452.
Cohen-Tannoudji, M., S. Robine, et al. (1998). I-SceI-induced gene replacement at a natural locus in embryonic stem cells. Mol Cell Biol 18 : 1444-1448.
Darling, D. S., R. L. Carter, et al. (1993). Different dimerization activities of alpha and beta thyroid hormone receptor isoforms. J Biol Chem 268 : 10221-10227.
Dean, D. C., B. J. Knoll, et al. (1983). A 5'-flanking sequence essential for progesterone regulation of an ovalbumin fusion gene. Nature 305 : 551-554.
Dean, D. C., R. Gope, et al. (1984). A similar 5'-flanking region is required for estrogen and progesterone induction of ovalbumin gene expression. J Biol Chem 259 : 9967-9970.
Dean, D. M., P. S. Jones, et al. (1996). Regulation of the chicken ovalbumin gene by estrogen and corticosterone requires a novel DNA element that binds a labile protein, Chirp-1. Mol Cell Biol 16 : 2015-2024.
Dean, D. M., R. R. Berger, et al. (1998). A winged-helix family member is involved in a steroid hormone-triggered regulatory circuit. Endocrinology 139 : 4967-4975.
Dean, D.M., Jones, P.S. et al. (2001). Alterations in chromatin structure are implicated in the activation of the steroid hormone response unit of the ovalbumin gene. DNA & Cell Biology 20 : 27-39.
Dierich, A., M. P. Gaub, et al. (1987). Cell-specificity of the chicken ovalbumin and conalbumin promoters. Embo J 6 : 2305-2312.
Dolle, A. and W. H. Stratling (1990). Genomic footprinting of proteins interacting with the chicken lysozyme promoter Gene 95 : 187-193.
Dominguez-Steglich, M., J. M. Jeltsch, et al. (1992). In situ mapping of the chicken progesterone receptor gene and the ovalbumin gene. Genomics 13 : 1343-1344.
Donoho, G., M. Jasin, et al. (1998). Analysis of gene targeting and intrachromosomal homologous recombination stimulated by genomic double-strand breaks in mouse embryonic stem cells. Mol Cell Biol 18 : 4070-4078.
Dronkert, M. L., H. B. Beverloo, et al. (2000). Mouse RAD54 affects DNA double-strand break repair and sister chromatid exchange. Mol Cell Biol 20 : 3147-3156.
Elliott, B. and M. Jasin (2001). Repair of double-strand breaks by homologous recombination in mismatch repair-defective mammalian cells. Mol Cell Biol 21 : 2671-2682.
Essers, J., R. W. Hendriks, et al. (1997). Disruption of mouse RAD54 reduces ionizing radiation resistance and homologous recombination. Cell 89 : 195-204.
Evans, M. I. and G. S. McKnight (1984). Regulation of the ovalbumin gene: effects of insulin, adenosine 3',5'- monophosphate, and estrogen. Endocrinology 115 : 368-377.
Faust, N., C. Bonifer, et al. (1999). Differential activity of the -2.7 kb chicken lysozyme enhancer in macrophages of different ontogenic origins is regulated by C/EBP and PU.1 transcription factors. DNA Cell Biol 18 : 631-642.
Fritton, H. P., A. E. Sippel, et al. (1983). Nuclease-hypersensitive sites in the chromatin domain of the chicken lysozyme gene. Nucleic Acids Res 11 : 3467-3485.
Fritton, H. P., T. Igo-Kemenes, et al. (1987). DNase I-hypersensitive sites in the chromatin structure of the lysozyme gene in steroid hormone target and non-target cells. Biol Chem Hoppe Seyler 368 : 111-119.
Fujitani, Y., K. Yamamoto, et al. (1995). Dependence of frequency of homologous recombination on the homology length. Genetics 140 : 797-809.
Gannon, F., K. O'Hare, et al. (1979). Organisation and sequences at the 5' end of a cloned complete ovalbumin gene. Nature 278 : 428-434.
Goethe, R. and P. V. Loc (1994). The far upstream chicken lysozyme enhancer at -6.1 kilobase, by interacting with NF-M, mediates lipopolysaccharide-induced expression of the chicken lysozyme gene in chicken myelomonocytic cells. J Biol Chem 269 : 31302-31309.
Goethe, R. and L. Phi-van (1998). Posttranscriptional lipopolysaccharide regulation of the lysozyme gene at processing of the primary transcript in myelomonocytic HD11 cells. J Immunol 160 : 4970-4978.
Gossen, M. and H. Bujard (1992). Tight control of gene expression in mammalian cells by tetracycline- responsive promoters. Proc Natl Acad Sci U S A 89 : 5547-5551.
Grajer, K. H., A. Horlein, et al. (1993). Hepatic nuclear factor 1 alpha (HNF-1 alpha) is expressed in the oviduct of hens and interacts with regulatory elements of the lysozyme gene. Biol Chem Hoppe Seyler 374 : 319-326.
Grewal, T., M. Theisen, et al. (1992). The -6.1-kilobase chicken lysozyme enhancer is a multifactorial complex containing several cell-type-specific elements. Mol Cell Bio 1 12: 2339-2350.
Haecker, S. A., T. Muramatsu, et al. (1995). Repression of the ovalbumin gene involves multiple negative elements including a ubiquitous transcriptional silencer. Mol Endocrinol 9 : 1113-1126.
Hasty, P., M. Crist, et al. (1994). Efficiency of insertion versus replacement vector targeting varies at different chromosomal loci. Mol Cell Biol 14 : 8385-8890.
Hasty, P., J. Rivera-Perez, et al. (1991). The length of homology required for gene targeting in embryonic stem cells." Mol Cell Biol 11 : 5586-5591.
Hasty, P., J. Rivera-Perez, et al. (1992). The role and fate of DNA ends for homologous recombination in embryonic stem cells. Mol Cell Biol 12 : 2464-2474.
Hasty, P., J. Rivera-Perez, et al. (1995). Gene conversion during vector insertion in embryonic stem cells. Nucleic Acids Res 23 : 2058-2064.
Hasty, P., J. Rivera-Perez, et al. (1991). Target frequency and integration pattern for insertion and replacement vectors in embryonic stem cells. Mol Cell Biol 11: 4509-4517.
Hecht, A., A. Berkenstam, et al. (1988). A progesterone responsive element maps to the far upstream steroid dependent DNase hypersensitive site of chicken lysozyme chromatin. Embo J 7 : 2063-2073.
Himly M, DN Foster et al. (1998). The DF-1 chicken fibroblast cell line: transformation induced by diverse oncogenes and cell death resulting from infection by avian leukosis viruses. Virology 248 : 295-304.
Huber, M. C., T. Graf, et al. (1995). Dynamic changes in the chromatin of the chicken lysozyme gene domain during differentiation of multipotent progenitors to macrophages. DNA Cell Biol 14 : 397-402.
Hwung, Y. P., D. T. Crowe, et al. (1988a). The COUP transcription factor binds to an upstream promoter element of the rat insulin II gene. Mol Cell Biol 8 : 2070-2077.
Hwung, Y. P., L. H. Wang, et al. (1988b). Differential binding of the chicken ovalbumin upstream promoter (COUP) transcription factor to two different promoters. J Biol Chem 263 : 13470-13474.
Indra A.K., Warot X. Et al., (1999). Temporally-controlled site-specific mutagenesis in the basal layer of the epidermis: comparison of the recombinase activity of the tamoxifen-inducible Cre-ER(T) and Cre-ER(T2) recombinases. Nucleic Acids Res 27 : 4324-4327.
Ivanov, E. L. and J. E. Haber (1997). DNA repair: RAD alert. Curr Biol 7 : R492-495.
Jantzen, K., H. P. Fritton, et al. (1986). The DNase 1 sensitive domain of the chicken lysozyme gene spans 24 kb. Nucleic Acids Res 14 : 6085-6099.
Jasin, M. (1996). Genetic manipulation of genomes with rare-cutting endonucleases. Trends Genet 12 : 224-228.
Jeltsch, J. M. and P. Chambon (1982). The complete nucleotide sequence of the chicken ovotransferrin mRNA. Eur J Biochem 122 : 291-295.
Jeltsch, J. M., R. Hen, et al. (1987). Sequence of the chicken ovotransferrin gene. Nucleic Acids Res 15 : 7643-7645.
Kato, S., L. Tora, et al. (1992). A far upstream estrogen response element of the ovalbumin gene contains several half-palindromic 5'-TGACC-3' motifs acting synergistically. Cell 68 : 731-742.
Kawaguchi T, Nomura K, Hirayama Y, Kitagawa T. (1987). Establishment and characterization of a chicken hepatocellular carcinoma cell line, LMH. Cancer Res. 47 : 4460-4463.
Kida, S., Y. Miura, et al. (1995). Effects of insulin-like growth factor-I, estrogen, glucocorticoid, and transferrin on the mRNA contents of ovalbumin and conalbumin in primary cultures of quail (Coturnix coturnix japonica) oviduct cells. Comp Biochem Physiol C Pharmacol Toxicol Endocrinol 110: 157- 164.
Kim S et al. (1990). Ongoing diversification of the rearranged immunoglobulin light-chain gene in a bursal lymphoma cell line. Mol. Cell. Biol. 10: 3224-3231.
Kim H., You S. et al. (2001). Increased mitochondrial-encoded gene transcription in immortal DF-1 cells. Exp Cell Res 265 : 339-347.
Kimura, A., A. Nishiyori, et al. (1993). Chicken ovalbumin upstream promoter-transcription factor (COUP-TF) represses transcription from the promoter of the gene for omithine transcarbamylase in a manner antagonistic to hepatocyte nuclear factor- 4 (HNF-4). J Biol Chem 268 : 11125-11133.
Klinge, C. M., B. F. Silver, et al. (1997). Chicken ovalbumin upstream promoter-transcription factor interacts with estrogen receptor, binds to estrogen response elements and half-sites, and inhibits estrogen-induced gene expression. J Biol Chem 272 : 31465-31474.
Knoll, B. J., T. Zarucki-Schulz, et al. (1983). Definition of the ovalbumin gene promoter by transfer of an ovalglobin fusion gene into cultured cells. Nucleic Acids Res 11 : 6733-6754.
Kohne, A. C., A. Baniahmad, et al. (1993). NeP1. A ubiquitous transcription factor synergizes with v-ERBA in transcriptional silencing. J Mol Biol 232 : 747-755.
Kontaraki, J., H. H. Chen, et al. (2000). Chromatin fine structure profiles for a developmentally regulated gene: reorganization of the lysozyme locus before trans-activator binding and gene expression. Genes Dev 14 : 2106-2122.
Kumar, S. and J. P. Simons (1993). The effects of terminal heterologies on gene targeting by insertion vectors in embryonic stem cells. Nucleic Acids Res 21 : 1541-1548.
Leblanc P., Dastugue B. et al. (1999). The integration machinery of ZAM, a retroelement from Drosophila melanogaster, acts as a sequence -specific endonuclease. J Virol 73 : 7061-7064.
Leblanc, P., Desset S. et al. (1997). invertebrate retroviruses : ZAM a new candidate in D.melanogaster.Embo J., 16: 7521-7531.
Ledermann, B. (2000). Embryonic stem cells and gene targeting. Exp Physiol 85 : 603-613.
LeMeur, M., N. Glanville, et al. (1981). The ovalbumin gene family: hormonal control of X and Y gene transcription and mRNA accumulation. Cell 23 : 561-571.
Li-Chan E. and S. Nakai. (1989). Critical reviews : Biochemical basis for the properties of egg white. Poultry Biology 2, 21-58.
Loc, P. V. and W. H. Stratling (1988). The matrix attachment regions of the chicken lysozyme gene co-map with the boundaries of the chromatin domain. Embo J 7 : 655-664.
Lukacsovich, T. and A. S. Waldman (1999). Suppression of intrachromosomal gene conversion in mammalian cells by small degrees of sequence divergence. Genetics 151 : 1559-1568.
McReynolds, L., B. W. O'Malley, et al. (1978). Sequence of chicken ovalbumin mRNA. Nature 273 : 723-728.
Metzger, D. and P. Chambon (2001). Site- and time-specific gene targeting in the mouse. Methods 24 : 71-80.
Metzger, D., J. Clifford, et al. (1995). Conditional site-specific recombination in mammalian cells using a ligand-dependent chimeric Cre recombinase. Proc Natl Acad Sci U S A 92: 6991-6995.
Mink, S., B. Mutschler, et al. (1996). A novel function for Myc: inhibition of C/EBP-dependent gene activation. Proc Natl Acad Sci U S A 93 : 6635-6640.
Mohammed, S. M., S. Morrison, et al. (1998). Deposition of genetically engineered human antibodies into the egg yolk of hens. Immunotechnology 4 : 115-125.
Monroe, D. G. and M. M. Sanders (2000). The COUP-adjacent repressor (CAR) element participates in the tissue- specific expression of the ovalbumin gene. Biochim Biophys Acta 1517 : 27-32.
Morrison, C., A. Shinohara, et al. (1999). The essential functions of human Rad51 are independent of ATP hydrolysis. Mol Cell Biol 19 : 6891-6897.
Morrison, C., E. Sonoda, et al. (2000). The controlling role of ATM in homologous recombinational repair of DNA damage. Embo J 19 : 463-471.
Morrison, C. and S. Takeda (2000). Genetic analysis of homologous DNA recombination in vertebrate somatic cells. Int J Biochem Cell Biol 32 : 817-831.
Moynahan, M. E., J. W. Chiu, et al. (1999). Brcal controls homology-directed DNA repair. Mol Cell 4 : 511-518.
Muramatsu, T., H. Hiramatsu, et al. (1995). Induction of ovalbumin mRNA by ascorbic acid in primary cultures of tubular gland cells of the chicken oviduct. Comp Biochem Physiol B Biochem Mol Biol 112 : 209-216.
Nguyen-Huu, M. C., M. Stratmann, et al. (1979). Chicken lysozyme gene contains several intervening sequences. Proc Natl Acad Sci U S A 76 : 76-80.
Nau F. (1987). Synthèse bibliographique, Le jaune d'oeuf, ENSA, INRA.
Nowock, J. and A. E. Sippel (1982). Specific protein-DNA interaction at four sites flanking the chicken lysozyme gene. Cell 30 : 607-615.
Pain B, Chenevier P, Samarut J (1999). Chicken embryonic stem cells and transgenic strategies. Cell Tissues Organs 165 : 212-219.
Pain, B., M. E. Clark, et al. (1996). Long-term in vitro culture and characterisation of avian embryonic stem cells with multiple morphogenetic potentialities. Development 122 : 2339-2348.
Palmiter, R. D., E. R. Mulvihill, et al. (1981). Steroid hormone regulation of ovalbumin and conalbumin gene transcription. A model based upon multiple regulatory sites and intermediary proteins. J Biol Chem 256 : 7910-7916.
Phillips, J. E. and M. P. Calos (1999). Effects of homology length and donor vector arrangement on the efficiency of double-strand break-mediated recombination in human cells. Somat Cell Mol Genet 25 : 91-100.
Phi-Van, L., J. P. von Kries, et al. (1990). The chicken lysozyme 5' matrix attachment region increases transcription from a heterologous promoter in heterologous cells and dampens position effects on the expression of transfected genes. Mol Cell Biol 10 : 2302-2307.
Phi-van, L., C. Sellke, et al. (1998). An initiation zone of chromosomal DNA replication at the chicken lysozyme gene locus. J Biol Chem 273 : 18300-18307.
Phi-Van, L. and W. H. Stratling (1996). Dissection of the ability of the chicken lysozyme gene 5' matrix attachment region to stimulate transgene expression and to dampen position effects. Biochemistry 35 : 10735-10742.
Phi van, L. (1996). Transcriptional activation of the chicken lysozyme gene by NF-kappa Bp65 (RelA) and c-Rel, but not by NF-kappa Bp50. Biochem J 313 : 39-44.
Phi-van, L. and W. H. Stratling (1999). An origin of bidirectional DNA replication is located within a CpG island at the 3" end of the chicken lysozyme gene. Nucleic Acids Res 27 : 3009-3017.
Porter, G., J. Westmoreland, et al. (1996). Homologous and homeologous intermolecular gene conversion are not differentially affected by mutations in the DNA damage or the mismatch repair genes RAD1, RAD50, RAD51, RAD52, RAD54, PMS1 and MSH2. Genetics 143 : 755-767.
Pereira, F. A., Y. Qiu, et al. (1995). Chicken ovalbumin upstream promoter transcription factor (COUP-TF): expression during mouse embryogenesis. J Steroid Biochem Mol Biol 53 : 503-508.
Ramirez-Solis, R., A. C. Davis, et al. (1993). Gene targeting in embryonic stem cells. Methods Enzymol 225: 855-878.
Regenhard, P., R. Goethe, et al. (2001). Involvement of PKA, PKC, and Ca2+ in LPS-activated expression of the chicken lysozyme gene. J Leukoc Biol 69 : 651-658.
Renkawitz, R., G. Schutz, et al. (1984a). Sequences in the promoter region of the chicken lysozyme gene required for steroid regulation and receptor binding. Cell 37 : 503-510.
Renkawitz, R., H. Beug, et al. (1982). Expression of a chicken lysozyme recombinant gene is regulated by progesterone and dexamethasone after microinjection into oviduct cells. Cell 31 : 167-176.
Renkawitz, R., U. Danesch, et al. (1984b). Steroid controlled expression of the chicken lysozyme and the rat tryptophan oxygenase gene after transfer into eukaryotic cells. J Steroid Biochem 20 : 99-104.
Robine, S., F. Jaisser, et al. (1997). Epithelial cell growth and differentiation. IV. Controlled spatiotemporal expression of transgenes: new tools to study normal and pathological states. Am J Physiol 273 : G759-762.
Robinson, C. E., X. Wu, et al. (1999). A corepressor and chicken ovalbumin upstream promoter transcriptional factor proteins modulate peroxisome proliferator-activated receptor- gamma2/retinoid X receptor alpha-activated transcription from the murine lipoprotein lipase promoter. Endocrinology 140 : 1586-1593.
Sanders, M. M. and G. S. McKnight (1988). Positive and negative regulatory elements control the steroid- responsive ovalbumin promoter. Biochemistry 27 : 6550-6557.
Sargent, R. G., M. A. Brenneman, et al. (1997). Repair of site-specific double-strand breaks in a mammalian chromosome by homologous and illegitimate recombination. Mol Cell Biol 17 : 267-277.
Sargent, R. G., J. L. Meservy, et al. (2000). Role of the nucleotide excision repair gene ERCC1 in formation of recombination-dependent rearrangements in mammalian cells. Nucleic Acids Res 28 : 3771-3778.
Sarig, R., V. Mezger-Lallemand, et al. (2000). Increased efficiency of homologous recombination in ES cells by cleavage at both ends of homology in the targeting vector. Transgenic Res 9 : 79-80.
Sauveur B. (1988). Reproduction des volailles et production d'oeufs. INRA eds Sawaya, B. E., O. Rohr, et al. (1996). Chicken ovalbumin upstream promoter transcription factor, a transcriptional activator of HIV-1 gene expression in human brain cells. J Biol Chem 271 : 23572-23576.
Sawicki, J. A., B. Monks, et al. (1998). Cell-specific ecdysone-inducible expression of FLP recombinase in mammalian cells.Biotechniques 25 : 868-870, 872-875.
Schaeffer, E., M. A. Lucero, et al. (1987). Complete structure of the human transferrin gene. Comparison with analogous chicken gene and human pseudogene. Gene 56 : 109-116.
Schweers, L. A., D. E. Frank, et al. (1990). The steroid-dependent regulatory element in the ovalbumin gene does not function as a typical steroid response element. J Biol Chem 265 : 7590-7595.
Schweers, L. A. and M. M. Sanders (1991). A protein with a binding specificity similar to NF-kappa B binds to a steroid-dependent regulatory element in the ovalbumin gene. J Biol Chem 266 : 10490-10497.
Sensenbaugh, K. R. and M. M. Sanders (1999). Multiple promoter elements including a novel repressor site modulate expression of the chick ovalbumin gene. DNA Cell Biol 18 : 147-156.
Shcherbakova, O. G., V. A. Lanzov, et al. (2000). Overexpression of bacterial RecA protein stimulates homologous recombination in somatic mammalian cells. Mutat Res 459: 65-71.
Shibata, H., Z. Nawaz, et al. (1997). Gene silencing by chicken ovalbumin upstream promoter-transcription factor I (COUP-TFI) is mediated by transcriptional corepressors, nuclear receptor-corepressor (N-CoR) and silencing mediator for retinoic acid receptor and thyroid hormone receptor (SMRT). Mol Endocrinol 11 : 714-724.
Shinohara, M., E. Shita-Yamaguchi, et al. (1997). Characterization of the roles of the Saccharomyces cerevisiae RAD54 gene and a homologue of RAD54, RDH54/TID1, in mitosis and meiosis. Genetics 147 : 1545-1556.
Short, M. L., J. Nickel, et al. (1996). Lysozyme gene expression and regulation. Exs 75: 243-257.
Short, M. L., J. Nickel, et al. (1996). In vivo protein interaction with the mouse M-lysozyme gene downstream enhancer correlates with demethylation and gene expression. Cell Growth Differ 7 : 1545-1550.
Sippel, A. E., H. Land, et al. (1978). Cloning of chicken lysozyme structural gene sequences synthesized in vitro. Nucleic Acids Res 5 : 3275-3294.
Skafar, D. F. and S. S. Seaver (1985). Desensitization of the chick oviduct to estrogen: mediation at different levels of gene expression. Endocrinology 116 : 1755-1762.
Skoufos, E. and M. M. Sanders (1992). Regulation of expression of the chicken ovalbumin gene: interactions between steroid hormones and second messenger systems. Mol Endocrinol 6 : 1412-1417.
Steiner, C., M. Muller, et al. (1987). Lysozyme gene activity in chicken macrophages is controlled by positive and negative regulatory elements. Nucleic Acids Res 15 : 4163-4178.
Stevens L. (1991). Egg white proteins. Comp. Biochem. Physiol. 100B, 1-9.
Stief, A., D. M. Winter, et al. (1989). A nuclear DNA attachment element mediates elevated and position- independent gene activity. Nature 341 : 343-345.
Takata, M., M. S. Sasaki, et al. (2000). The Rad51 paralog Rad51B promotes homologous recombinational repair. Mol Cell Biol 20 : 6476-6482.
Takata, M., M. S. Sasaki, et al. (1998). Homologous recombination and nonhomologous end-joining pathways of DNA double-strand break repair have overlapping roles in the maintenance of chromosomal integrity in vertebrate cells. Embo J 17 : 5497-5508.
Templeton, N. S. (2000). Strategies for improving the frequency and assessment of homologous recombination. Methods Mol Biol 133 : 45-60.
Te Riele, H., E. R. Maandag, et al. (1992). Highly efficient gene targeting in embryonic stem cells through homologous recombination with isogenic DNA constructs. Proc Nat1 Acad Sci U S A 89 : 5128-5132.
Thompson, S., A. R. Clarke, et al. (1989). Germ line transmission and expression of a corrected HPRT gene produced by gene targeting in embryonic stem cells. Cell 56 : 313-321.
Theisen, M., A. Stief, et al. (1986). The lysozyme enhancer: cell-specific activation of the chicken lysozyme gene by a far-upstream DNA element. Embo J 5 : 719-724.
Thomas, K. R. and M. R. Capecchi (1987). Site-directed mutagenesis by gene targeting in mouse embryo-derived stem cells. Cell 51 : 503-512.
Vallier, L., J. Mancip, et al. (2001). An efficient system for conditional gene expression in embryonic stem cells and in their in vitro and in vivo differentiated derivatives. Proc Nat1 Acad Sci U S A 98 : 2467-2472.
von der Ahe, D., J. M. Renoir, et al. (1986). Receptors for glucocorticosteroid and progesterone recognize distinct features of a DNA regulatory element. Proc Natl Acad Sci U S A 83 : 2817-2821,
von Kries, J. P. and W. H. Stratling (1988). Lysozyme gene specific transcription in isolated hen oviduct nuclei. Int J Biochem 20 : 633-637.
Wang, L. H., S. Y. Tsai, et al. (1987). Purification and characterization of chicken ovalbumin upstream promoter transcription factor from HeLa cells. J Biol Chem 262: 16080-16086.
Woo, S. L., W. G. Beattie, et al. (1981). Complete nucleotide sequence of the chicken chromosomal ovalbumin gene and its biological significance. Biochemistry 20 : 6437-6446.
Yagi T, Y. Ikawa, et al. (1990). Homologous recombination at c-fyn locus of mouse embryonic stem cells with use of diphtheria toxin A-fragment gene in negative selection. Proc Natl Acad Sci U S A 87 :9918-9922.
Yamaguchi-Iwai, Y., E. Sonoda, et al. (1998). Homologous recombination, but not DNA repair, is reduced in vertebrate cells deficient in RAD52. Mol Cell Biol 18 : 6430-6435.
Yamaguchi-Iwai, Y., E. Sonoda, et al. (1999). Mre11 is essential for the maintenance of chromosomal DNA in vertebrate cells. Embo J 18 : 6619-6629.
Yanagawa, Y., T. Kobayashi, et al. (1999). Enrichment and efficient screening of ES cells containing a targeted mutation: the use of DT-A gene with the polyadenylation signal as a negative selection maker. Transgenic Res 8 : 215-221.
Yancey-Wrona, J. E. and R. D. Camerini-Otero (1995). The search for DNA homology does not limit stable homologous pairing promoted by RecA protein. Curr Biol 5 : 1149-1158.
Yanez, R. J. and A. C. Porter (2000). Uracil incorporation into a gene targeting construct reduces the frequency of homologous and nonhomologous recombinants in human cells. Mutat Res 461 : 157-62.
Yanez, R. J. and A. C. Porter (1999). Influence of DNA delivery method on gene targeting frequencies in human cells. Somat Cell Mol Genet 25 : 27-31.
Zheng, H., P. Hasty, et al. (1991). Fidelity of targeted recombination in human fibroblasts and murine embryonic stem cells. Proc Natl Acad Sci U S A 88 : 8067-8071.

## Revendications

1. Procédé d'obtention d'une cellule souche embryonnaire (ES) aviaire modifiée par recombinaison homologue, ledit procédé comprenant les étapes suivantes :
A) l'introduction d'un vecteur de recombinaison homologue dans ladite cellule ES aviaire par une méthode de transfection ;
B) la sélection des cellules par l'addition d'un agent de sélection dans le milieu de culture ; et
C) le criblage des clones résistants et amplification,
**caractérisé en ce que** :
ledit vecteur de recombinaison homologue introduit à l'étape A) comprend dans une base plasmidique un enchaînement d'au moins un élément pris successivement parmi
a) un fragment d'ADN génomique contenant le bras d'homologie 5' du gène ciblé fusionné à,
b) une séquence nucléotidique signal de sécrétion fusionnée à,
c) une courte séquence nucléotidique intronique fusionnée à,
d) une séquence nucléotidique codant pour une protéine exogène d'intérêt fusionnée à,
e) une séquence poly A de terminaison de la transcription fusionnée à,
f) une cassette de sélection positive comprenant un promoteur, un gène de résistance à un agent de sélection et une séquence poly A de terminaison de transcription, ladite cassette pouvant être fusionnée à,
g) un fragment d'ADN génomique contenant le bras d'homologie 3' du gène ciblé,
h) une cassette de sélection négative comprenant un promoteur, un gène assurant la transformation d'un substrat présent dans le milieu de culture en une substance toxique pour la cellule qui exprime le gène et une séquence polyA de terminaison de transcription, et
**caractérisé en ce que** :
le locus ciblé par le vecteur de recombinaison est le locus de l'ovalbumine ou le locus du lysozyme.

2. Procédé selon la revendication 1, **caractérisé en ce que** le vecteur comprend d) la séquence codante pour la protéine exogène fusionnée en son extrémité 5' avec c) une courte séquence intronique en particulier comprenant la séquence SEQ ID N° 1 elle même fusionnée avec b) une séquence peptide signal de sécrétion, en particulier la séquence codante pour le peptide signal du lysozyme comprenant la séquence SEQ ID N° 2.

3. Procédé selon l'une des revendications 1 et 2, **caractérisé en ce que** le vecteur comprend d) la séquence codante pour la protéine exogène fusionnée en son extrémité 3' avec une séquence poly A.

4. Procédé selon l'une des revendications 1 à 3, **caractérisé en ce que** le vecteur comprend au moins une séquence site interne d'entrée des ribosomes IRES en fusion avec au moins deux séquences codantes pour la protéine d'intérêt exogène.

5. Procédé selon l'une des revendications 1 à 4, **caractérisé en ce que** le vecteur comprend au moins une séquence IRES en fusion avec au moins deux séquences codantes pour différentes chaînes constituant une protéine d'intérêt, en particulier les chaînes lourde et légère d'un anticorps de quelle que nature que ce soit, en particulier un anticorps monoclonal, un fragment fab.

6. Procédé selon l'une des revendications 4 et 5, **caractérisé en ce que** la séquence IRES est prise dans le groupe des séquences IRES de groupe I ou de groupe II, en particulier les séquences V130, Idemfix, Zam.

7. Procédé selon l'une des revendication 1 à 6, **caractérisé en ce qu'**à l'étape A), la méthode de transfection est choisie parmi les méthodes de transfection à l'aide d'un liposome, d'un polycation ou par électroporation.

8. Procédé selon la revendication 7, **caractérisé en ce qu'**à l'étape A), la méthode de transfection est choisie parmi les méthodes par électroporation.

9. Procédé selon l'une des revendications 1 à 8, **caractérisé en ce que** les cellules sont des cellules ES aviaires issues de la mise en culture de blastodermes.

10. Procédé selon l'une des revendications 1 à 9, **caractérisé en ce que** les cellules sont des cellules ES aviaires qui réagissent spécifiquement avec au moins un anticorps sélectionné parmi ECMA-7, SSEA-1, SSEA-3, TEC-01, EMA-1 et EMA-6.

11. Procédé selon l'une des revendications 1 à 10, **caractérisé en ce que** le surnageant de culture des clones recombinés contient la protéine exogène d'intérêt, en particulier après induction du clone à l'aide de différents inducteurs, en particulier l'acide rétinoïque, le diméthylsulfoxide, le TPA ou de conditions de cultures spécifiques, en particulier par la formation de corps embryoïdes.

12. Procédé selon l'une des revendications 1 à 11, **caractérisé en ce que** les deux allèles du locus ciblé sont modifiés.

13. Procédé selon l'une des revendications 1 à 12, **caractérisé en ce que** les cellules ES sont des cellules présentant un phénotype alcaline phosphatase positive.

14. Procédé selon l'une des revendications 1 à 13, **caractérisé en ce que** le milieu utilisé comprend une cytokine choisie dans le groupe constitué de LIF, IL-11, IL-6 et leurs différents mélanges.

15. Procédé selon l'une des revendications 1 à 14, **caractérisé en ce que** le milieu utilisé comprend différents facteurs en particulier le SCF, l'IGF-1, le bFGF, le CNTF et l'oncostatine.

16. Procédé de production d'une protéine d'intérêt comprenant l'extraction de la protéine exogène exprimée dans le surnageant des cellules issues du procédé selon l'une des revendications 1 à 15.

17. Utilisation d'une cellule aviaire obtenue par un procédé selon l'une des revendications 1 à 15 pour la production de la protéine exogène d'intérêt.

18. Procédé d'obtention d'un animal appartenant à l'espèce aviaire capable d'exprimer une protéine exogène d'intérêt, **caractérisé en ce qu'**il comprend les étapes suivantes :
a) obtention de cellules aviaires modifiées par le procédé défini selon l'une des revendications 1 à 15,
b) introduction de la cellule obtenue à l'étape a) dans la cavité sub-germinale d'un embryon, ou dans la circulation sanguine de l'embryon, et
c) incubation de l'embryon obtenu à l'étape b).

19. Procédé d'obtention d'un animal appartenant à l'espèce selon la revendication 18, **caractérisé en ce que** le vecteur utilisé à l'étape a) d'obtention de cellules aviaires modifiées permet une expression tissu-spécifique.

20. Procédé d'obtention d'un animal selon la revendication 19, **caractérisé en ce que** le vecteur utilisé à l'étape a) permet une expression de la protéine exogène tissu-spécifique dans le foie, le sang, la moelle, les organes lymphoïdes ou l'oviducte.

21. Procédé de production d'une protéine d'intérêt comprenant l'extraction de la protéine exogène exprimée dans les tissus d'un animal obtenu à partir du procédé selon l'une des revendications 18 à 20.

22. Procédé selon la revendication 21, **caractérisé en ce que** la protéine est extraite du sang, du jaune ou du blanc d'oeuf.

23. OEuf susceptible d'être obtenu à partir d'un animal obtenu par un procédé selon l'une des revendications 18 à 20, **caractérisé en ce qu'**une partie de l'ovalbumine, ou du lysozyme est partiellement ou totalement remplacée par la protéine d'intérêt exogène.

24. OEuf selon la revendication 23, **caractérisé en ce que** la protéine d'intérêt exogène est sélectionnée parmi les peptides à intérêt thérapeutique, les interleukines, les cytokines, les hormones et les anticorps.

## Claims

1. Method to obtain an avian embryonic stem (ES) cell modified by means of homologous recombination, said method comprising the following steps:
A) introduction of a homologous recombination vector into said avian ES cell by means of a transfection method;
B) selection of cells by addition of a selection agent in the culture medium; and
C) screening of resistant clones and amplification,
**characterised in that**:
said homologous recombination vector introduced in step A) comprises a linkage in a plasmidic base of at least one element taken successively from amongst:
a) a genomic DNA fragment containing the 5' homologous arm of the targeted gene fused with,
b) a secretion signal nucleotide sequence fused with,
c) a short intronic nucleotide sequence fused with,
d) a coding nucleotide sequence for an exogenous protein of interest fused with,
e) a termination poly A transcription sequence fused with,
f) a positive selection cassette comprising a promoter, a gene for resistance to a selection agent and a poly A sequence for transcription termination, said cassette being able to be fused with,
g) a genomic DNA fragment containing the 3' homologous arm of the targeted gene,
h) a negative selection cassette comprising a promoter, a gene ensuring the transformation of a substrate present in the culture medium into a toxic substance for the gene expression cell and a poly A sequence for transcription termination, and
**characterised in that**:
the locus targeted by the recombination vector is the ovalbumin locus or lysozyme locus.

2. Method according to claim 1, **characterised in that** the vector comprises d) the coding nucleotide sequence for the fused exogenous protein of interest at its 5' end with c) a short intronic sequence comprising in particular the sequence SEQ ID N°1 itself fused with b) a secretion peptide signal sequence, in particular the coding sequence for the peptide signal of the lysozyme comprising the SEQ ID N°2 sequence.

3. Method according to any of claims 1 and 2, **characterised in that** the vector comprises d) the coding nucleotide sequence for the fused exogenous protein of interest at its 3' end with a poly A sequence.

4. Method according to any of claims 1 to 3, **characterised in that** the vector comprises at least one IRES internal ribosome entry site sequence in fusion with at least two coding sequences for the exogenous protein of interest.

5. Method according to any of claims 1 to 4, **characterised in that** the vector comprises at least one IRES sequence in fusion with at least two coding sequences for different chains constituting a protein of interest, in particular the light and heavy chains of an antibody of any nature whatsoever, in particular a monoclonal antibody, a fab fragment.

6. Method according to any of claims 4 and 5, **characterised in that** the IRES sequence is taken in the group of IRES sequences of group I or group II, in particular the V130, Idemfix, Zam sequences.

7. Method according to any of claims 1 to 6, **characterised in that** in step A), the transfection method is selected from the transfection methods with the aid of a liposome, a polycation or by electroporation.

8. Method according to claim 7, **characterised in that** in step A), the transfection method is selected from the electroporation methods.

9. Method according to any of claims 1 to 8, **characterised in that** the cells are avian ES cells resulting from the culture of blastoderms.

10. Method according to any of claims 1 to 9, **characterised in that** the cells are avian ES cells that react specifically with at least one antibody selected from amongst ECMA-7, SSEA-1, SSEA-3, TEC-01, EMA-1 and EMA-6.

11. Method according to any of claims 1 to 10, **characterised in that** the culture supernatant of the recombined clones contains the exogenous protein of interest, in particular after induction of the clone with different inducers, in particular retinoic acid, dimethylsulphoxide, TPA or specific culture conditions, in particular by forming embryonic bodies.

12. Method according to any of claims 1 to 11, **characterised in that** the two alleles of the targeted locus are modified.

13. Method according to any of claims 1 to 12, **characterised in that** the ES cells are cells showing a positive phosphatase alkaline phenotype.

14. Method according to any of claims 1 to 13, **characterised in that** the medium used comprises a cytokine chosen from amongst the group constituted by LIF, IL-11, IL-6 and their different mixtures.

15. Method according to any of claims 1 to 14, **characterised in that** the medium used comprises different factors, in particular SCF, IGF-1, bFGF, CNTF and oncostatin.

16. Method to produce a protein of interest comprising the extraction of the exogenous protein expressed in the supernatant of the cells obtained from the method according to any of claims 1 to 15.

17. Use of an avian cell obtained by means of a method according to any of claims 1 to 15 to produce the exogenous protein of interest.

18. Method to obtain an animal belonging to the avian species capable of expressing an exogenous protein of interest, **characterised in that** it comprises the following step:
a) obtainment of avian cells modified by the method defined according to any of claims 1 to 15,
b) introduction of the cell obtained in step a) into the sub-germinal cavity of an embryo, or in the blood circulation of the embryo, and
c) incubation of the embryo obtained in step b).

19. Method to obtain an animal belonging to the species according to claim 18, **characterised in that** the vector used in step a) to obtain modified avian cells enables tissue-specific expression.

20. Method to obtain an animal according to claim 19, **characterised in that** the vector used in step a) enables a tissue-specific expression of the exogenous protein in the liver, blood, bone marrow, lymphoid organs or oviduct.

21. Method to produce a protein of interest comprising the extraction of the exogenous protein expressed in the tissues of an animal obtained from the method according to any of claims 18 to 20.

22. Method according to claim 21, **characterised in that** the protein is extracted from the blood, the yolk or white of an egg.

23. Egg that can be obtained from an animal obtained by a method according to any of claims 18 to 20, **characterised in that** part of the ovalbumin, or lysozyme is partly or totally replaced by the exogenous protein of interest.

24. Egg according to claim 23, **characterised in that** the exogenous protein of interest is selected from amongst the peptides of therapeutic interest, interleukins, cytokines, hormones and antibodies.

## Patentansprüche

1. Verfahren zur Gewinnung einer embryonalen Stammzelle (ES) von Vögeln, welche durch homologe Rekombination modifiziert ist, wobei das Verfahren die folgenden Schritte umfasst:
A) die Einführung eines eine homologe Rekombination ermöglichenden Vektors in die ES-Zelle von Vögeln durch ein Transfektionsverfahren;
B) die Selektion der Zellen durch die Zugabe eines Selektionsmittels in das Kulturmedium; und
C) das Screenen der resistenten Klone und Amplifizierung,
**dadurch gekennzeichnet, dass**:
der eine homologe Rekombination ermöglichende Vektor, der in Schritt A) eingeführt wird, in einer Plasmid-Grundstruktur eine Aneinanderreihung von mindestens einem Element umfasst, welches nacheinander ausgewählt wird unter:
a) einem genomischen DNA-Fragment, enthaltend den 5' gelegenen Homologie-Sequenzabschnitt des Zielgens, fusioniert mit
b) einer Sekretionssignal-Nukleotidsequenz, fusioniert mit
c) einer kurzen Intron-Nukleotidsequenz, fusioniert mit
d) einer Nukleotidsequenz, welche ein exogenes Protein von Interesse kodiert, fusioniert mit
e) einer poly A-Transkriptionsterminationssequenz, fusioniert mit
f) einer eine positive Selektion ermöglichenden Kassette, umfassend einen Promotor, ein Resistenz gegen ein Selektionsmittel verleihendes Gen und eine poly A-Transkriptionsterminationssequenz, wobei die Kassette fusioniert sein kann mit
g) einem genomischen DNA-Fragment, welches den 3' gelegenen Homologie-Sequenzabschnitt des Zielgens enthält,
h) einer eine negative Selektion ermöglichenden Kassette, umfassend einen Promotor, ein Gen, welches die Umwandlung eines in dem Kulturmedium vorhandenen Substrats zu einer für die Zelle, die das Gen exprimiert, toxischen Substanz sicherstellt, und eine poly A-Transkriptionsterminationssequenz und
**dadurch gekennzeichnet, dass**:
der Genort, der zielgerichtet durch den Rekombinationsvektor angesteuert wird, der Genort von Ovalbumin oder der Genort von Lysozym ist.

2. Verfahren nach Anspruch 1, **dadurch gekennzeichnet, dass** der Vektor d) die das exogene Protein kodierende Sequenz fusioniert an ihrem 5'-Ende mit c) einer kurzen Intron-Sequenz, umfassend insbesondere die Sequenz SEQ ID No. 1, welche ihrerseits fusioniert ist mit b) einer Peptidsekretionssignalsequenz, insbesondere der das Signalpeptid von Lysozym kodierenden Sequenz, welche die Sequenz SEQ ID No. 2 umfasst, umfasst.

3. Verfahren nach einem der Ansprüche 1 und 2, **dadurch gekennzeichnet, dass** der Vektor d) die das exogene Protein kodierende Sequenz fusioniert an ihrem 3'-Ende mit einer poly A-Sequenz umfasst.

4. Verfahren nach einem der Ansprüche 1 bis 3, **dadurch gekennzeichnet, dass** der Vektor mindestens eine interne Ribosomeneintrittssequenz IRES fusioniert mit mindestens zwei Sequenzen, welche das exogene Protein von Interesse kodieren, umfasst.

5. Verfahren nach einem der Ansprüche 1 bis 4, **dadurch gekennzeichnet, dass** der Vektor mindestens eine IRES-Sequenz fusioniert mit mindestens zwei Sequenzen, welche unterschiedliche Ketten, welche ein Protein von Interesse bilden, insbesondere die schwere und die leichte Kette eines Antikörpers unabhängig von dessen Natur, insbesondere einen monoklonalen Antikörper, ein fab-Fragment, kodieren, umfasst.

6. Verfahren nach einem der Ansprüche 4 und 5, **dadurch gekennzeichnet, dass** die IRES-Sequenz aus der Gruppe der IRES-Sequenzen der Gruppe I oder der Gruppe II, insbesondere den Sequenzen V130, Idemfix, Zam ausgewählt wird.

7. Verfahren nach einem der Ansprüche 1 bis 6, **dadurch gekennzeichnet, dass** in Schritt A) das Transfektionsverfahren unter den Transfektionsverfahren mit Hilfe eines Liposoms, eines Polykations oder durch Elektroporation ausgewählt wird.

8. Verfahren nach Anspruch 7, **dadurch gekennzeichnet, dass** in Schritt A) das Transfektionsverfahren unter den Verfahren durch Elektroporation ausgewählt wird.

9. Verfahren nach einem der Ansprüche 1 bis 8, **dadurch gekennzeichnet, dass** die Zellen ES-Zellen von Vögeln sind, welche aus dem Inkulturnehmen von Blastodermen stammen.

10. Verfahren nach einem der Ansprüche 1 bis 9, **dadurch gekennzeichnet, dass** die Zellen ES-Zellen von Vögeln sind, die spezifisch mit mindestens einem unter ECMA-7, SSEA-1, SSEA-3, TEC-01, EMA-1 und EMA-6 ausgewählten Antikörper reagieren.

11. Verfahren nach einem der Ansprüche 1 bis 10, **dadurch gekennzeichnet, dass** der Kulturüberstand der rekombinierten Klone das exogene Protein von Interesse enthält, insbesondere nach Induktion des Klons mit Hilfe von verschiedenen Induktoren, insbesondere Retinsäure, Dimethylsulfoxid, TPA oder speziellen Kulturbedingungen, insbesondere durch die Bildung von "embryoid bodies".

12. Verfahren nach einem der Ansprüche 1 bis 11, **dadurch gekennzeichnet, dass** die beiden Allele des Ziel-Genorts modifiziert sind.

13. Verfahren nach einem der Ansprüche 1 bis 12, **dadurch gekennzeichnet, dass** die ES-Zellen Zellen sind, die einen alkalische Phosphatase-positiven Phänotyp aufweisen.

14. Verfahren nach einem der Ansprüche 1 bis 13, **dadurch gekennzeichnet, dass** das eingesetzte Medium ein Zytokin, welches in der aus LIF, IL-11, IL-6 und deren verschiedenen Mischungen gebildeten Gruppe ausgewählt wird, umfasst.

15. Verfahren nach einem der Ansprüche 1 bis 14, **dadurch gekennzeichnet, dass** das eingesetzte Medium verschiedene Faktoren, insbesondere SCF, IGF-1, bFGF, CNTF und Oncostatin, umfasst.

16. Verfahren zur Herstellung eines Proteins von Interesse, umfassend die Extraktion des in den Überstand der Zellen, die aus dem Verfahren nach einem der Ansprüche 1 bis 15 stammen, exprimierten exogenen Proteins.

17. Verwendung einer Vogel-Zelle, die durch ein Verfahren nach einem der Ansprüche 1 bis 15 erhalten worden ist, für die Herstellung des exogenen Proteins von Interesse.

18. Verfahren zur Gewinnung eines Tiers, das zu der Spezies der Vögel gehört, welches in der Lage ist, ein exogenes Protein von Interesse zu exprimieren, **dadurch gekennzeichnet, dass** es die folgenden Schritte umfasst:
a) Gewinnung von Vogel-Zellen, die durch das gemäß einem der Ansprüche 1 bis 15 definierte Verfahren modifiziert worden sind,
b) Einführung der in Schritt a) erhaltenen Zelle in die Keimhöhle eines Embryos oder in den Blutkreislauf des Embryos und
c) Inkubation des in Schritt b) erhaltenen Embryos.

19. Verfahren zur Gewinnung eines Tiers, das zu der Spezies gehört, nach Anspruch 18, **dadurch gekennzeichnet, dass** der in Schritt a) zur Gewinnung von modifizierten Vogel-Zellen eingesetzte Vektor eine gewebespezifische Expression erlaubt.

20. Verfahren zur Gewinnung eines Tiers nach Anspruch 19, **dadurch gekennzeichnet, dass** der in Schritt a) eingesetzte Vektor eine gewebespezifische Expression des exogenen Proteins in der Leber, im Blut, im Knochenmark, in den lymphatischen Organen oder dem Eileiter erlaubt.

21. Verfahren zur Herstellung eines Proteins von Interesse, umfassend die Extraktion des in den Geweben eines Tiers, das ausgehend von dem Verfahren nach einem der Ansprüche 18 bis 20 erhalten worden ist, exprimierten exogenen Proteins.

22. Verfahren nach Anspruch 21, **dadurch gekennzeichnet, dass** das Protein aus dem Blut, dem Eigelb oder dem Eiweiß extrahiert wird.

23. Ei, welches ausgehend von einem durch ein Verfahren nach einem der Ansprüche 18 bis 20 erhaltenen Tier erhalten werden kann, **dadurch gekennzeichnet, dass** ein Teil des Ovalbumins oder des Lysozyms teilweise oder vollständig durch das exogene Protein von Interesse ersetzt ist.

24. Ei nach Anspruch 23, **dadurch gekennzeichnet, dass** das exogene Protein von Interesse unter den Peptiden von therapeutischem Interesse, den Interleukinen, den Zytokinen, den Hormonen und den Antikörpern ausgewählt wird.


### LISTE DE SEQUENCES

<110> VIVALIS
   INSTITUT NATIONAL DE LA RECHERCHE AGRONOMIQUE - INRA
   CENTRE NATIONAL DE LA RECHERCHE SCIENTIFIQUE - CNRS
   ECOLE NORMALE SUPERIEURE DE LYON - ENS
<120> Système d'expression de protéines exogènes dans un système aviaire
<130> D 19 900
<150> FR 01/15 111
   <151> 2001-11-22
<160> 17
<170> PatentIn Ver. 2.1
<210> 1
   <211> 161
   <212> ADN
   <213> Séquence artificielle
<220>
   <223> Description de la séquence artificielle: Séquence de l'intron artificiel des vecteurs pOvaRH et pLysoRH
<400> 1
   gtaagtatca aggttacaag aattcgcggc cgctctagaa ctagtggatc ccccgggctg 60
   caggaattcg atatcaagct tataacttcg tataatgtat gctatacgaa gttatcccat 120
   cattggtctt actgacatcc actttgcctt tctctccaca g                     161
<210> 2
   <211> 54
   <212> ADN
   <213> Séquence artificielle
<220>
   <223> Description de la séquence artificielle: Peptide signal de sécrétion du lysozyme
<220>
   <221> CDS
   <222> (1) .. (54)
<400> 2
   atg agg tct ttg cta atc ttg gtg ctt tgc ttc ctg ccc ctt gct gct   48
   Met Arg Ser Leu Leu Ile Leu Val Leu Cys Phe Leu Pro Leu Ala Ala
     1               5                  10                  15
   ctt ggt                                                           54
   Leu Gly
<210> 3
   <211> 18
   <212> PRT
   <213> Séquence artificielle
<223> Description de la séquence artificielle: Peptide signal de sécrétion du lysozyme
<400> 3
   Met Arg Ser Leu Leu Ile Leu Val Leu Cys Phe Leu Pro Leu Ala Ala
     1               5                  10                  15
   Leu Gly
<210> 4
   <211> 34
   <212> ADN
   <213> Séquence artificielle
<220>
   <223> Description de 1a séquence artificielle: Oligonucléotide cDNA-SmaI AS
<220>
   <221> misc_feature
   <222> (9)..(34)
   <223> n signifie a, t, c ou g
<400> 4
   atacccggnn nnnnnnnnnn nnnnnnnnnn nnnn 34
<210> 5
   <211> 52
   <212> ADN
   <213> Séquence artificielle
<220>
   <223> Description de la séquence artificielle: Oligonucléotide GE-Int-AS
<400> 5
   aaccttgata cttactccaa gagcagcaag gggcaggaag caaagcacca ag 52
<210> 6
   <211> 82
   <212> ADN
   <213> Séquence artificielle
<220>
   <223> Description de la séquence artificielle: Oligonucléotide GE-Int-Ceu AS
<400> 6
   tcgctacctt aggaccgtta tagttattgt aaccttgata cttactccaa gagcagcaag 60
   gggcaggaag caaagcacca ag 82
<210> 7
   <211> 30
   <212> ADN
   <213> Séquence artificielle
<220>
   <223> Description de la séquence artificielle: Oligonucléotide IL-11 (-ATG) S
<400> 7
   ctgaagcttg ttaactgtgt ttgccgcctg 30
<210> 8
   <211> 30
   <212> ADN
   <213> Séquence artificielle
<220>
   <223> Description de la séquence artificielle: Oligonucléotide IL-11 3'ncRevAS
<400> 8
   cgctctagat gtatgacaca tttaattccc 30
<210> 9
   <211> 33
   <212> ADN
   <213> Séquence artificielle
<220>
   <223> Description de la séquence artificielle: Oligonucléotide Lyso 1-18 S
<400> 9
   ggggaattcc atgggtatga ggtctttgct aat 33
<210> 10
   <211> 30
   <212> ADN
   <213> Séquence artificielle
<220>
   <223> Description de la séquence artificielle: Oligonucléotide Lyso 1789 S
<400> 10
   actatgacta ctggcaggag aatgaggaac 30
<210> 11
   <211> 28
   <212> ADN
   <213> Séquence artificielle
<220>
   <223> Description de la séquence artificielle: Oligonucléotide Lyso 2859 S
<400> 11
   acttgcacac ggaatatgga agggcatt 28
<210> 12
   <211> 33
   <212> ADN
   <213> Séquence artificielle
<220>
   <223> Description de la séquence artificielle: Oligonucléotide Lyso 3185 AS
<400> 12
   gtttgcgtat agtcgtttta atgagggatg cgt 33
<210> 13
   <211> 40
   <212> ADN
   <213> Séquence artificielle
<220>
   <223> Description de la séquence artificielle: Oligonucléotide OvaL 1657 S
<400> 13
   cgcgtctcga gccagagagc tcatcaattc ctgggtagaa 40
<210> 14
   <211> 40
   <212> ADN
   <213> Séquence artificielle
<220>
   <223> Description de la séquence artificielle: Oligonucléotide OvaL 7707 AS
<400> 14
   cgcgtctcga gttctatatg gggtgtggtg acagagcaat 40
<210> 15
   <211> 39
   <212> ADN
   <213> Séquence artificielle
<220>
   <223> Description de la séquence artificielle: Oligonucléotide OvaL 80 AS
<400> 15
   aattcggatc ccatgatggc aatggggcag tagaagatg 39
<210> 16
   <211> 40
   <212> ADN
   <213> Séquence artificielle
<220>
   <223> Description de la séquence artificielle: Oligonucléotide Oval-2995 S
<400> 16
   aattcggatc caacatttac tgggaagcac atctatcatc 40
<210> 17
   <211> 57
   <212> ADN
   <213> Séquence artificielle
<220>
   <223> Description de la séquence artificielle: Oligonucléotide SmaI3' in cDNA S
<220>
   <221> misc_feature
   <222> (50)..(57)
   <223> n signifie a, t, c ou g
<400> 17
   atacccggga ttggtcttac tgacatccac tttgcctttc tctccacagn nnnnnnn 57